(19) Europäisches Patentamt
European Patent Office
Office européen des brevets

(11) EP 0 621 899 B1

(12) # EUROPÄISCHE PATENTSCHRIFT

(45) Veröffentlichungstag und Bekanntmachung des Hinweises auf die Patenterteilung:
**19.06.1996 Patentblatt 1996/25**

(21) Anmeldenummer: **93902129.1**

(22) Anmeldetag: **28.12.1992**

(51) Int Cl.[6]: **C12N 15/58**, C12N 1/21, C12N 9/52

(86) Internationale Anmeldenummer:
**PCT/EP92/02989**

(87) Internationale Veröffentlichungsnummer:
**WO 93/13209 (08.07.1993 Gazette 1993/16)**

(54) **EXPRESSION SIGNALPEPTID-FREIER STAPHYLOKINASEN**
EXPRESSION SIGNAL-PEPTIDE-FREE STAPHYLOKINASES
STAPHYLOKINASES EXEMPTES DE PEPTIDES-SIGNAL D'EXPRESSION

(84) Benannte Vertragsstaaten:
**AT BE CH DE DK ES FR GB GR IE IT LI LU MC NL PT SE**

(30) Priorität: **30.12.1991 DE 4143279**
**22.06.1992 DE 4220516**
**01.12.1992 DE 4240801**

(43) Veröffentlichungstag der Anmeldung:
**02.11.1994 Patentblatt 1994/44**

(73) Patentinhaber: **Medac Gesellschaft für klinische Spezialpräparate mbH**
**D-20354 Hamburg (DE)**

(72) Erfinder:
- **BEHNKE, Detlev**
**D-6902 Jena (DE)**
- **SCHLOTT, Bernhard**
**D-6900 Jena (DE)**
- **ALBRECHT, Sybille**
**D-8020 Dresden (DE)**
- **GÜHRS, Karl-Heinz**
**D-6908 Jena (DE)**
- **HARTMANN, Manfred**
**D-6900 Jena (DE)**

(74) Vertreter: **UEXKÜLL & STOLBERG**
**Patentanwälte**
**Beselerstrasse 4**
**22607 Hamburg (DE)**

(56) Entgegenhaltungen:
**EP-A- 0 077 664**

- **CHEMICAL ABSTRACTS, vol. 88, no. 5, 30. Januar 1978, Columbus, Ohio, US; abstract no. 33686, G. PAPKE AND H. BLOBEL 'Purification of staphylokinase' Seite 178 ;**
- **BIOLOGICAL ABSTRACTS vol. 85, no. 6 , 1988, Philadelphia, PA, US; abstract no. 57275, D. BEHNKE AND D. GERLACH 'Cloning and expression in E. coli, B. subtilis and Streptococcus sanguis of a gene for staphylokinase' Seite 416 ;**
- **S. FERRONE AND M. DIERICH 'Handbook of monoclonal antibodies' 1988 , NOYES PUBLICATIONS , PARK RIDGE, NJ, USA**
- **TIBTECH (1992) 10: 310-315**

Bemerkungen:
Die Akte enthält technische Angaben, die nach dem Eingang der Anmeldung eingereicht wurden und die nicht in dieser Patentschrift enthalten sind.

EP 0 621 899 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die vorliegende Erfindung betrifft Signalpeptid-frei exprimierte Formen des bakteriellen Plasminogenaktivators Staphylokinase (Kurzbezeichnung: SAK), einschließlich neuer Staphylokinase-Formen, sowie Verfahren zu ihrer Herstellung auf gentechnologischem Wege und ihre Verwendung. Die Erfindung schließt somit DNA-Sequenzen, die für die erfindungsgemäßen SAK-Formen kodieren, rekombinante Plasmide, auf denen diese DNA-Sequenzen mit hochwirksamen Expressionssignalen gekoppelt vorliegen, ausgewählte rekombinante Wirtszellen, die nach Einfügung jeweils eines dieser Expressionsplasmide unter Kultivierungsbedingungen zur Biosynthese der erfindungsgemäßen SAK-Formen befähigt sind, sowie Teilverfahren zu dieser Kultivierung und zur Isolierung der Staphylokinase-Formen (Ziel-Polypeptide) aus den erhaltenen Kulturbrühen ein.

Staphylokinase, ein Polypeptid aus *Staphylococcus aureus*-Stämmen, ist, wie man seit den Untersuchungen von CH. LACK (Nature, **161** (1948), 558-560) sowie von K. C. ROBBINS et al. (J. Biol. Chem., **242** (1967), 2333-2342) und von K. W. JACKSON et al. (Methods in Enzymology, **80** (1981), 387) weiß, in der Lage, die Umwandlung des im menschlichen bzw. tierischen Blutplasma lokalisierten Proenzyms Plasminogen in das fibrinolytisch aktive Enzym Plasmin zu vermitteln, ohne selbst proteolytische Aktivität aufzuweisen. Der Mechanismus der Aktivierung ist hierbei noch weitgehend ungeklärt.

Die erfindungsgemäßen Staphylokinase-Formen sind somit als pharmazeutische Präparate in der Human- sowie gegebenenfalls in der Veterinärmedizin bei der Behandlung von thromboembolischen Gefäßerkrankungen geeignet, während die anti-SAK-mAK sowohl bei der Wirkstoffgewinnung als auch, wie unten näher erläutert, als pharmazeutische Präparate Einsatz finden können.

Staphylokinase (Moleküllänge: 136 Aminosäuren) gehört zu der Gruppe jener Substanzen, die eine Aktivierung des fibrinolytischen Systems über die Umwandlung von Plasminogen zu Plasmin und dadurch bedingte Spaltung des Fibrins bewirken können. Die bekanntesten endogenen Aktivatoren sind der Human-Gewebeplasminogenaktivator (t-PA) und die Urokinase. Ein anderer Plasminogenaktivator bakteriellen Ursprungs ist die Streptokinase. Die genannten Stoffe werden bereits therapeutisch zur Behandlung von Patienten mit thromboembolischen Gefäßerkrankungen eingesetzt. Da die Inzidenz dieser Erkrankungen hoch ist, besteht ein erheblicher Bedarf an Substanzmengen mit hohem Reinheitsstandard.

Anders als etwa für Streptokinase sind mit Hilfe der konventionellen technischen Mikrobiologie für SAK keine industriellen Herstellungsverfahren entwickelt worden, obwohl dieser bakterielle Plasminogenaktivator beispielsweise die Vorteile einer

- hohen Proteinstabilität gegenüber thermischen sowie chemischen Einflüssen,
- Tertiärstruktur ohne Disulfidbrücken und
- geringen Molekülgröße (15,8 kD)

aufweist. Dieser Sachverhalt hatte seine Ursache in erster Linie darin, daß durch die *Staphylococcus aureus*-Produzenten-Stämme eine große Zahl von toxischen Beiprodukten gebildet wird.

Nach der Einführung der rekombinanten DNA-Technik wurde die Synthese von SAK durch rekombinante Wirtsorganismen möglich, so daß eine technisch effiziente Erzeugung pharmazeutischer Präparationen mit diesem Polypeptid als Wirkstoff realisierbar war.

Im Stand der Technik sind zwei für Staphylokinase kodierende Gene beschrieben, die aus den Genomen von *Staphylococcus aureus*-Phagen isoliert worden sind. Als Gendonoren wurden dabei einerseits der *S. aureus*-Phage C (T. SAKO et al., Mol. Gen. Genet., **190** (1983), 271-277; T. SAKO/N. TSUCHIDA, Nucl. Acids Res., **11** (1983), 7679-7693; vgl. EP 0 077 664) und andererseits der *S. aureus*-Phage 42D (vgl. DD 245 444 sowie D. BEHNKE/D. GERLACH, Mol. Gen. Genet., **210** (1987), 528-534) verwendet. Die Gene wurden zunächst jeweils auf dem *E. coli*-Plasmid pBR322 primärkloniert und nach verschiedenen Subklonierungsschritten mittels DNA-Sequenzanalyse identifiziert.

Kürzlich ist die Isolierung eines weiteren Staphylokinase-Gens beschrieben worden (D. COLLEN et al., Fibrinolysis, **6** (1992), 226-231). Dieses als STAR bezeichnete Gen ist aus chromosomaler DNA eines *S. aureus*-Stammes gewonnen worden. Alle diese Gene stellen natürliche allelische Varianten der Staphylokinase dar.

Das für das SAK-C Polypeptid kodierende Gen wurde nach Umklonierung in ein *E. coli*-Expressionssystem in dem *E. coli*-Stamm WA802 überexprimiert (EP 0 077 664; T. SAKO, Eur. J. Biochem., **149** (1985), 557-563).

Das für SAK42D kodierende Gen gemäß Seq. Id. Nr. 9 hingegen wurde sowohl auf gramnegative als auch in grampositive Plasmidvektoren kloniert und in *Escherichia coli, Bacillus subtilis* sowie *Streptococcus sanguis* exprimiert (siehe wiederum DD 245 444 und D. BEHNKE/D. GERLACH, Mol. Gen. Genet., **210** (1987), 528-534).

Das *sak*-STAR-Gen wurde bisher nur unter der Kontrolle seiner natürlichen Signale in *E. coli* exprimiert (D. COLLEN et al., Fibrinolysis, **6**, (1992), 203-213).

Die drei Gene *sak*42D*, sak*-C und *sak*-STAR wurden, wie aus den angegebenen Schriften hervorgeht, bislang ausschließlich in ihrer natürlichen Form zur Expression gebracht, d. h. als Primärprodukt lagen Signalpeptid-tragende Präpolypeptide vor. Die Synthese solcher Präpolypeptide mit Signalsequenzen bedingt die Ausschleusung der gebildeten Produkte ins Periplasma oder in das umgebende Kulturmedium. Diese Vorgehensweise hat die wesentlichen Nachteile, daß bei einer Ausschleusung ins Kulturmedium, wie sie bei Verwendung grampositiver rekombinanter Wirtszellen stattfindet, die SAK-Polypeptide der Einwirkung der extrazellulären Proteasen dieser Zellen ausgesetzt sind, so daß es zu einer schnellen Zerstörung der Polypeptide kommt.

Bei der Verwendung von coliformen Wirtsbakterien, wie sie insbesondere in der EP 0 077 664 beschrieben ist, wird dieser Nachteil dadurch zu umgehen versucht, daß das Ziel-Polypeptid in den periplasmatischen Raum ausgeschleust wird, wodurch gewissermaßen ein Containment erreicht wird, das Schutz vor proteolytischer Degradation und einfacheren Zugriff auf das Ziel-Polypeptid beim Zellaufschluß bieten soll. Dieser Expressionsmodus bringt jedoch andere Nachteile mit sich, die nun ihrerseits wieder eine technisch effiziente Herstellung der SAK-Ziel-Polypeptide behindern. Dies hat folgende Ursachen:

- Die Ausschleusung großer Mengen an Ziel-Polypeptiden (wie sie für eine technisch effiziente Produktherstellung nötig sind) ins Periplasma gramnegativer Wirtszellen überfordert den sekretorischen Apparat dieser Zellen und führt zum physiologischen Zusammenbruch der Mikroorganismen.

- Im Kultivierungsschritt des Gesamtprozesses wird daher die für die Expression verfügbare Zeitdauer und damit die erreichbare Ausbeute an den jeweiligen Ziel-Polypeptiden stark verringert.

- Ein reproduzierbarer Fermentationsablauf wird erheblich erschwert und

- als Resultat dieses Biosynthesemodus ensteht ein heterologes Gemisch aus reifen SAK-Polypeptiden und SAK-Präpolypeptiden.

Gemäß EP 0 077 664 wird die SAK-C unter Nutzung ihrer natürlichen Expressionssignale in heterologen Wirten exprimiert, was eine zu geringe Expressionshöhe für ein Verfahren mit ausreichender technischer Effizienz zur Folge hat. Die thermische Überexpression von SAK-C wie sie von T. SAKO (Eur. J. Biochem., **149** (1985), 557-563) vorgeschlagen wird, ist neben den oben angeführten Problemen zusätzlich durch den Nachteil gekennzeichnet, daß eine thermisch induzierte Protein-Biosynthese unter technischen Fermentationsbedingungen - wenn überhaupt - sehr aufwendig und nur unpräzise zu handhaben ist.

Die synthetisierte SAK42D wird gemäß DD 245 444 hauptsächlich aus *B. subtilis*-Kulturüberständen isoliert. Bei diesem Verfahren werden die Expression und die Sekretion unter Nutzung der entsprechenden nativen Kontrollsequenzen des *sak*42D-Gens durch das rekombinante Plasmid pDB15 vermittelt. Auch dieses Verfahren weist jedoch eine unbefriedigende Expressionshöhe auf; weiterhin ist es durch eine Heterogenität des exprimierten SAK-Polypeptids sowie durch einen schnellen Abbau des Ziel-Polypeptids im Kulturmedium nach längerer Kultivierung gekennzeichnet (vgl. D. GERLACH et al., Zbl. Bakt. Hyg., **A269** (1988), 314-322).

Auch die weitere als SAK-STAR bekannte natürliche allelische Form der SAK ist bisher nur unter Verwendung ihrer natürlichen Signale in *E. coli* heterolog synthetisiert worden. (D. COLLEN et al., Fibrinolysis, **6**, (1992) 203-213), was zu den gleichen Problemen wie oben für SAK-C dargelegt führt.

Die bisher beschriebenen Verfahren zur Herstellung von SAK-C, SAK42D oder SAK-STAR sind demgemäß ausnahmslos an die Expression der vollständigen natürlichen Gensequenzen gebunden und zwangsläufig mit den oben erläuterten Nachteilen behaftet.

Auf dem Hintergrund des oben beschriebenen Standes der Technik liegt der Erfindung somit die Aufgabe zugrunde, Verfahren zur technologisch effizienten Biosynthese von SAK-Polypeptiden zu schaffen, die es gestatten, sowohl hochreine als auch homogene Präparationen des jeweiligen Ziel-Polypeptids herzustellen, welche für einen Einsatz in der Human- bzw. Veterinärmedizin bei der Therapie von thromboembolischen Gefäßerkrankungen geeignet sind.

Zur Lösung dieser Aufgabe wird vorgeschlagen, bei der rekombinanten Herstellung von Staphylokinase-Polypeptiden mit Plasminogenaktivator-Wirkung solche diese Polypeptide kodierenden DNA-Sequenzen - d. h. solche *sak*-Gene - einzusetzen, die frei sind von Regionen, welche für Signalpeptide kodieren. Durch die Entfernung der Nukleotidsequenzen, die für die Signalpeptide kodieren, werden überraschenderweise die nachfolgend erläuterten, erfindungsgemäßen Vorteile erreicht, welche erstmals eine technisch effiziente Herstellung der SAK-ziel-Polypeptide ermöglichen.

Die erfindungsgemäß hergestellten Signalpeptid-freien Polypeptide sammeln sich im Zellinneren und es hat sich überraschenderweise gezeigt, daß dies nicht zu den erwarteten Komplikationen, wie insbesondere zur Bildung von unlöslichen, biologisch inaktiven Proteinaggregaten führt (vgl. Kane, J. F. et al., TIBTECH **6**, S. 95 (1988), Mitraki, A. et al., Bio/Techn. **7**, S.690 (1989). Vielmehr liegen die intrazellulär exprimierten SAK-Ziel-Polypeptide überraschender Weise in löslicher und biologisch aktiver Form vor, so daß der technische Reinigungsprozeß ohne komplizierte und kostenaufwendige De- und Renaturierungsschritte möglich wird;
Mit dem erfindungsgemäßen Verfahren werden somit die nachfolgend angegebenen Vorteile erreicht:

- Die exprimierten SAK-ziel-Polypeptide sind intrazellulär lokalisiert und damit technischen Prozessen der Produktkonzentration leicht zugänglich;

- die SAK-ziel-Polypeptide liegen in homogener Form vor, da keine Präpolypeptide mehr gebildet werden;

- die intrazelluläre Überexpression der SAK-Ziel-Polypeptide gestattet eine lange Produktsynthesephase; ein physiologischer Zusammenbruch der Zellen bleibt aus, so daß eine hohe Ausbeute an SAK-Ziel-Polypeptiden (10 bis 15% des Gesamtproteins der Zellen) möglich wird;

- die chemische Induktion der Überexpression der SAK-Ziel-Polypeptide, wie sie mit den erfindungsgemäßen Expressionvektoren möglich ist, gestattet eine gute und einfache technische Handhabbarkeit während des Fermentationsprozesses.

Darüberhinaus hat sich gezeigt, daß die erfindungsgemäß intrazellulär exprimierten SAK-Polypeptide kein N-terminales Methionin aufweisen und so von den natürlich exprimierten reifen sekretorischen SAK-Polypeptiden nicht zu unterscheiden sind.

Zusätzlich zu diesen Vorteilen der Signalpeptid-freien Überexpression der SAK-Ziel-Polypeptide weisen die erfindungsgemäßen Ziel-Polypeptide der artifiziellen allelischen SAK-Varianten gemäß Seq. Id. Nr. 6 und 7 sowie der SAK-Fragmente gemäß Seq. Id. Nr. 4 und 5 solche vorteilhaften Eigenschaften wie erhöhte spezifische Aktivität bei der Plasminogenaktivierung, eine schnellere Kinetik der Plasminogenaktivierung und /oder ein geringeres Molekulargewicht und damit eine niedrigere Antigenität auf. Diese Eigenschaften haben insbesondere für den Einsatz der SAK-Ziel-Polypeptide zur Behandlung thromboembolischer Erkrankungen bei Mensch und Tier eine besondere Bedeutung.

Die erfindungsgemäß verwendeten DNA-Sequenzen sind somit grundsätzlich dadurch gekennzeichnet, daß

- sie für Signalpeptid-freie reife native Staphylokinase sowie für allelische SAK-Varianten oder SAK-Fragmente, die gleichermaßen die bekannte Plasminogenaktivator-Wirkung des nativen Moleküls aufweisen, kodieren sowie zusätzlich dadurch, daß

- ihrem 5'-Ende jeweils unmittelbar das Translations-Initiations-Kodon ATG vorgeschaltet ist.

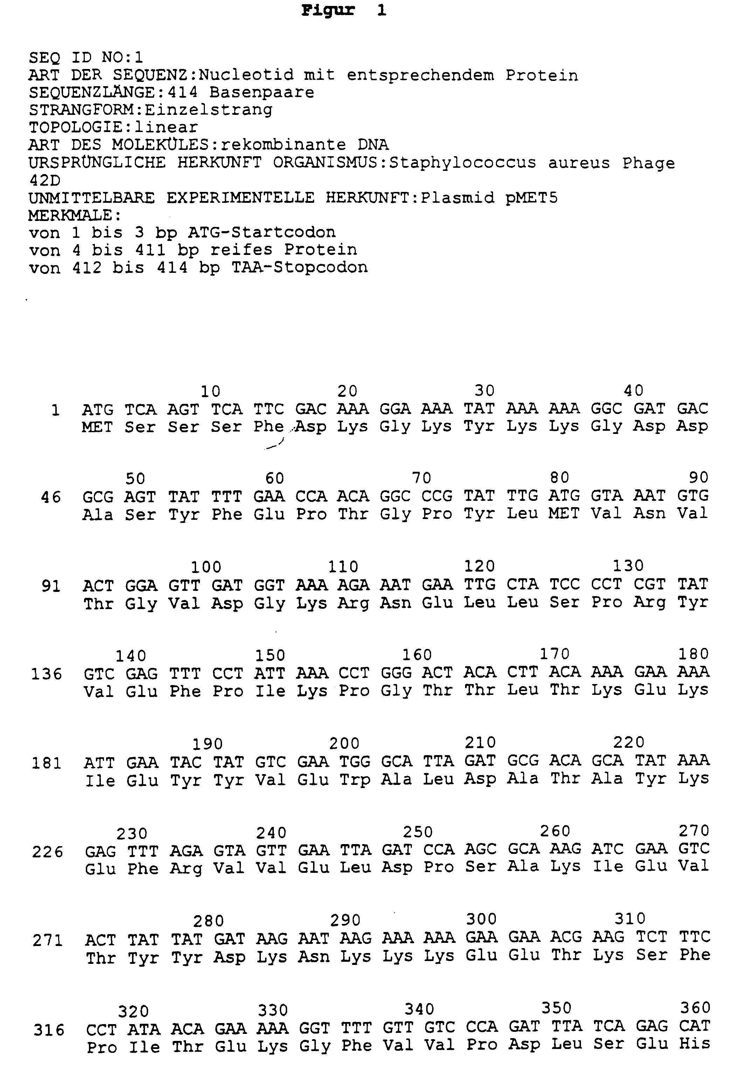

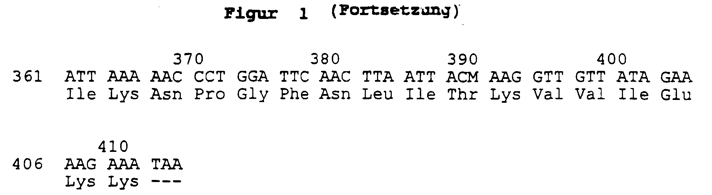

Bevorzugt werden zur Lösung der Aufgabe der Erfindung solche DNA-Sequenzen des oben erläuterten Typs eingesetzt, die entweder die Nukleotidfolge gemäß Figur 1 (Seq. Id. Nr. 1) oder die Nukleotidfolgen von allelischen Varianten bzw. Fragmenten der Folge gemäß Seq. Id. Nr. 1 aufweisen. Insbesondere werden zu diesem Zweck - neben der Nukleotidfolge gemäß Figur 1 - auch die Nukleotidfolgen nach Seq. Id. Nr. 2 und Seq. Id. Nr. 3 herangezogen, die natürliche allelische Varianten der primären Nukleotidfolge gemäß Figur 1 sind.

Die Nukleotidfolge gemäß Figur 1 (Seq. Id. Nr. 1) stellt das natürliche *sak*42D-Strukturgen dar, erfindungsgemäß

- am 5'-Ende nunmehr mit dem ATG-Startkodon sowie
- am 3'-Ende mit dem Terminationskodon TAA

verknüpft.

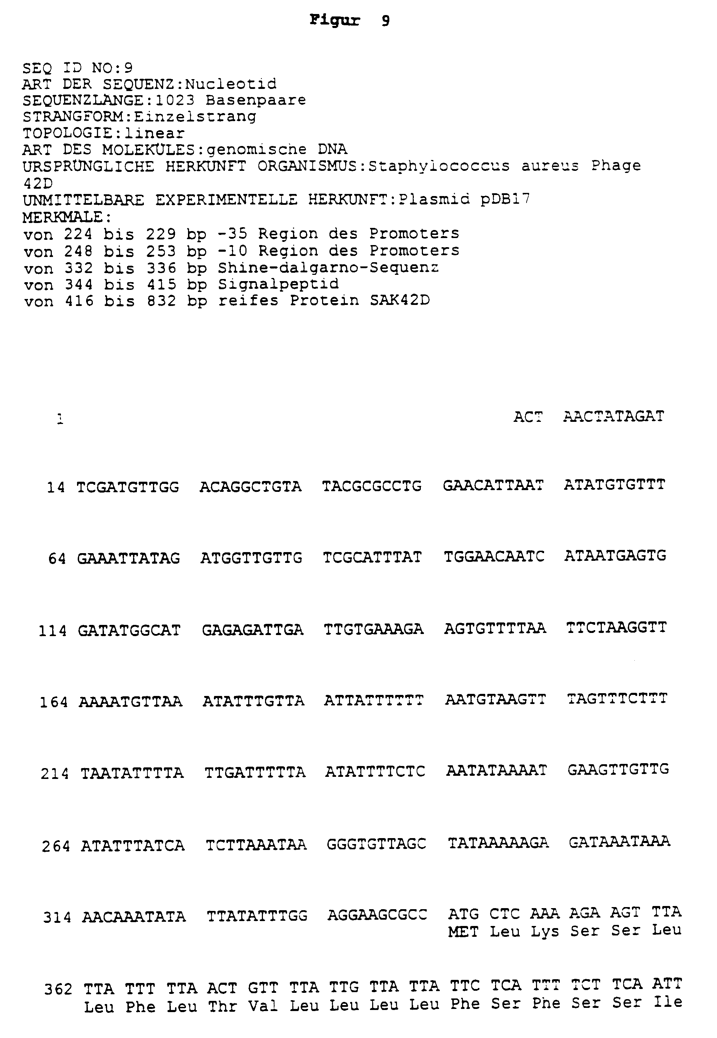

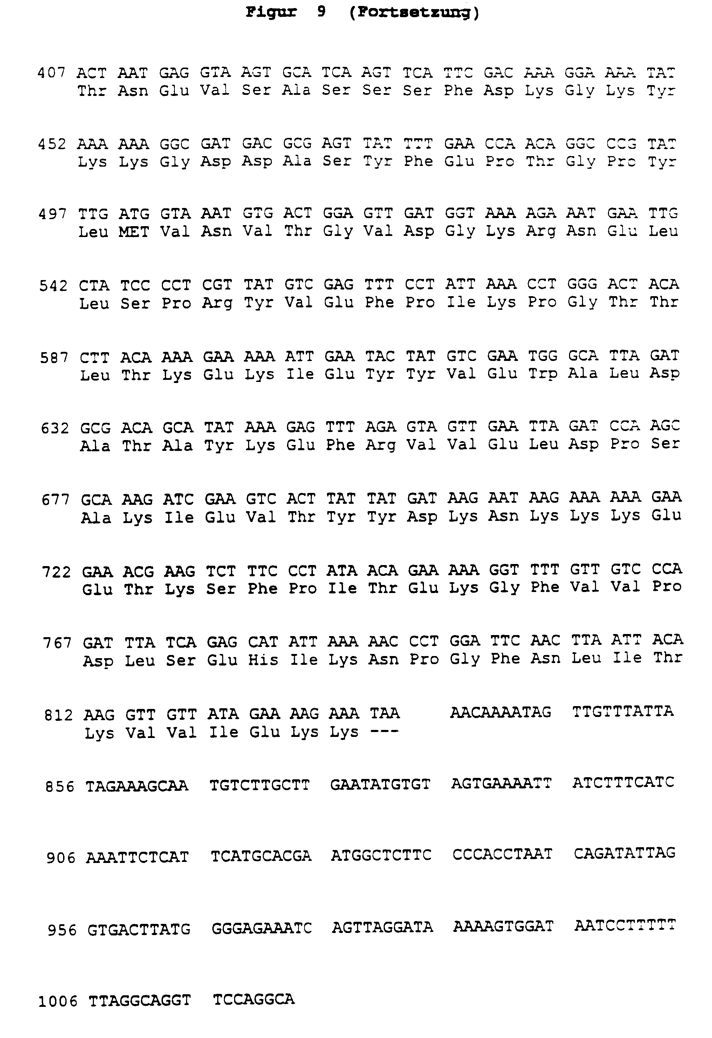

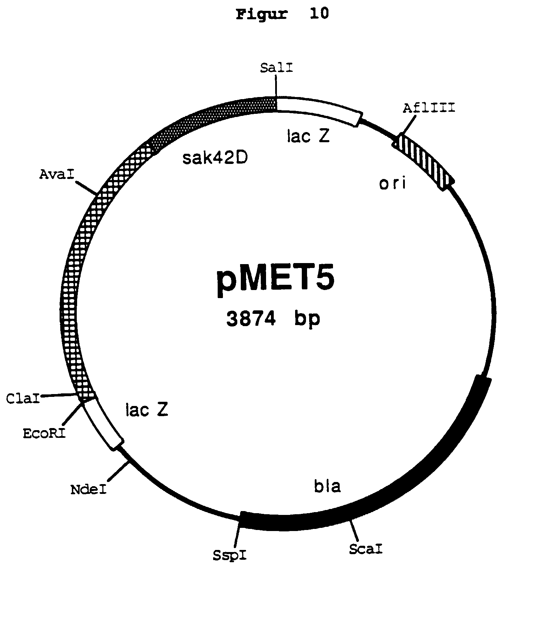

Das *sak*42D-Strukturgen kann für die Belange der vorliegenden Erfindung auf dem rekombinanten Ausgangsplasmid pMET5 (Figur 10) bereitgestellt werden. Das Plasmid pMET5 - ein Derivat des gebräuchlichen Klonierungsvektors pUC19 - trägt einen DNA-Abschnitt, der für das sak42D-Gen kodiert und der aus einem der aus der Literatur bekannten Plasmide der pDB-Familie (siehe DD 245 444 oder D. BEHNKE/D. GERLACH, Mol. Gen. Genet., **210** (1987), 528-534), vorzugsweise aus pDB15 oder pDB17, mittels an sich bekannter Verfahrensschritte der rekombinanten DNA-Technik isoliert worden ist. Im Detail trägt das Plasmid pMET5 den in der Figur 9 (Seq. Id. Nr. 9) durch die Nukleotidfolge 436 bis 1023 gekennzeichneten Abschnitt des nativen *sak*42D-Gens. Am 5'-Ende fehlen diesem Abschnitt im Vergleich zur *sak*42D-Sequenz (vgl. die vollständige Sequenz wie in Seq. Id. Nr. 9 angegeben) beispielsweise auf pDB17 die nativen 5'-flankierenden regulatorischen Bereiche, die kodierende Sequenz für das Signalpeptid sowie die Kodons für die ersten vier Aminosäuren der reifen SAK42D; letztere sind durch einen TaqI-Restriktaseschnitt vom ursprünglichen Gen abgetrennt worden (an diesem 5'-Ende ist in der pMET5-DNA stattdessen ein unikaler SalI-Spaltort gebildet worden.). Stromabwärts der für SAK42D kodierenden Sequenz sind auf pMET5 jedoch wie auf pDB17 noch die 3'-flankierenden nichtkodierenden Bereiche der primär isolierten *sak*42D-DNA sowie Abschnitte der DNA des *E. coli*-Plasmids pBR322 enthalten.

Für die Belange der vorliegenden Erfindung kann die Rekonstitution des *sak*42D-Genabschnitts von Plasmid pMET5 zur Signalpeptid-frei kodierenden Sequenz für reife native SAK42D, wie sie in der Figur 1 (Seq. Id. Nr. 1) dargestellt ist, durch Anlagerung eines chemisch synthetisierten Linkerpaares mit der entsprechenden spezifischen Nukleotidfolge erfolgen.

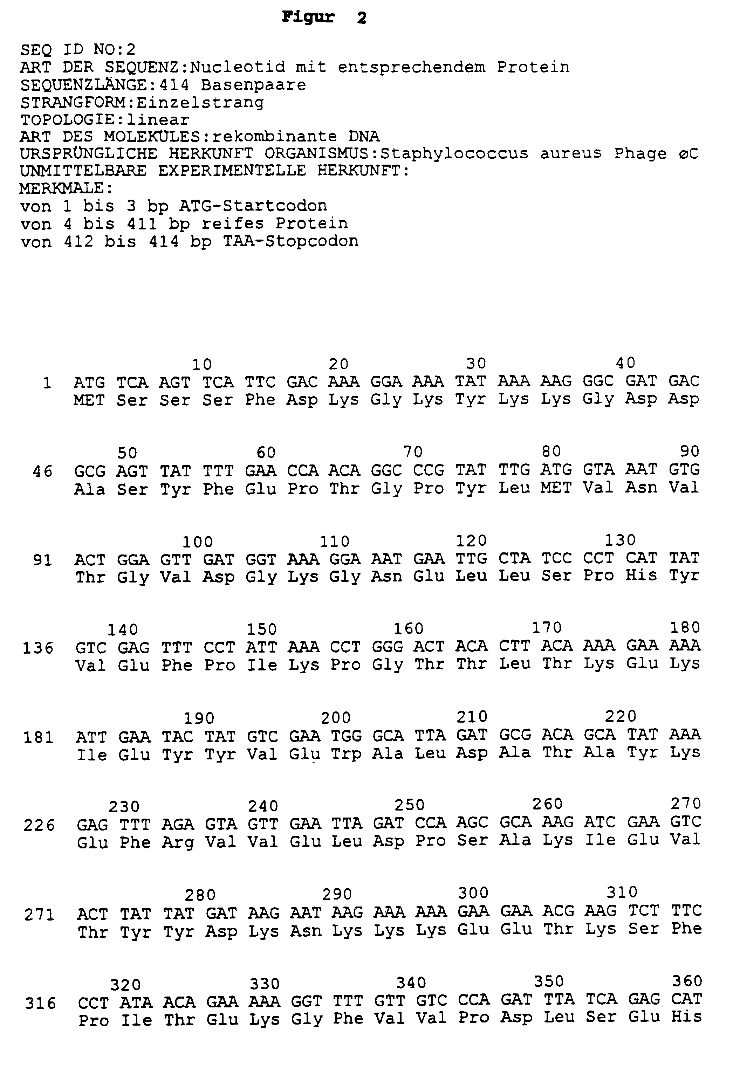

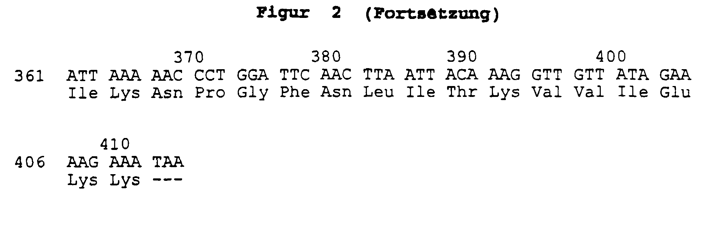

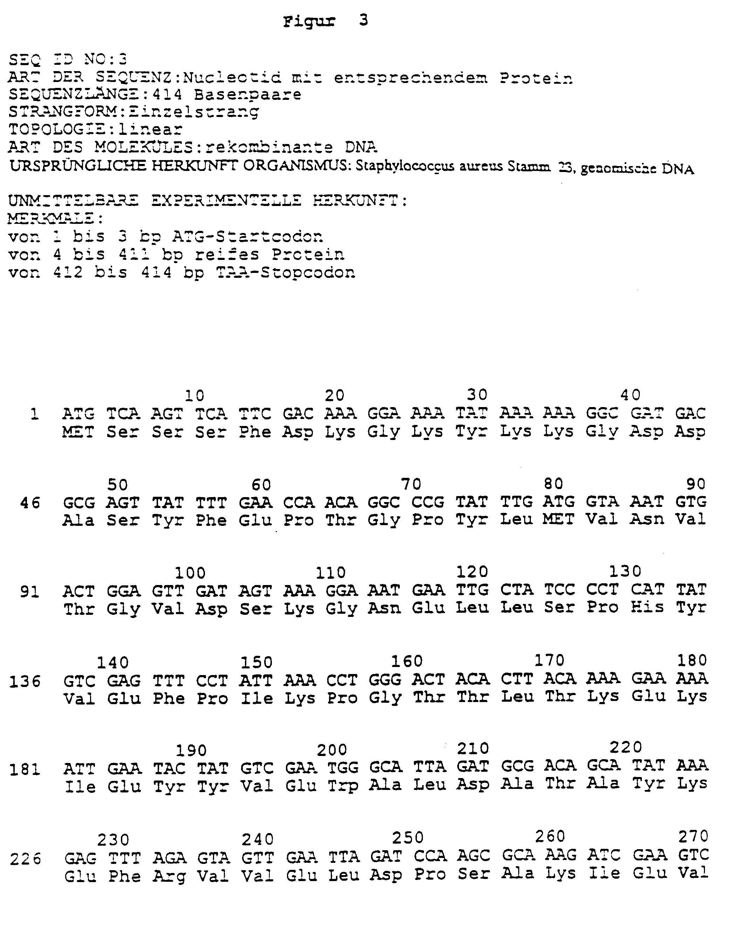

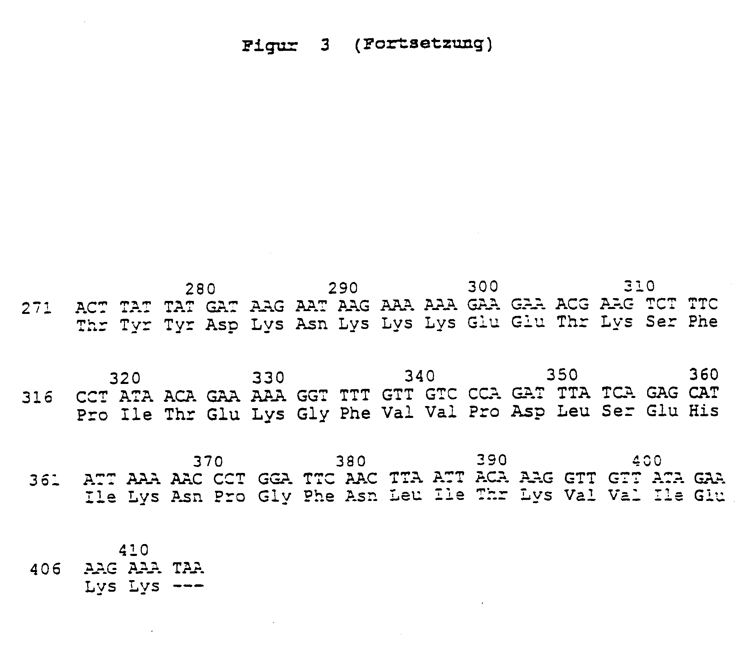

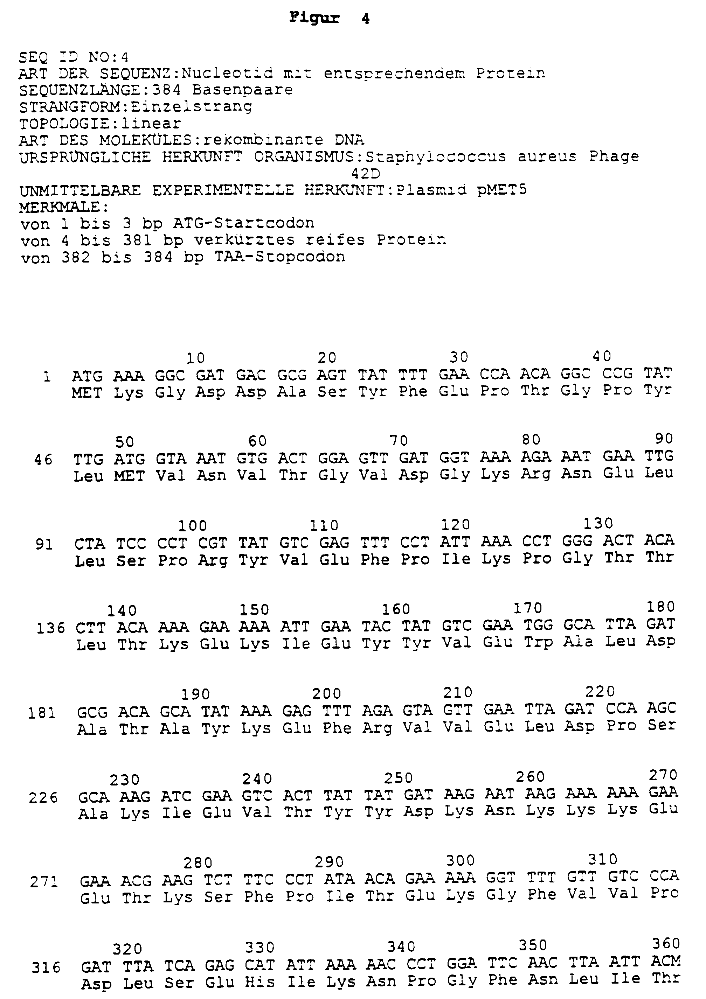

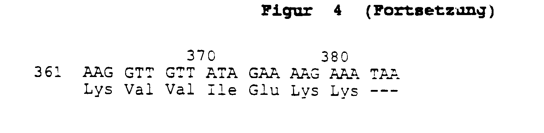

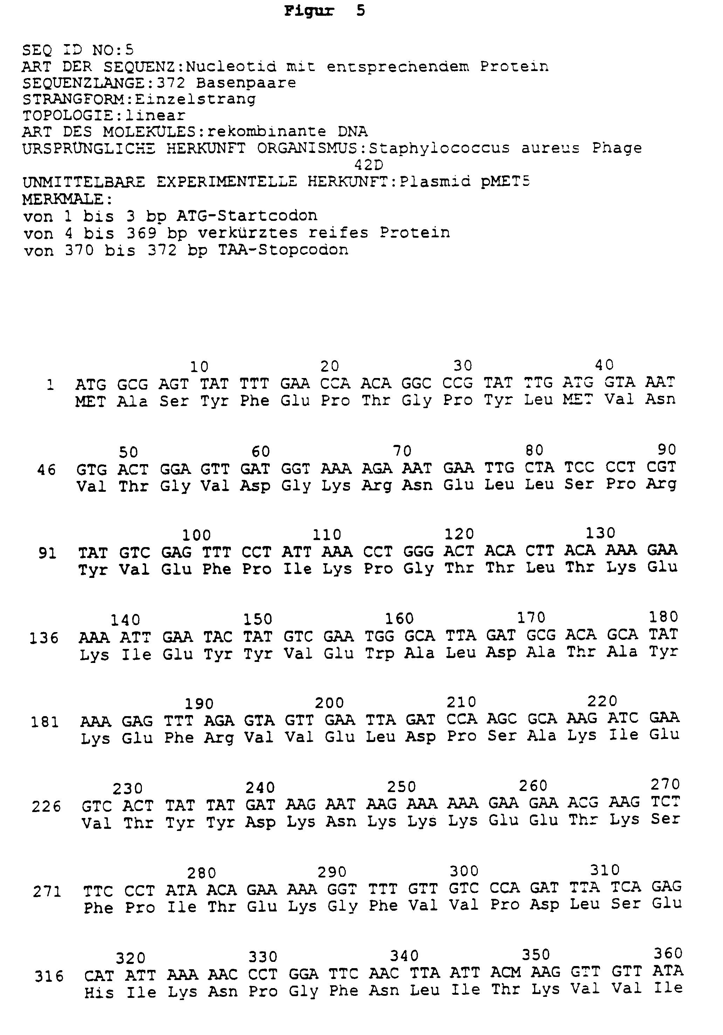

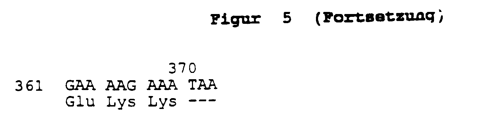

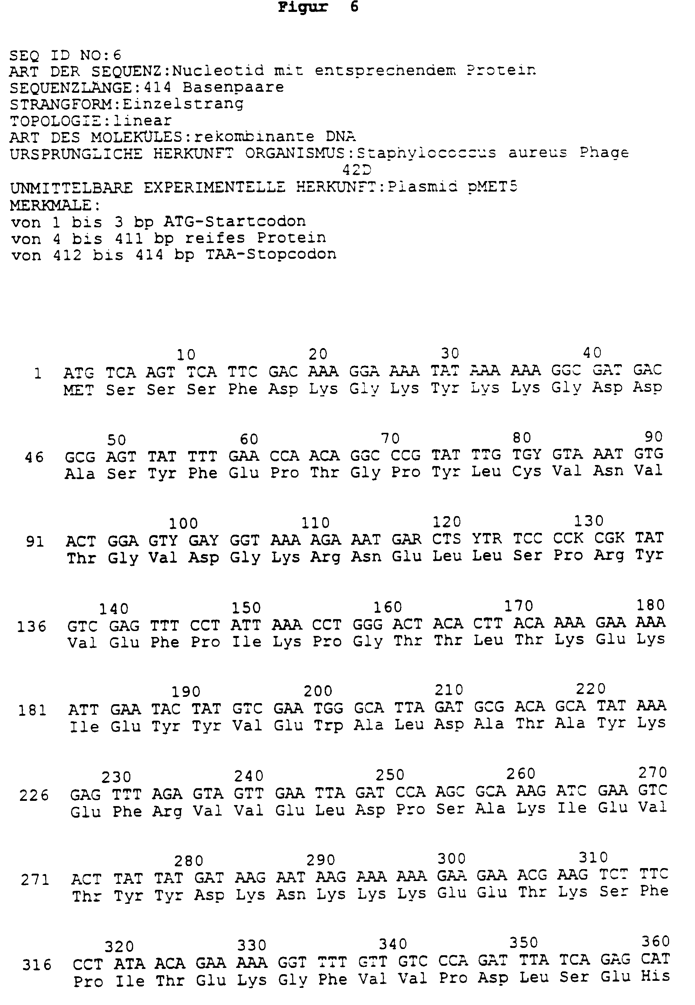

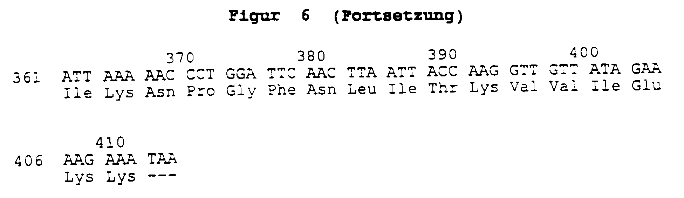

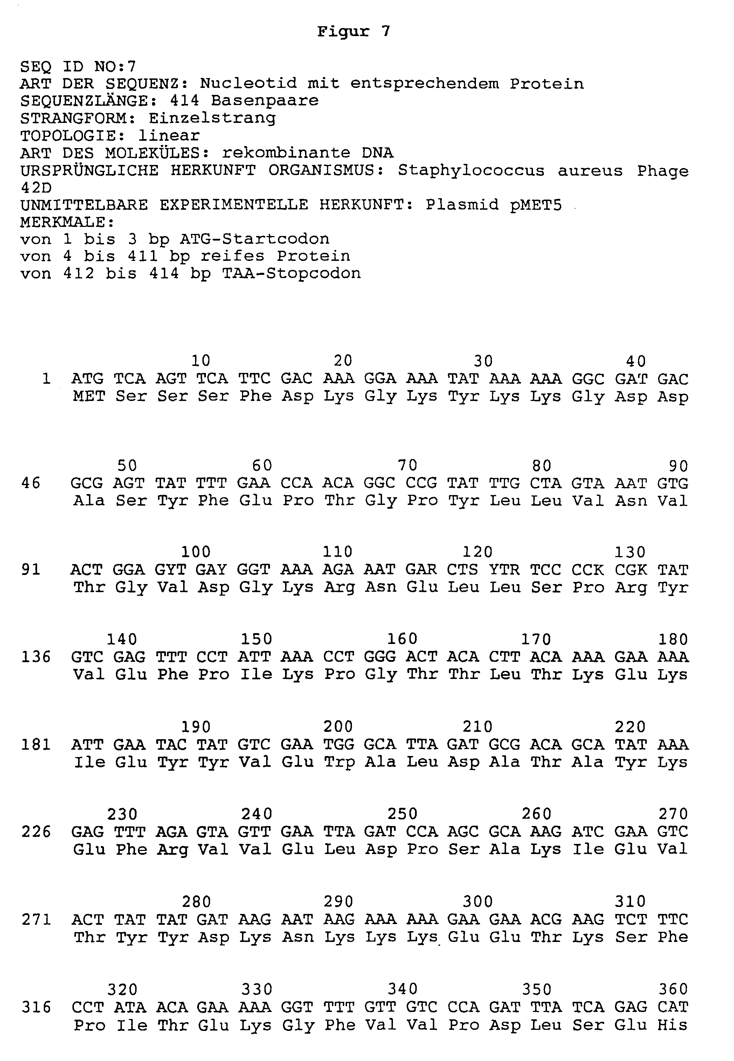

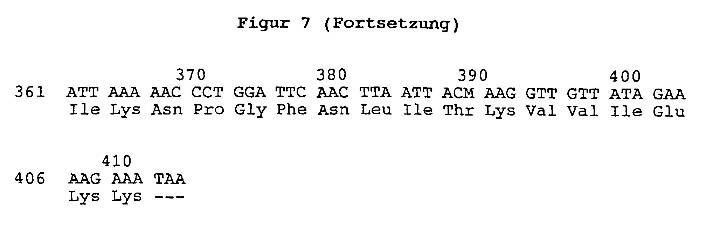

In analoger Weise sind aus dem aus Plasmid pMET5 entnehmbaren *sak*42D-DNA-Abschnitt durch an sich bekannte Einzelschritte der rekombinanten DNA-Technik, welch die Verknüpfung von chemisch synthetisierten Linker-Molekülen ausdrücklich einschließen, die erfindungsgemäßen weiteren natürlichen und artifiziellen allelischen Formen und Fragmente der *sak*-DNA-Sequenzen hergestellt worden, insbesondere

a) jene beiden DNA-Sequenzen mit den Nukleotidfolgen gemäß den Figuren 2 und 3 (Seq. Id. Nrn. 2 und 3), die - wie erwähnt eingebettet zwischen ATG-Startkodon und Terminationskodon - die Strukturgene für die bisher bekannten natürlichen allelischen Varianten des nativen reifen SAK-Polypeptids sind, weiterhin

b) jene beiden DNA-Sequenzen mit den Nukleotidfolgen gemäß den Figuren 4 und 5 (Seq. Id. Nrn. 4 und 5), welche die Signalpeptid-freien Strukturgene für neuartige N-terminal verkürzte Fragmente des nativen reifen Polypeptids SAK42D sind, sowie schließlich

c) jene beiden DNA-Sequenzen mit den Nukleotidfolgen gemäß den Figuren 6 und 7 (Seq. Id. Nrn. 6 und 7), die Signalpeptid-freien Strukturgene für neuartige artifizielle allelische Varianten des nativen reifen Polypeptids SAK42D darstellen.

Die voranstehend beschriebenen DNA-Sequenzen, insbesondere die im Sequenzprotokoll unter den Seq. Id. Nrn. 1 bis 7 angeführten DNA-Sequenzen, können auf an sich bekannte Weise über geeignete Vektoren in prokaryotische oder eukaryotische Wirtszellen eingebracht und dort als Signalpeptid-freie Staphylokinase-Formen mit Plasminogenaktivator-Wirkung exprimiert werden.

Ergänzend zu den oben angegebenen DNA-Sequenzen, vorzugsweise zu den im Sequenzprotokoll unter den Seq. Id. Nrn. 1 bis 7 angegebenen DNA-Sequenzen, sind in diesem Sinne in den Gegenstand der Erfindung eingeschlossen,

a1) solche DNA-Sequenzen, die mit den SAK-Polypeptid-kodierenden Bereichen der voranstehend beschriebenen DNA-Sequenzen, vorzugsweise mit den im Sequenzprotokoll unter den Seq. Id. Nrn. 1 bis 7 angeführten DNA-Sequenzen oder mit Fragmenten derselben, unter Standard-Bedingungen (s.u.) hybridisieren sowie

b1) solche DNA-Sequenzen, die mit Ausnahme der durch die Degeneration des genetischen Kodes verursachten Abweichungen, ihrerseits mit den unter a) genannten DNA-Sequenzen hybridisieren.

Erfindungsgemäß werden als Standard Bedingungen bezeichnet, bei denen die Hybridisierung bei Temperaturen zwischen 55 und 68°C und einer Salzkonzentration von 5 x SSC Puffer durchgeführt wird.

Zu den unter a) bzw. al) zusammengefaßten DNA-Sequenzen werden erfindungsgemäß namentlich solche gezählt, die die in den Nukleotidsequenzen gemäß Seq. Id. Nrn. 1 bis 7 eingeschlossenen sogenannten stillen Mutationen tragen, d. h. punktuelle Nukleotidaustausche aufweisen, welche jedoch keine Änderung in der Aminosäuresequenz der jeweils entsprechenden Staphylokinase-Form nach sich ziehen.

In der vorliegenden Erfindung werden sämtliche erfindungsgemäßen DNA-Sequenzen bevorzugt mittels Transformation in die geeigneten pro- und/oder eukaryotischen Wirtszellen eingebracht. Dementsprechend schließt die Erfindung insbesondere Expressionsplasmide ein, welche die erfindungsgemäßen DNA-Sequenzen operativ mit Expressionskontroll-Sequenzen verknüpft enthalten.

Erfindungsgemäß werden jene DNA-Abschnitte der Expressionsplasmide, in denen jeweils eine Kombination aus erfindungsgemäßem *sak*-Strukturgen und ATG-Startkodon sowie TAA-Terminationskodon an Expressionskontrollsequenzen gekoppelt ist, von Regionen freigehalten, die für Signalpeptide kodieren. Auf diese Weise gewährleisten die in der vorliegenden Erfindung eingesetzten *sak*-rekombinanten Expressionsplasmide die Signalpeptid-freie und damit intrazelluläre Expression der Ziel-Polypeptide in den jeweils verwendeten Wirtszellen.

Im Zusammenhang mit der Erfindung werden ausschließlich aus prokaryotischer DNA aufgebaute *sak*-rekombinante Expressionsplasmide verwendet, insbesondere solche, deren Expressionsboxen (Gesamtheit der Expressionssignale wie unten im Detail ausgeführt) die *sak*-Gene gemäß den Figuren 1 bis 7 (Seq. Id. Nr. 1 bis 7), operativ mit prokaryotischen Kontrollsequenzen verknüpft enthalten, wenn Wirtszellen prokaryotischer Herkunft für die Expression der erfindungsgemäßen SAK-Polypeptide vorgesehen sind.

Für die vorliegende Erfindung hat es sich als zweckmäßig erwiesen, in den Expressionsboxen der Vektoren - in Leserichtung gesehen - die folgende Kombination von prokaryotischen Kontrollsequenzen anzuordnen:

- *tac*-Promotor plus Bindungssequenz für den *lac*-Repressor,

- Translations-Initiations-Region, ausgeführt als Tandem-Anordnung von Shine-Dalgarno-Sequenzen mit definierten Spacern,

- Transkriptionsterminator.

(Kurzbezeichnung für eine derartige Kombination: R;tac;SD;T-Konfiguration).

In solche R;tac;SD;-;T-Konfigurationen werden die erfindungsgemäßen *sak*-Sequenzen, die - wie beschrieben - jeweils schon mit dem ATG-Startkodon sowie mit einem TAA-Kodon verknüpft sind, passgerecht eingefügt.

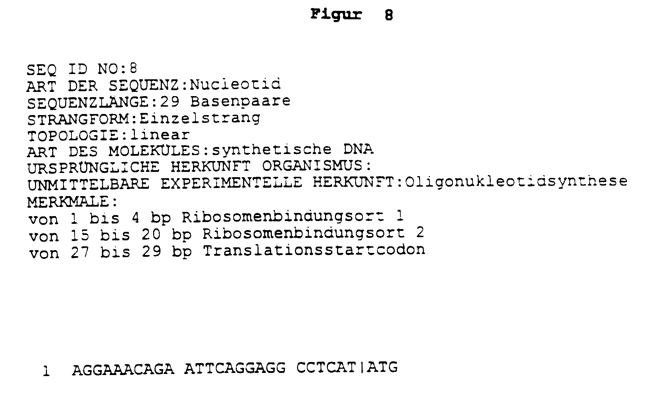

Im Rahmen der vorliegenden Erfindung lassen sich besonders hohe Expressionsleistungen bei der Einbeziehung von Boxen mit derartigen R;tac;SD;T-Konfigurationen dann erreichen, wenn sie als Translations-Initiations-Sequenz die DNA-Folge gemäß Figur 8 (Seq. Id. 8) enthalten.

Erfindungsgemäß hat es sich unter dem voranstehend beschriebenen Aspekt weiterhin als außerordentlich vorteilhaft erwiesen, in Kombination sowohl Expressionsplasmide aus bakterieller DNA als auch Bakterienzellen - vorzugsweise gram-negative Bakterien, insbesondere *E. coli* Stämme, aber auch Stämme der Gattung *Bacillus* - als Wirtszellen heranzuziehen. Mit derartigen bakteriellen Expressionssystemen ist eine hocheffiziente Expression der erfindungsgemäßen Signalpeptid-freien SAK-Ziel-Polypeptide zu erreichen. Besonders vorteilhafte Effekte konnten bei der Biosynthese der erfindungsgemäßen SAK-Ziel-Polypeptide in *E. coli*-Expressionssystemen erzielt werden. Für diese bevorzugt Ausführungsform der Erfindung wurden Expressionsplasmide konstruiert, die neben Merkmalen wie Replikationsorigin, selektiver Marker und Polylinkerbereich

a) Expressionsboxen mit einer R;tac;SD;T-Konfiguration aufweisen, in der die jeweils einbezogenen DNA-Sequenzen gemäß den Figuren 1 bis 7 (Seq. Id. Nrn. 1 bis 7) direkt mit der Translations-Initiations-Region gemäß Figur 8 (Seq. Id. Nr. 8) verknüpft sind, und

b) die sich von gebräuchlichen *E. coli*-Klonierungs- bzw. Expressionsvektoren ableiten, vorzugsweise von den pUC- und den pMEX-Vektoren.

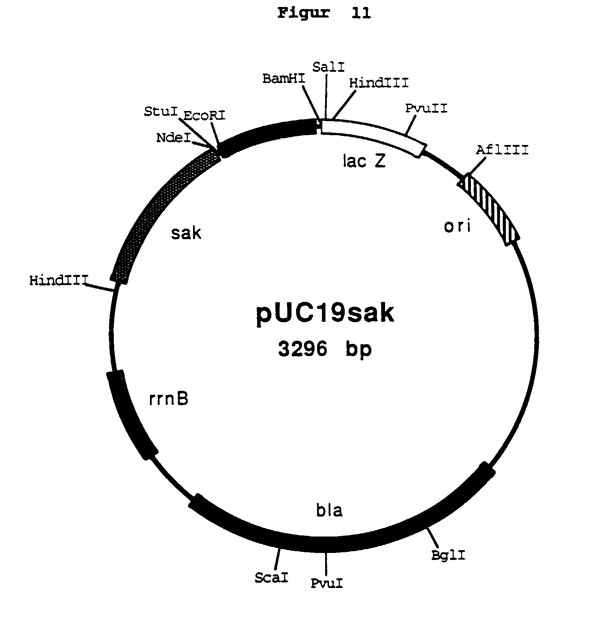

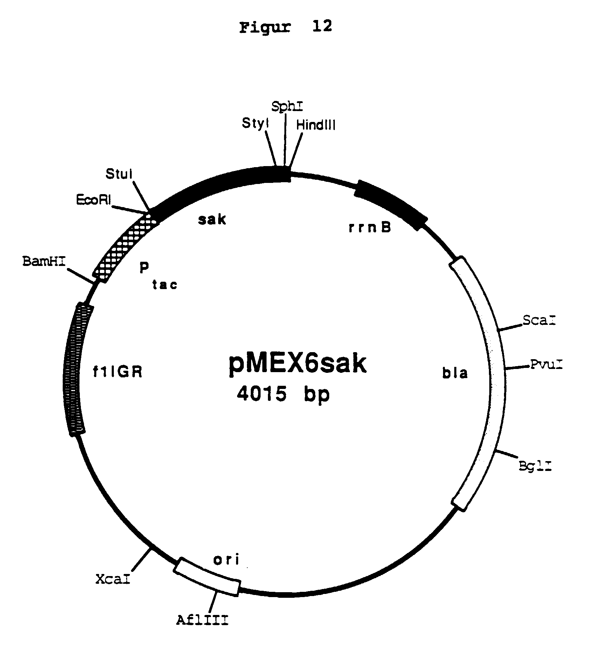

Beispiele für erfindungsgemäß hergestellte Expressionsplasmide, welche die *sak*-Gene tragen, sind (s. a. Figuren 11 und 12 und Beispiele 3, 4, 7, 8):

- pMEX602sak
- pMEx6ΔN10sak
- pMEx6ΔN14sak
- pMEX6sakM26C
- pMEX6sakM26L

Entsprechend kpönnen ebenfalls Plasmide des pUC-, pBR- und p 15A-Typs verwendet werden.

Mit diesen erfindungsgemäßen *E. coli*-Expressionsplasmiden wurden jeweils *E. coli*-Wirtszellen nach Standardprotokoll transformiert.

Für die intrazelluläre Expression der SAK-Ziel-Polypeptide lassen sich in diesem bevorzugten Expressionssystem *E. coli*-Rezipientenstämme wie beispielsweise JM101, K12 oder C600 einsetzen. Besonders gute Expressionsergebnisse konnten bei Verwendung von *E. coli* TG1-Wirtszellen erzielt werden.

Beispiele für erfindungsgemäß hergestellte rekombinante Stämme sind (vgl. Beispiele 3, 4, 7, 8)

*E. coli* TG1(pMEX602sak), *E. coli* TG1(pMEx6ΔN10sak), *E. coli* TG1(pMEX6ΔN14sak), *E. coli* TG1 (pMEX6sakM26C), *E. coli* TG1(pMEX6sakM26L),

Die oben genannten Stämme sind Beispiele für solche Wirtszellen, welche die erfindungsgemäßen Vorteile aufweisen, nämlich:

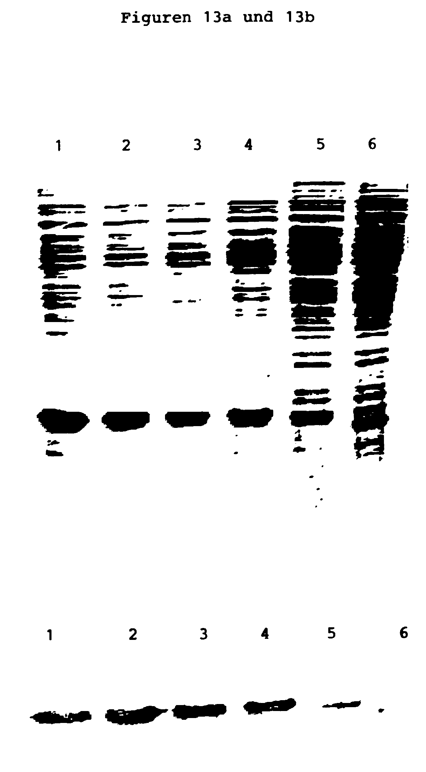

- sie exprimieren die erfindungsgemäßen *sak*-Gene korrekt und auf einem hohen Niveau (s. a. Figur 13a),

- sie realisieren eine vollständig intrazelluläre Produktbildung und reichern die Ziel-Polypeptide jeweils im Zytoplasma der Wirtszellen an (Ziel-Polypeptid wird nicht in den periplasmatischen Raum der Zellen ausgeschleust),

- sie synthetisieren und akkumulieren jeweils ein lösliches und biologisch aktives Ziel-Polypeptid,

- sie reagieren in Kultur effektiv auf den Algorithmus der chemischen Induktion und

- sie erweisen sich als ausreichend stabile rekombinante Mikroorganismen auch für Fermentationen im großen Maßstab sowie für Hochzelldichte-Verfahren, mit denen die erforderlichen hohen Ausbeuten an Ziel-Polypeptiden für eine effiziente technische Produktgewinnung erreicht werden können (bis zu 15% des Gesamtproteins der Wirtszellen).

Darüber hinaus wurde überraschenderweise gefunden, daß die erfindungsgemäß eingesetzten *E. coli* TG1-Stämme SAK-Formen synthetisieren, die in ihrer Aminosäuresequenz kein N-terminales Methionin aufweisen.

Die Kultivierung der erfindungsgemäßen *sak*-rekombinanten Wirtsszellen - insbesondere der rekombinanten *E. coli* TG1-Stämme - erfolgt unter sterilen und aerob-submersen Bedingungen in an sich bekannter Weise in den Stufen der Vor- sowie der Hauptkulturen in jeweils einem Nährmedium mit assimilierbaren Kohlenstoff- und Stickstoffquellen sowie mit einem definierten Gehalt an Mineralsalzen.

Gemäß einer besonders bevorzugten Ausführungsform der Erfindung werden die *sak*-rekombinanten *E. coli* TG1-Stämme einem Fermentationsverfahren unterworfen, welches zusätzlich dadurch gekennzeichnet ist, daß man

- die Kultivierung bei Temperaturen zwischen 28°C und 42°C für die Dauer von 4 Stunden bis 16 Stunden in einem Vollmedium oder einem sogenannnten synthetischen Medium, jeweils mit Zusatz von 50mg/l bis 100mg/l Ampicillin, durchgeführt und

- dabei 2 bis 4 Stunden nach Fermentationsbeginn die Biosynthese des rekombinanten Ziel-Polypeptids durch die Wirtszellen mittels Zugabe von Isopropylthiogalaktopyranosid (IPTG) zum Medium bis zu einer Endkonzentration von o,3 mmol/l eingeschaltet, wenn die Biomasse eine Zelldichte von ca. 0,2 bis 1,0 A600-Einheiten erreicht hat.

Vorzugsweise werden die SAK-Ziel-Polypeptide in folgender Weise aus den Wirtszellen isoliert: Die Wirtszellen werden aufgeschlossen, vorzugsweise mit Ultraschall, und die löslichen Anteile des Aufschlusses werden von den unlöslichen getrennt. Die löslichen Anteile werden danach direkt auf einen Ionenaustauscher geleitet. Im nächsten Schritt wird das Ziel-Polypeptid mit einer wässrigen NaCl-Lösung selektiv vom Ionenaustauscher eluiert. Das Eluat wird in vorteilhafter Weise nach einfacher Anreicherung mit NaCl direkt einer hydrophoben Interaktionschromatographie zugeleitet. Schließlich wird das Ziel-Polypeptid von dem hydrophoben Interaktionschromatographie-Medium mit hoher Selektivität durch einen absteigenden NaCl-Gradienten eluiert und in der Folge aus dem Eluat gewonnen. Diese Reinigung des Ziel-Polypeptids aus Lysaten rekombinanter *E. coli* TG1-Zellen ist in besonderer Weise effizient durch die einfache Möglichkeit der Pufferanpassung zwischen den beiden Reinigungsstufen.

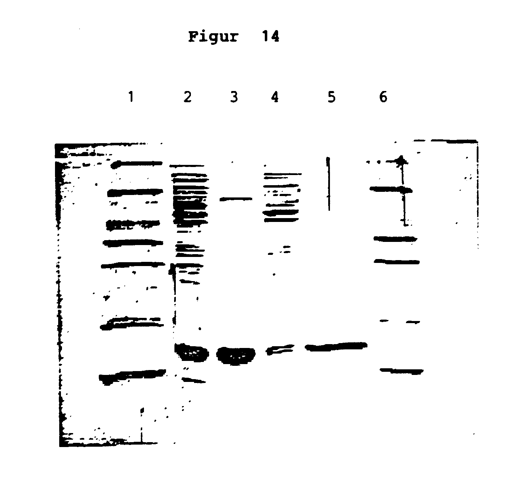

Das oben angegebene Aufarbeitungsverfahren ist besonders effizient, wenn die NaCl-Konzentrationen im ersten Elutionsschritt zwischen 30 und 500, vorzugsweise 200 bis 300 mM betragen, vor Zuleitung zur hydrophen Interaktionschromatographie auf 2,0 bis 3,0 M erhöht werden und entsprechend für den absteigenden NaCl-Gradienten eine Anfangskonzentration von 2,0 bis 3,0 M gewählt wird, die absteigend bis zu einer Endkonzentration von 15 bis 100mM geführt wird (siehe auch Figur 14).

Der oben beschriebene Aufarbeitungsprozeß erlaubt in nur zwei Chromatographieschritten eine gleichermaßen unaufwendige wie technisch leicht ausführbare Abtrennung der Ziel-Polypeptide. Letztere werden in diesem Verfahren in hochreiner Form und chromatographisch homogen erhalten. Der Reinigungsprozeß ist leicht auf den technischen Maßstab ausdehnbar. Dieser Prozeß erlaubt ferner eine schonende, verlustarme Aufarbeitung (ohne Deund Renaturierungsschritte) des im Zellrohlysat enthaltenen SAK-Ziel-Polypeptids. Besonders vorteilhaft wirkt sich dabei für den beschriebenen Reinigungsprozeß der Umstand aus, daß in der Fermentationsstufe der Erfindung die Ziel-Polypeptide nicht als Gemisch aus Prä-Polypeptiden und reifen Ziel-Polypeptiden vorliegen.

Neben den bereits bekannten natürlichen allelischen Staphylokinase-Formen wie SAK42D, SAK-C und SAK-STAR werden im Rahmen der Erfindung erstmals auch bestimmte neuartige SAK-Formen mit vorteilhaften Eigenschaften verfügbar gemacht, insbesondere

- die SAK-Fragmentform N10ΔSAK42D mit der Aminosäuresequenz gemäß Figur 4 (Seq. Id. Nr. 4),

- die artifizielle allelische SAK-Form SAKM26C mit der Aminosäuresequenz gemäß Figur 6 (Seq. Id. Nr. 6) sowie

- die artifizielle allelische SAK-Form SAKM26L mit der Aminosäuresequenz gemäß Figur 7 (Seq. Id. Nr. 7).

Mit den Fragmentformen N10ΔSAK42D und N14ΔSAK42D liegen N-terminal verkürzte SAK-Polypeptide mit Plasminogenaktivator-Wirkung vor; ihre Moleküllängen betragen 126 bzw. 122 Aminosäuren.

Die artifiziellen allelischen Formen SAKM26C und SAKM26L stellen erstmals Methionin-freie SAK-Formen mit Plasminogenaktivator-Wirkung dar; sie besitzen die Moleküllänge von 136 Aminosäuren.

Für die Fragmente wurde überraschenderweise eine beschleunigte Aktivierungskinetik für Plasminogen gefunden. Darüberhinaus läßt die verringerte Molekülgröße eine geringere Antigenität erwarten.

Für die artifiziellen allelischen Formen wurde neben einer zum Teil zweifach höheren spezifischen Aktivität (vgl. Tabelle 2) ebenfalls eine beschleunigte Aktivierungskinetik für Plasminogen gefunden. Diese neuen Eigenschaften machen die SAK-Fragmente bzw. die artifiziellen allelischen SAK-Formen besonders attraktiv für den therapeutischen Einsatz zur Behandlung von thromboembolischen Erkrankungen bei Mensch und Tier.

Die erfindungsgemäß hergestellten SAK-Polypeptide, sowohl die erstmals Signalpeptid-frei und damit intrazellulär synthetisierten natürlichen allelischen SAK-Formen als auch die neuen artifiziellen allelischen SAK-Formen bzw. -Fragmente, sind aufgrund ihrer Plasminogenaktivator-Eigenschaft ausgezeichnete Wirkstoffe zur Verwendung in pharmazeutischen Zusammensetzungen zur Behandlung von thromboembolischen Erkrankungen.

Insbesondere kommen für diesen Zweck solche pharmazeutischen Zusammmensetzungen in Frage, in denen eine oder mehrere nach dem erfindungsgemäßen Verfahren synthetisierte SAK-Formen in Kombination mit pharmazeutisch verträglichen Verdünnungsmitteln, Adjuvantien und/oder Trägerstoffen enthalten sind. Vorzugsweise sind die Formulierungen für die parenterale, in besonders bevorzugter Weise für die intravenöse Injektion vorgesehen. Bevorzugte Dosierungsmengen liegen im Bereich von 3 bis 100 mg Polypeptid pro Injektion.

In Verbindung mit der vorliegenden Erfindung sind in der Deutschen Sammlung für Mikroorganismen (DSM), Mascheroder Weg 1B, D-3300 Braunschweig, biologisch reine Proben von

| | |
|---|---|
| Plasmid pMET5 | (DSM 6841) |
| Hybridoma-Zellinie alpha-SAK IG8/H6 | (ZIM 0511) |
| | (DSM ) |
| Hybridoma-Zellinie alpha-SAK IIA4/A10 | (ZIM 0512) |
| | (DSM ) |

hinterlegt worden. Außerdem wurde auf folgende hinterlegte Plasmide bzw. Rezipientenstämme zugegriffen :

| | |
|---|---|
| Plasmidvektor pUC19 | (DMS 3425) |
| Rezipientenstamm *E. coli* TG1 | (DMS 6056). |

Die Erfindung wird nachfolgend anhand einer detaillierten Beschreibung von bevorzugten Ausführungsformen zur Konstruktion der erfindungsgemäßen *sak*-Gene, Expressionsplasmide und Wirtszellen sowie anhand von Ausführungsbeispielen erläutert.

Die in den Beispielen verwendeten Oligonukleotid-Linker sind in Tabelle 1 zusammengefaßt.

<u>Beschreibung der Figuren</u>:

Fig.1: Nukleotid- und sich daraus ergebende Aminosäuresequenz des für reife Wildtyp-Staphylokinase SAK42D kodierenden Genes entsprechend SEQ ID Nr. 1.

Fig.2: Nukleotid- und sich daraus ergebende Aminosäuresequenz des für reife Wildtyp-Staphylokinase SAK-Cϕc kodierenden Genes entsprechend SEQ ID Nr. 2.

Fig.3: Nukleotid- und sich daraus ergebende Aminosäuresequenz des für reife Wildtyp-Staphylokinase STAR kodierenden Genes (Genomische DNA aus S. aureus Stamm 23) entsprechend SEQ ID Nr. 3.

Fig.4: Nukleotid- und sich daraus ergebende Aminosäuresequenz des für verkürzte reife Staphylokinase SAKΔN10 kodierenden Fragmentes entsprechend SEQ ID Nr. 4.

Fig.5: Nukleotid- und sich daraus ergebende Aminosäuresequenz des für verkürzte reife Staphylokinase SAKΔN14 kodierenden Fragmentes entsprechend SEQ ID Nr. 5.

Fig.6: Nukleotid- und sich daraus ergebende Aminosäuresequenz der für Staphylokinase SAKM26C kodierenden artifiziellen allelischen Varianten entsprechend SEQ ID Nr. 6.

Fig.7: Nukleotid- und sich daraus ergebende Aminosäuresequenz der für Staphylokinase SAKM26L kodierenden artifiziellen allelischen Varianten entsprechend SEQ ID Nr. 7.

Fig.8: Nukleotidsequenz der RBS-Tandemkonfiguration entsprechend SEQ ID Nr. 8.

Fig.9: Nukleotid- und sich daraus ergebende Aminosäuresequenz des auf dem Genom des Staphylococcus aureus Phagen 42D für native Wildtyp-Staphylokinase SAK42D kodierenden Genes entsprechend SEQ ID Nr. 9.

Fig.10: Gen- und Restriktionskarte des Plasmides pMET5.

Fig.11: Gen- und Restriktionskarte der Plasmidfamilie pUC19sak.

Fig.12: Gen- und Restriktionskarte der Plasmidfamilie pMEX6sak.

Fig.13a: Darstellung der Überexpression der beschriebenen Staphylokinase-Polypeptide anhand eines Geles aus der SDS-PAGE von Zellrohlysaten.

Fig.13b: Darstellung der Bindung des monoklonalen Antikörpers IG8/H6 an alle beschriebenen Staphylokinase-Polypeptide anhand eines Western-Blots der in Abb.13a getrennten Zellrohlysate.

Fig.14: Darstellung der Effizienz der Reinigungsverfahren am Beispiel der Aufarbeitung von SAK42D.

Fig.15: Eichkurve des ELISA-Testes zum quantitativen Nachweis des Staphylokinase-Polypeptides SAK42D.

Fig.16: Auswertung einer Immunpräzipitationsreaktion unter Verwendung des mAK IG8/H6.

Gemäß einer bevorzugten Ausführungsform der Erfindung wird in den nachfolgend angegebenen Teilschritten vorgegangen:

Teilschritt A: Konstruktion eines die reife SAK42D kodierenden Basisplasmids (Plasmid-Bezeichnung: pTZ19Rsak1)

Für die Konstruktion des die reife SAK42D kodierenden Basisplasmides pTZ19Rsak1 wird das rekombinante Plasmid pMET5 (Figur 10) als Gendonor-DNA benutzt. Letzteres ist aus dem Plasmid pDB17 durch eine Folge gentechnischer Arbeitsschritte konstruiert worden.

Die Rekonstitution der kompletten kodierenden Sequenz für reife SAK42D einschließlich der Einführung portabler Translationssignale (Ribosomenbindungssequenz (RBS) und ATG-Startcodon) und einer Übergangs sequenz zum Expressionsanschluß des *sak*42D-Genes an ein geeignetes Transkriptionssignal (Promoter) erfolgt durch Anlagerung eines chemisch synthetisierten Linkerpaares stromaufwärts des unikalen SalI-Ortes. Zu diesem Zweck wird pMET5 zunächst am singulären SalI-Ort geöffnet und erfindungsgemäß das mittels T4-Polynukleotidkinase semiphoshorylierte Linkerpaar L1/L2* (vergleiche Tabelle 1; * zeigt in nachfolgender Beschreibung den phosphorylierten Linkerpartner an), das beim Annealing kompatible Enden zu SalI- und HindIII-Schnittstellen ausbildet, mittels T4-DNA-Ligase an die Enden des linearisierten Plasmides pMET5 ligiert. Durch nachfolgende HindIII-Verdauung wird ein beidseitig von HindIII-Enden flankiertes, etwa 1,2 kb großes SAK42D-kodierendes Fragment erhalten, welches im HindIII-Ort des kommerziellen Vektorplasmides pTZ19R (USB, Bad Homburg) zwischenkloniert wird. Dabei entsteht unter anderem das Plasmid pTZ19Rsak0 mit in-frame-Orientierung des *sak42D*-Genes zum *lac*-Gen des Vektorplasmides.

Zur Abtrennung der 3'-flankierenden, nicht für SAK42D kodierenden DNA-Abschnitte wird zunächst das 1,2 kb große EcoRI/HindIII-Fragment aus pTZ19Rsak0 isoliert und daraus durch konsekutive Restriktaseverdauung mit AvaI, AvaII und HinfI auf günstige Weise ein 388 bp großes EcoRI/HinfI-Fragment gewonnen, in welchem durch den HinfI-Schnitt die letzten 27 bp der für SAK42D kodierenden Sequenz mit entfernt worden sind. Zur Wiederherstellung der vollständigen Sequenz am 3'-Ende des *sak42D*-Genes wird das an HinfI- und PstI-Enden adaptierbare Linkerpaar L3*/L4 (vgl. Tabelle 1) in an sich bekannter Weise an den HinfI-Terminus des EcoRI/HinfI-Fragmentes angelagert. Das so entstehende 433 bp große EcoRI/PstI-Fragment mit dem nunmehr kompletten *sak42D*-Strukturgen wird wiederum in das Vektorplasmid pTZ19R inseriert, welches zuvor mit den gleichen Enzymen behandelt worden war.

Das derart hergestellte Plasmid pTZ19Rsakl fungiert als Basisplasmid für die nachfolgenden gentechnischen Verfahrensschritte zur Herstellung von Expressionsvektoren für SAK42D und N-terminal verkürzte SAK42D-Formen.

Teilschritt B: Konstruktion von Expressionsvektoren für SAK42D und N-terminal verkürzte SAK42-Polypeptide

Als Expressionsvektoren werden die kommerziellen Plasmide pMEX5 und pMEX6 (beide medac, Hamburg) verwendet, in denen die Expresssion durch den synthetischen tac-Promoter vermittelt und die Transkription durch die zwei Tandem-Transkriptionsterminatoren rrnBT1 und rrnBT2 gestoppt wird. Die zu exprimierenden Gene werden zwischen die Regulationssignalsequenzen (nachfolgend als R;tac;SD;-;rrnBT1T2-Anordnung bezeichnet) inseriert. Die beiden genannten Plasmide unterscheiden sich nur dadurch, daß sie den zusätzlich die Bildung einzelsträngiger DNA ermöglichenden Bereich aus dem *E. coli*-f1-Phagen in unterschiedlicher Orientierung 5 enthalten.

Aus dem Basisplasmid pTZ19Rsak1 wird das oben beschriebene EcoRI/PstI-Fragment isoliert und nachfolgend in die mit EcoRI und PstI behandelten Plasmide pMEX5 und pMEX6 inseriert. Nach Transformation in den *E. coli*-Stamm TG1 entstehen die die Expressionsplasmide pMEX503sak und pMEX602sak (Figur 12) tragenden Stämme *E. coli* TG1(pMEX503sak) und *E. coli* TG1(pMEX602sak). In beiden Plasmiden wird die Expression des Ziel-Polypeptids durch eine gleichartige Anordnung der Expressionssignale bewirkt, die dadurch charakterisiert ist, daß in der R;tac; SD;-;rrnBT1T2-Konfiguration zwei gemäß Figur 8 in Tandem-Orientierung angeordnete RBS dem zu exprimierenden *sak42D*-Gen vorgeschaltet sind. Die zweite RBS ist bereits durch das zur Rekonstitution des 5'-Endes des reifen *sak42D*-Genes verwendete Linkerpaar L1/L2 (siehe Tabelle 1) ebenso wie die Erkennungssequenzen für die Restriktasen StuI und NdeI in das Plasmid pTZ19Rsak0 eingeführt worden.

Zur fermentativen Produktion von reifer SAK42D wird bevorzugt der Stamm *E. coli* TG1 (pMEX602sak) eingesetzt.

Zur Erzeugung der verfahrensgemäßen Expressionsplasmide für definiert N-terminal verkürzte Staphylokinasen wird die an sich bekannte Methode der Linkeradaption in der nachfolgend beschriebenen Version benutzt. Die verwendeten Linker sind jeweils so aufgebaut, daß sie am 5'-Ende nach der Ligation an eine mit StuI gespaltene DNA die Erkennungssequenz von StuI rekonstituieren. Stromabwärts des StuI-Ortes folgt eine Übergangssequenz bis zum ATG-Startcodon, dem die Nukleotidsequenz des um die entsprechenden Codons deletierten nativen *sak42D*-Gens bis zu dem im *sak42D*-Gen nächstfolgenden unikalen Restriktionsspaltort angeschlossen ist.

Für die Konstruktion der Expressionsplasmide für die am N-Terminus um 10 Aminosäuren verkürzte SAK42D-Form wird der im *sak42D*Gen unikale BstUI-Spaltort und für die Konstruktion der Expressionsplasmide für die am N-Terminus um 14 Aminosäuren verkürzte SAK42D-Form wird der im *sak42D*-Gen unikale HaeIII-Spaltort benutzt. Verfahrensgemäß werden in beiden Fällen zunächst die semiphophorylierten Linkerpaare L5*/L6 (für ΔN10-SAK42DΔ) und L7*/L8 (für ΔN14-SAK42D) in bewährter Weise an die beiden Enden des mit StuI geöffneten Expressionsplasmides pMEX602sak mit Hilfe von T4-DNA-Ligase angelagert. Durch nachfolgende Verdauung mit HindIII wird das *sak42D*-Gen vollständig aus den jeweiligen linkermodifizierten Vektoren entfernt. Die so erhaltenen, nunmehr von BstUI/HindIII- bzw. HaeIII/HindIII-Enden flankierten linearen Expressionsvektoren werden mit dem das jeweilige verkürzte *sak42D*-Gen tragenden BstUI/HindIII- bzw. HaeIII/HindIII-Fragment (beide Fragmente werden nach Spaltung mit den entsprechenden Restriktase-Paaren aus pTZ19Rsakl isoliert) mit Hilfe von T4-DNA-Ligase verknüpft.

Nach Klonierung in *E. coli* TG1 werden nach der geschilderten Vorgehensweise die die Expressionsplasmide pMEX6N10sak und pMEX6N14sak replizierenden Stämme E. coli TG1(pMEX6N10sak) bzw. E. coli TG1 (pMEX6N14sak) erhalten.

Teilschritt C: Konstruktion der die reifen SAKM26X (mit X = I, R, V, K, L, C, A, H, G und S) tragenden Basisplasmide vom Typ pUC19sakM26X

Die gentechnische Konstruktion der Plasmidfamilie vom Typ pUC19sakM26X (s. a. Figur 11) wird in 3 Unterschritten vollzogen, indem

- in einem ersten Schritt (Teilschritt C1) zunächst die kodierende Sequenz für Aminosäure 1 bis 22 von SAK42D einschließlich Translationsanschlußsequenzen,
- in einem zweiten Schritt (Teilschritt C2) die ab Aminosäure 32 von SAK42D kodierende Sequenz und
- in einem abschließenden dritten Schritt (Teilschritt C3) unter Verwendung einer in Tabelle 1 aufgeführten Palette von chemisch synthetisierten Oligonukleotidlinkern die kompletten Gene für die reifen SAKM26X

aufgebaut werden.

Teilschritt C1:

Der Aufbau der kodierenden Sequenz von Aminosäurenposition 1 bis 22 einschließlich der Einführung einer portablen Translationssignalsequenz (RBS) und von Übergangssequenzen zum Expressionsstartcodon ATG sowie einer Zusatzsequenz zum finalen Expressionsanschluß der kodierenden DNA-Sequenz von SAKM26X an die Translationssignalsequenz (aus pMEX6) erfolgt durch Klonierung von chemisch synthetisierten Oligonukleotidlinkern mit der entsprechenden Nukleotidabfolge in 2 Stufen über das Zwischenplasmid pUC19sak0.

Zunächst wird an die beiden Enden des durch EcoRI-Spaltung linearisierten Vektorplasmides pUC19 das semiphosphorylierte Linkerpaar L9*/L10 auf an sich bekannte Weise mittels T4-DNA-Ligase angelagert. Das verwendete Linkerpaar ist verfahrensmäßig so aufgebaut, daß es neben der portablen Nukleotidsequenz für den Translationsanschluß die kodierende DNA-Sequenz für die Aminosäuren 1 bis 3 und die ersten beiden Nukleotide von Aminosäure 4 sowie die auf pUC19 nicht vorkommende Erkennungssequenz der Restriktase SfuI (überlappt Kodon 4) und ein BamHI-kompatibles 5'-Nukleotidende enthält. Durch nachfolgende Spaltung mit BamHI wird das ebenfalls an der Polyklonierungssequenz (PKS) angelagerte Linkerpaar wieder abgespalten. Der gebildete L9/L10-modifizierte lineare pUCl9-Vektor wird nunmehr mittels T4-DNA-Ligase zirkularisiert und anschließend zwischenkloniert. Dabei entsteht das Plasmid pUC19sak0.

Im nachfolgenden Verfahrensschritt wird in prinzipiell gleicher Verfahrensweise das partiell phosphorylierte Linkerpaar L11*/L12 (vgl. Tabelle 1) an das über den eingeführten SfuI-Ort geöffnete Plasmid pUC19sak0 angelagert. Dabei wird die Sequenz von Kodon 4 des reifen *sakM26X*-Genes komplettiert und die durchgängig kodierende Nukleotidsequenz für die Aminosäuren 1 bis 13 der reifen SAK-Produkte einschließlich der portablen Übergangssequenz aufgebaut. Im Anschluß daran wird durch Spaltung mit der Restriktase SphI das an die PKS angelagerte Linkerpaar L11*/L12 wieder abgespalten. An das entstehende SphI-Ende des linearen Plasmides wird nunmehr das vorher kinasierte Linkerpaar L13/L14* mittels T4-DNA-Ligase angeknüpft. Auf diese Weise wird vom rekonstituierten SphI-Ort in Leserichtung zum überhängenden 5'-Ende des Linkerpaares die kodierende Nukleotidsequenz der Aminosäuren 22 bis 16 sowie ein singulärer Spaltort für die Restriktase NaeI, der das Kodon 22 und den SphI-Ort überlappt, eingeführt. Das entstehende lineare pUC19-Derivat trägt darüber hinaus an seinen Enden eine 6 Nukleotide lange einzelsträngige, zueinander komplementäre DNA-Sequenz, welche nach dem Annealing die Kodone 14 und 15 komplettiert.

Nach Phosphorylierung der freien überhängenden 5' -Nukleotidenden wird das linkermodifizierte Vektorfragment auf die beschriebene Weise zirkularisiert. Durch Klonierung wird das Plasmid pUC19sak1Δ erhalten, welches unter anderem den singulären Spaltort für NaeI mit Anschlußsequenz an Kodon 23 enthält.

Teilschritt C2:

Die Konstruktion des Plasmides pUC19sakA2, das die für den C-Terminus von SAKM26X ab Aminosäure 32 kodierende DNA-Sequenz in einer für die nachfolgenden Verfahrensschritte angepaßter Form enthält, erfolgt in 2 aufeinander abgestimmten gentechnischen Verfahrensschritten unter Verwendung des Zwischenplasmides 5 pUC19sakA1 aus Teilschritt C1.

Zur Bereitstellung der verfahrensmäßig benötigten, für die Aminosäuren 44 bis 124 von SAKM26X kodierenden DNA-Teilsequenz wird wiederum Plasmid pMET5 benutzt. Zur Abtrennung des 3'-flankierenden DNA-Abschnittes wird in diesem Falle zunächst aus pMET5 das 1,2 kb große SalI/PstI-Fragment isoliert und aus diesem - wie bereits beschrieben - durch konsekutive Restriktaseverdauung mit AvaI, AvaII und HinfI auf günstige Weise das 361 bp große SalI/HinfI-Fragment gewonnen, in welchem ebenfalls die letzten 27 bp der für SAK42D kodierenden Sequenz entfernt worden sind.

In einem parallelen Verfahrensschritt wird das HinfI/PstI-adaptierbare Linkerpaar L15/L16* (vgl. Tabelle 1) in vorangehend beschriebener Weise an die PstI-Enden des mit den gleichen Enzymen verdauten Vektors pUC19 angelagert und anschließend nach Spaltung mit SalI der nunmehr lineare SalI/HinfI-kompatible, L15/L16-linkermodifizierte Klonierungsvektor pUC19 gewonnen. Das angelagerte Linkerpaar enthält in vorgefertigter Weise die wiederhergestellte kodierende Sequenz stromab von Kodon 125 des reifen *sak42D*-Genes und darüber hinaus unter Beibehaltung der Aminosäureabfolge zwischen den Aminosäuren 129 und 130 (stille Mutation) der reifen SAK42D (d.h. an Nukleotidposition 391) eine zusätzliche Erkennungssequenz für die Restriktase StyI.

In dem vorangehend beschriebenen modifizierten linearen Vektor pUC19 wird das isolierte 361 bp große SalI/HinfI-Fragment (aus pMET5) mittels T4-DNA-Ligase inseriert und nach Tansformation zunächst das Zwischenplasmid pUC19sakAI gewonnen, welches die vollständige kodierende DNA-Sequenz des reifen *sak42D*-Genes ab Kodon 4 enthält.

Das so hergestellte Zwischenplasmid wird in Fortführung des gentechnischen Verfahrens über den EcoRI-Spaltort linearisiert und nach dem beschriebenen Prinzip durch das semiphosphorylierte Linkerpaar L17*/L18 modifiziert. Diese zueinander komplementären Linker enthalten unter anderem die kodierende Teilsequenz von Kodon 32 bis 43 sowie die ersten beiden Nukleotide von Kodon 44 der reifen Zielsequenz für SAKM26X, wobei in den Kodonen 32 und 33, 38 bis 40 sowie 42 und 43 abweichend zum gleichen Genabschnitt des reifen nativen *sak42D*-Genes zusätzlich 9 stille Mutationen eingeführt wurden. Dadurch entstehen unter Beibehaltung der Aminosäresequenz von SAKM26X in dieser Region verfahrensgemäß zusätzlich erwünschte singuläre Erkennungsorte für die Restriktasen SalI, SacI und SacII. Nach anschließender HindIII-Verdauung des linkersubstituierten Plasmides werden aus dem Spaltgemisch gleichzeitig der lineare L17/L18-HindIII-flankierte Vektor pUC19 und das 414 bp große *sak*-Gensubfragment isoliert. Aus letzterem entsteht durch Spaltung mit der Restriktase MnlI unter anderem das 290 bp große MnlI/HindIII-Fragment, welches 5' -seitig kompatibel zum 3' -Ende des Linkerpaares L17/L18 ist und die ab Aminosäure 46 den C-Terminus von SAKM26X kodierende DNA-Sequenz umfaßt, welche nach dem TAA-Stopkodon trunkiert ist.

Die so erhaltenen DNA-Fragmente werden auf bekannte Weise mittels T4-DNA-Ligase miteinander rekombiniert. Nach Transformation und Selektion wird schließlich das zweite Basisplasmid pUC19sakA2 erhalten, welches gemeinsam mit pUC19sakΔI als Ausgangsplasmid für die nachfolgenden gentechnischen Verfahrensschritte fungiert.

Teilschritt C3:

Die Vereinigung der in den Teilschritten C1 und C2 beschriebenen und zwischenklonierten N- und C-terminalen Genabschnitte zu den durchgängigen reifen *sakH26X*-Genen wird mit Hilfe von komplementären Adapteroligonukleotiden vollzogen, welche für die noch fehlenden DNA-Teilsequenzen kodieren, die den Bereich zwischen den Aminosäuren 23 bis 31 umfassen.

Verfahrensgemäß werden 2 Serien von Adapterpaaren (vgl. Tabelle 1) verwendet, die durch folgende gemeinsame Eigenschaften gekennzeichnet sind:

- Sie besitzen die gleiche Sequenzlänge;
- sie sind 5'-terminal an ein NaeI- und 3'-terminal an ein SalI-Restriktionsspaltende adaptierbar und
- sie enthalten für Kodon-Position 26 solche Nukleotidabfolgen, die jeweils 12 unterschiedlichen Kodonen entsprechen.

Aus diesen Merkmalen resultiert, daß die so aufgebauten, über automatisierte Oligonukleotidsynthese hergestellten Adapteroligonukleotide ein Gemisch von jeweils 12 unterschiedlichen Oligonukleotidsequenzen repräsentieren (AG) und demzufolge in jeder Serie ein Gemisch von jeweils 12 unterschiedlichen Adapterpaaren erzeugt wird. Die Mischung der Nukleotide bei der Synthese der Oligonukleotide ist dabei so konzipiert, daß durch die Adapterserie AG1/AG2 11 und durch die Adapterserie AG3/AG4 6 verschiedene Aminosäuren an Position 26 verschlüsselt sind und potentiell demzufolge der Einbau von insgesamt 17 unterschiedlichen Kodonen für Aminosäure 26 ermöglicht wird.

Im gentechnischen Verfahrensablauf werden in getrennten Reaktionsschritten in an sich bekannter Weise zunächst die oben beschriebenen Adapterpaare AG1/AG2* bzw. AG3/AG4* an die nach SalI-Spaltung von pUC19sakA2 entstehenden freien Enden angekoppelt. Durch nachfolgende HindIII-Verdauung werden die nunmehr AG1/AG2-HindIII- bzw. AG3/AG4-HindIII-terminierten Fragmentgemische isoliert, die den kodierenden Sequenzbereich ab Kodon 24 der reifen Gene umfassen. Die durch den jeweiligen Adaptertyp determinierten Fragmentgemische werden abschließend in dem mit NaeI und HindIII geöffneten Basisplasmidvektor pUC19sak1 kloniert. Die nach Transformation erhaltenen Klone werden durch Doppelspaltung der enthaltenen Plasmid-DNA mit EcoRI und HindIII auf *sakH26X*-Gen tragende Plasmide vorselektiert. Nach DNA-Sequenzanalyse einer repräsentativen Anzahl vorselektierter rekombinanter Plasmide werden schließlich aus den unter Verwendung der Adapterpaare AG1/AG2 erhaltenen Klonen die Plasmide pUC19sakM26I, pUC19sakM26R, pUC19sakM26V, pUC19sakM26T, pUC19M26K und pUC19sakM26L selektiert, die für reife SAKM26I, SAKM26R, SAKM26V, SAKM26T, SAKM26K und SAKM26L kodieren. Auf die gleiche Weise werden aus der AG3/AG4-Serie die für SAKM26C, SAKM26A, SAKM26H, SAKM26G und SAKM26S kodierenden Plasmide pUC19sakM26C, pUC19sakM26A, pUC19sakM26H, pUC19sakM26G und pUC19sakM26S identifiziert und selektiert.

Teilschritt D: Konstruktion der Expressionsplasmide pMEX6sakM26X; Gewinnung von rekombinanten Produzentenstämmen

Als Expressionsvektor wird erneut das kommerziell erwerbbare Plasmid pMEX6 verwendet, in dem die Expresssion bekanntlich durch den synthetischen tac-Promoter vermittelt wird. Aus den vorangehend beschriebenen 11 Plasmiden der Familie pUC19sakM26X, die reife *sakM26X*-Gene tragen, werden die EcoRI/PstI-Fragmente isoliert und diese nachfolgend in den mit EcoRI und PstI behandelten Expressionsvektor pMEX6 inseriert, und mit der erhaltenen rekombinanten DNA werden dann erneut Proben des *E. coli*-Stammes TG1 transformiert. Hierbei entsteht eine Serie von Expressionstämmen *E. coli* TG1(pMEX6sakM26X), welche die jeweiligen Vertreter der Plasmidfamilie pMEX6sakM26X beherbergen. In allen Plasmiden dieser Familie wird die Expression des jeweiligen Ziel-Polypeptides durch eine zu den vorangehend beschriebenen Expressionsplasmiden analoge Anordnung von Expressionssignalen bewirkt.

Nachdem die gebildeten SAKM26X-Polypeptide in einem primären Screening - durchgeführt mittels der nachfolgend beschriebenen Methoden - auf die Ausbildung von Plasminogenaktivator-Aktivität geprüft worden sind, werden für die weiteren Verfahrensschritte zur Fermentation und Anreicherung der Ziel-Polypeptide die Stämme *E. coli* TG1 (pMEX6sakM26L) und *E. coli* TG1(pMEX6sakM26C) als Produzentenstämme für die Gewinnung von SAKM26L und SAKM26C ausgewählt.

Teilschritt E: Bestimmung der Expression und der Aktivität der Plasminogenaktivatoren

Die transformierten *E. coli*-Stämme *E. coli* TG1(pMEX602sak), *E. coli* TG1(pMEX6ΔN10sak), *E. coli* TG1 (pMEX6ΔN14sak), *E. coli* TG1(pMEX6sakM26L) und *E. coli* TG1(pMEX6sakM26C) werden zum Nachweis der intrazellulären SAK-Bildung in einem komplexen Vollmedium oder in einem synthetischen Medium bis zur mittleren oder späten log-Phase submers kultiviert. In dieser Wachstumsphase wird die Expression der SAK-Polypeptide durch Zugabe von Isopropylthiogalactopyranosid (IPTG) induziert. Nach weiteren 1 bis 6 Stunden werden die Zellen durch Zentrifugation geerntet und in einem Phenylmethylsulfonylfluorid (PMSF) enthaltenden Phosphat-Puffer resuspensiert. Mittels Ultraschallaufschluß und hochtouriger Zentrifugation werden aus den Zellen Zellrohextrakte hergestellt.

Aus den klarzentrifugierten Rohextrakten werden in einem zweistufigen säulenchromatographischen Reinigungsverfahren, welches aufeinanderfolgende Chromatographien an S-Sepharose fast flow (Pharmacia, Freiburg) und an Hiload-Phenylsepharose (Pharmacia, Freiburg) umfaßt, die Plasminogenaktivator-Polypeptide u.a. SAK42D, SAKN10, SAKN14, SAKM26L bzw. SAKM26C bis zur elektrophoretischen Homogenität aufgereinigt.

Die Konzentration der Plasminogenaktivatoren in den Rohextrakten sowie in den chromatografischen Fraktionen werden semiquantitativ nach SDS-PAGE durch Kalkulation der Bandenintensitäten nach Coomassie-Brilliant-Blue-Anfärbung oder auf indirektem Wege über die proteolytische Aktivität der aus Plasminogen freigesetzten Protease Plasmin bestimmt. Methodisch erfolgt dieser Nachweis durch Bestimmung der Größe der Lysehöfe auf Plasminogen-Casein-Agar-Platten (D. Behnke und D. Gerlach , Mol. Gen. Genet., 210 (1987), 528-534) und durch kolorimetrische Bestimmung des aus dem synthetischen Plasminsubstrat Chromozym PL freigesetzten Nitrophenolates.

Des weiteren werden Dissoziationskonstanten von Komplexen aus Plasminogen und SAK42D bzw. den erfindungsgemäßen SAK-Polypeptiden durch eine in diesem Zusammenhang neuartige affinitätschromatografische Methode bestimmt.

## Ausführungsbeispiele

Die folgenden Ausführungen sollen an Beispielen die einzelnen Schritte der Verfahren zur Konstruktion der Vektoren, zur Durchführung der Produktsynthesen sowie zum Aufarbeitungsregime veranschaulichen. Sie enthalten jedoch keine detaillierten Angaben zu standardisierten Einzelverfahren der Gentechnologie wie Gewinnung von Plasmid-DNA, Spaltung von DNA mit Restriktionsenzymen, Isolierung von DNA-Fragmenten aus Agarosegelen (ausschließlich nach Glasmilchmethode mit Geneclean$^R$, BiolOl, La Jolla), Einfügen von DNA-Bruchstücken in Vektorplasmide, Transformation von bakteriellen Rezipientenstämmen mit Plasmid-DNA und DNA-Sequenzanalyse. Solche Verfahren sind in einer Reihe von Veröffentlichungen (z. B. in "Molecular Cloning, a Laboratory Manual", T.Maniatis, E.R.Fritsch und J.Sambrook, Cold Spring Harbor Laboratory Press, 2nd Edition, 1989 oder in "Recombinant DNA Methodology", J-A. R.Dillon, A.Nasim und E.R.Nestman, John Wiley & Sons,) ausführlich beschrieben und dem Fachmann als Stand der Technik bekannt.

Sämtliche erfindungsgemäß eingesetzten Oligonukleotide werden durch automatisierte Oligonukleotidsynthese (Modell 380B DNA Synthesizer, Applied Biosystems, Weiterstadt) nach der Phosphoramidit-Methode hergestellt.

## Beispiel 1

### Herstellung von pTZ19Rsak0

Aus dem Stamm *E. coli* TG1(pMET5) wird in an sich bekannter Weise Plasmid-DNA isoliert. Eine ausreichende Menge pMET5 (5 bis 10 µg) wird in 20 µl des entsprechenden Reaktionspuffer mit 20 Einheiten Sall (Boehringer, Mannheim) 2 Stunden bei 37°C inkubiert. Die Reaktion wird durch Phenolextraktion gestoppt und die DNA nach dreimaliger Etherextraktion mit Ethanol (EtOH) gefällt. Der Niederschlag wird in bidestilliertem Wasser ($H_2O$) aufgenommen. Ein Aliquot (200 ng) der linearen Plasmid-DNA wird mit jeweils dem 50-fachen molaren Überschuß des Linkers Ll und des in an sich bekannter Weise phosphorylierten Linkers L2 versetzt und das Gemisch wird mit 10-fachem TM-Puffer (700 mM Tris-HCl (pH 7,5), 60 mM $Mgcl_2$) auf Ligationsbedingungen eingestellt. Die Bildung der doppelsträngigen Linker-DNA wird durch Erwärmen des Reaktionansatzes auf 80°C und nachfolgendes langsames Abkühlen auf Raumtemperatur (RT) erreicht.

Zum Reaktionsgemisch werden jeweils 1/10 Volumenäquivalente 100 mM Dithiothreitol (DTT) (Serva, Heidelberg) und 10 mM Adenosintriphosphat (ATP) (Boehringer) sowie 0,2 Einheiten T4-DNA-Ligase (Boehringer) zugesetzt und 12 Stunden bei 15°C inkubiert. Aus dem Reaktionsgemisch wird die DNA durch Zugabe des 2,5-fachen Volumens EtOH ausgefällt. Der Niederschlag wird in 50 µl B-Puffer (Boehringer) aufgenommen und mit 20 Einheiten HindIII (Boehringer) 2 Stunden bei 37°C inkubiert. Durch Agarose-Gelelektrophorese und nachfolgende Elektroelution wird in an sich bekannter Weise ein 1,2 kb großes Fragment isoliert und der Ligation mit dem zuvor nach Standardvorschriften mit HindIII-Restriktase verdauten Vektor pTZ19R (USB, Bad Homburg) unterworfen. Die Reaktion wird durch 10-minütiges Erwärmen auf 70°C gestoppt. Die Hälfte des Reaktionsgemisches wird zur Transformation kompetenter Zellen des Stammes *E. coli* TG1 eingesetzt.

Die Selektion auf Transformanten erfolgt durch Zusatz von 5-Bromo-4-chloro-3-indolyl-beta-D-galactosid (X-Gal, Boehringer) und Isopropylthiogalactopyranosid (IPTG, Boehringer) zum Agar und Auswahl weißer Zellkolonien. Aus 10 weißen Transformantenklonen wird mittels der Mini-Präparationsmethode nach Standardprotokoll (Plasmid-Minipräp) Plasmid-DNA isoliert und nach Restriktionsanalyse der Sequenzierung nach dem Sanger-Verfahren unter Verwendung von Sequenase™ (USB) unterzogen. Unter den analysierten Klonen befinden sich 8, die die erwartete Sequenz des wiederhergestellten *sak42D*-Genes aufweisen. Eines dieser Plasmide wird ausgewählt und erhält die Bezeichnung pTZ19Rsak0.

**Beispiel 2**

**Konstruktion des Plasmides pTZ19Rsak1**

Zur Abtrennung der auf dem Plasmid pTZ19Rsak0 enthaltenen 3'-flankierenden, nicht für SAK42D kodierenden Bereiche wird nach Standardvorschriften die DNA dieses Plasmides pTZ19Rsak0 isoliert und daraus durch Restriktionsverdauung mit EcoRI und HindIII (Boehringer) und Agarose-Gelelektrophorese ein 1,2 kb großes Fragment gewonnen. Etwa 2 µg dieses Fragmentes werden in an sich bekannter Weise nacheinander mit den Restriktionsenzymen AvaII und HinfI (alle Boehringer) behandelt, wodurch die elektrophoretische Isolierung des Zielfragmentes erleichtert wird. Das bei der Reaktion neben sehr kleinen Spaltfragmenten enstehende 388 bp große HindIII/Hinf-sak42D-Genfragment wird durch Elektrophorese in einem 1,8%-igem Agarosegel so auf günstige Weise isoliert. Ein Aliquot (200 ng) der wäßrigen DNA-Lösung wird mit dem jeweils 50-fachen molaren Überschuß des in an sich bekannter Weise phosphorylierten Linkers L3* und des Linkers L4 versetzt und mit 1/10 Volumenäquivalenten 10-fach konzentrierten TM-Puffers auf die Reaktion eingestellt. Die Bildung der doppelsträngigen Linker-DNA wird durch Erwärmen des Reaktionansatzes auf 65°C und nachfolgendes langsames Abkühlen auf Raumtemperatur (RT) erreicht.

Zum Reaktionsgemisch werden jeweils 1/10 Volumenäquivalente 100 mM DTT und 10 mM ATP sowie 0,2 Einheiten T4-DNA-Ligase zugesetzt und 16 Stunden bei 15°C inkubiert. Aus dem Reaktionsgemisch wird die DNA durch Zugabe des 2,5-fachen Volumens EtOH ausgefällt. Der so erhaltene Niederschlag wird in 15 µl Ligationspuffer (Boehringer) aufgenommen und mit ca. 50 ng des in Wasser gelösten Plasmides pTZ19R versetzt, das zuvor auf an sich bekannte Weise mit den Restriktionsenzymen EcoRI und PstI (Boehringer) behandelt worden ist. Zu diesem Reaktionsgemisch werden 0,2 Einheiten T4 DNA-Ligase zugefügt, und es wird 16 Stunden bei 15°C inkubiert. Die Hälfte dieses Ansatzes wird zur Transformation kompetenter Zellen des Stammes *E. coli* TG1 nach Standardvorschriften eingesetzt. Die Selektion auf Transformanten erfolgt - wie vorausgehend beschrieben - durch Zusatz von X-Gal und IPTG zum Agar und durch Auswahl weißer Zellkolonien.

Aus 10 weißen Transformantenklonen werden in bekannter Weise Plasmid-Minipräps hergestellt und vier dieser Plasmide werden der Plasmidsequenzierung unterzogen. Drei der so analysierten Klone weisen die erwartete, von EcoRI- und PstI-Orten flankierte kodierende Sequenz für reife SAK42D auf. Eines dieser Plasmide wird ausgewählt und erhält die Bezeichnung pTZ19Rsak1.

**Beispiel 3**

**Herstellung von pMEX503sak und pMEX602sak**

Aus dem Basisplasmid pTZ19Rsak1 wird auf bekannte Weise das 433 bp große, von EcoRI und PstI flankierte Fragment nach Elektrophorese des Reaktionsgemisches an 1,6%-iger Agarose isoliert. Parallel dazu werden die Vektoren pMEX5 und pMEX6 (medac) nach Standardvorschriften mit den Restriktionsenzymen EcoRI und PstI behandelt und die Vektor-Fragmente isoliert. Jeweils 50 ng der so vorbehandelten Vektorplasmide werden mit jeweils 50 ng des obigen 433 bp-Fragmentes in Ligationspuffer (Boehringer) vereinigt und nach Zugabe von 0,2 Einheiten T4-DNA-Ligase 16 Stunden bei 15°C inkubiert. Die nach der Transformation in Zellen von *E. coli* TG1 erhaltenen ampicillinresistenten Kolonien werden durch Übertragen auf einen plasminogenhaltigen Casein-Agar (siehe D. Behnke und D. Gerlach (1987), a. a. O.), welcher zusätzlich noch mit 200 µM IPTG ausgestattet ist, auf die Expression von Staphylokinase-Aktivität getestet.

Die Plasmid-DNA von je 4 auf den Casein-Testplatten Lysehöfe ergebenden Klonen wird in bekannter Weise gewonnen und die Nukleotidsequenz durch Plasmidsequenzierung nach dem Standardprotokoll ermittelt. Die so analysierten Plasmide zeigten ausnahmslos die richtige Sequenz des reifen *sak42D*-Geninsertes und der 5'-flankierenden translationsdeterminierenden Bereiche.

Eines dieser Plasmide aus der pMEX5-Serie erhält die Bezeichnung pMEX503sak, und ein weiteres von pMEX6 abgeleitetes Plasmid wird mit pMEX602sak bezeichnet. Der mit dem Expressionsplasmid pMEX602sak transformierte Stamm *E. coli* TG1(pMEX602sak) wird für die nachfolgenden Verfahrensschritte zur Fermentation und Anreicherung von SAK42D als Produzentenstamm ausgewählt.

**Beispiel 4**

**Konstruktion der Plasmide pMEX6ΔH10sak und pMEX6ΔH14sak**

Zur Herstellung der Expressionsplasmide für die verkürzten *sak42D*-Gene werden 10 µg des Plasmides pMEX602sak in 50 µl H-Puffer (Boehringer, Mannheim) mit je 80 Einheiten StuI (Boehringer) 2 Stunden bei 37°C inkubiert. Die lineare Vektor-DNA wird auf bekannte Weise nach Agarose-Gelelektrophorese (AGE) an einem 1%-igen Gel isoliert. In zwei getrennten Reaktionsansätzen werden jeweils 2 µg der linearen Plasmid-DNA mit jeweils dem 50-fachen molaren Überschuß der Linkerpaare L5*/L6 bzw. L7*/L8 versetzt, von denen jeweils der gekennzeichnete Linker vorangehend nach Standardvorschriften phosphoryliert worden ist. Die Bildung der doppelsträngigen (ds) Linker-DNA wird durch Erwärmen der entsprechenden Linkerpartner in TM-Puffer auf 80°C und nachfolgendes langsames Abkühlen auf RT (Annealing) erreicht. Zu den Reaktionsgemischen werden jeweils 1/10 Volumenäquivalente 100 mM DTT und 10 mM ATP sowie 0,2 Einheiten T4 DNA Ligase zugesetzt und 12 Stunden bei 15°C inkubiert. Aus den Reaktionsgemischen wird die jeweilige L5/L6- bzw. L7/L8-flankierte lineare Plasmid-DNA durch Zugabe des 2,5-fachen Volumens EtOH ausgefällt. Die Niederschläge werden in 20 µl B-Puffer (Boehringer) aufgenommen und mit je 20 Einheiten HindIII (Boehringer) 2 Stunden bei 37°C inkubiert, wodurch das gesamte für native reife SAK42D kodierende Gen jeweils vom restlichen Teil der Vektorplasmide abgespalten wird. Die *sak*-Gen-freien, linkermodifizierten pMEX6-Fragmente werden durch Elektrophorese in einem 1%-igen Agarosegel (AG) isoliert.

Parallel dazu wird zunächst aus 50 µg des Plasmides pTZ19RsakI durch Doppelverdauung mit je 50 Einheiten der Restriktasen EcoRI und HindIII (Boehringer) sowie anschließender AGE auf an sich bekannte Weise das 450 bp große sak42D-Gen-Fragment isoliert. In wiederum parallelen Reaktionsansätzen werden jeweils 50 µl dieses *sak*42-Fragmentes (etwa 800 ng) durch Zugabe des jeweiligen 10-fach konzentrierten Reaktionspuffers auf Reaktionsbedingungen eingestellt. In dem einen Ansatz wird das Fragment mit 10 Einheiten BstUI (NEB) bei 60°C und im zweiten Ansatz mit 10 Einheiten HaeIII (Boehringer) bei 37°C 2 Stunden verdaut. Durch nachfolgende AGE in einem 1,8%-igen AG werden in einem Falle das 370 bp große BstUI/HindIII-Fragment und im anderen Falle das 358 bp große HaeIII/HindIII-Fragment abgetrennt und isoliert.

Jeweils etwa 10 µl (80 ng) der in Wasser gelösten, das *sak42D*-Gen enthaltenden Fragmente werden mit je 10 µl (50 ng) der vorangehend hergestellten, in Wasser gelösten linkermodifizierten Vektor-DNA vereinigt, mit 10-fachem Ligationspuffer auf Ligationsbedingungen eingestellt und mit 3 Einheiten T4 DNA Ligase für 15 Stunden bei 15°C inkubiert.

Jeweils die Hälfte der Volumina dieser Ligationsansätze wird zur Transformation kompetenter Zellen des Stammes *E. coli* TG1 nach Standardvorschriften eingesetzt. Etwa je 20 auf Ampicillin-Nähragarplatten (Amp-NA) gewachsene Transformanten werden nach Übertragen auf plasminogenhaltigen Casein-Nähragar auf die Expression von Staphylokinase-Aktivität geprüft, welche nach 2-bis 5-stündiger Inkubation bei 37°C in Form von Lysehöfen um die Einstichlöcher der Zellen zu erkennen ist. Aus je 10 der in diesem Test positive Ergebnisse liefernden Klone wird auf bekannte Weise Plasmid-DNA isoliert.

Mittels Restriktionsanalyse unter Verwendung von HindIII und StuI (Boehringer) werden diejenigen Plasmide vorselektiert, die die erwarteten StuI/HindIII-Inserte tragen. Durch nachfolgende Plasmidsequenzierung von je 4 Plasmid-Minipräps der entsprechenden Serie wird die Nukleotidsequenz der jeweiligen Plasmid-Inserte festgestellt. Die so untersuchten 4 Klone beider Serien enthalten ausnahmslos die erwarteten, von EcoRI- und HindIII-Orten flankierten *sak*42D-Sequenz-Inserte. Das das N-terminal um 10 Aminosäuren verkürzte *sak42D*-Gen tragende Expressionsplamid trägt die Bezeichnung pMEX6ΔN10sak, während das das um 14 Aminosäuren verkürzte *sak42D*-Gen tragenden Expressionsplamid die Bezeichnung pMEXN14sak erhält. Die jeweiligen mit diesen Plasmiden transformierten Stämme *E. coli* TG1(pMEXN10sak) bzw. *E. coli* TG1(pMEx6ΔN14sak) werden verfahrensgemäß als Produzentenstämme für SAKN10 bzw. SAKN14 benutzt.

**Beispiel 5**

**Herstellung des pUC19sakA1**

Aus dem Stamm *E. coli* JM109(pUC19) wird zunächst in an sich bekannter Weise über Ethidiumbromid-Cscl-Dichtegradientenzentrifugation der Vektor pUC19 isoliert und für alle nachfolgenden gentechnischen Verfahrensschritte bereitgestellt.

Etwa 2 µg pUC19 werden in 30 µl H-Puffer mit 10 Einheiten EcoRI bis zur vollständigen Linearisierung des Vektors (ca. 1 Stunde) inkubiert. Nach Zugabe von $H_2O$ ad 90 µl und 10 µl 1 M Tris-HCl (pH 8,5) wird mit 100 µl wassergesättigtem und mit 0,5 % 8-Oxychinolin stabilisiertem Phenol (nachfolgend nur Phenol genannt) extrahiert und das Gemisch anschließend 2 Minuten bei 15000 rpm zentrifugiert. Nach Einstellen der separierten wäßrigen Phase auf eine Ammoniumacetat ($NH_4$-Azetat)-Konzentration von 2,5 M wird der EcoRI-gespaltene Vektor mit EtOH ausgefällt, der Niederschlag 2-mal mit 70 %-igem EtOH gewaschen und nachfolgend in 20 µl $H_2O$ gelöst.

Parallel dazu wird die Menge des Linkers L9, die einem 50- bis 80-fachen molaren Überschuß an freien EcoRI-Enden des linearen Vektors pUC19 entspricht (je µg linearem Vektor ca.0,1 $A_{260}$-Einheiten Linker der Nukleotidlänge 30 bis 40) in 15 µl Reaktionsvolumen in Gegenwart von 2 mM ATP unter Kinasebedingungen bei 37°C PNK phosphoryliert. Nach 20 Minuten wird die Reaktion durch Erhitzen auf 70°C für 15 Minuten vollständig gestoppt und nach Abkühlen des Ansatzes im Eisbad die gleiche molare Menge des Linkers L10 zugegeben. Die Bildung des ds Linkerpaares L9*/L10 wird durch Annealing erreicht. Zu dem so hergestelltem Linkerpaar L9*/L10 werden 18 µl EcoRI-gespaltene pUC19-DNA hinzugegeben sowie mit 10-fach konzentriertem T4-DNA-Ligase-Puffer und $H_2O$ auf DNA-Ligase-Bedingungen in einem Reaktionsvolumen von 100 µl eingestellt. Nach Zugabe von 5 Einheiten T4-DNA-Ligase wird bei 15°C über Nacht (üN) inkubiert. Der linkermodifizierte Vektor wird in Gegenwart von $NH_4$-Az mit EtOH ausgefällt, das Präzipitat mit 70%-igem EtOH 2-mal gewaschen und nach Trocknung in 50 µl H-Puffer aufgenommen. Nach Zugabe von 15 Einheiten BamHI (Boehringer) wird 2 Stunden bei 37°C inkubiert. Das Reaktionsgemisch wird in einem 1%-igem Agarosegel aufgetrennt und das Vektorfragment mittels 5 µl Glasmilch aus der Kaliumjodid-Gellösung isoliert.

Etwa 50 ng des L9/L10-BamHI-flankierten pUC19-Vektors werden mit 0,5 Einheiten T4-DNA-Ligase in einem Endvolumen von 20 µl unter Ligasebedingungen 2 Stunden bei 15°C inkubiert und anschließend nach Standardvorschriften in kompetente Zellen von *E. coli* TG1 transformiert. Diese werden nach der Transformation auf Nähragarplatten (Serva) ausgestrichen, die mit 80mg Ampicillin/ml Agar, X-Gal und IPTG supplementiert sind (X-Gal-Amp-NA). Aus 6 weißen Klonen wird mittels der Mini-Präparationsmethode Plasmid-DNA isoliert, dieses durch Spaltung mit der entsprechenden Restriktase auf Anwesenheit eines singulären SfuI-Ortes (Boehringer) getestet und durch Plasmidsequenzierung der korrekte Einbau der Linker bestätigt. Einer der positiven Klone wird zur Isolierung des nunmehr als pUC19sak0 bezeichneten Zwischenplasmides ausgewählt.

Auf die vorangehend exemplarisch beschriebene Weise werden ca. 10 µg pUC19sak0 in 100 µl H-Puffer mit 30 Einheiten SfuI linearisiert, das Reaktionsgemisch mit Phenol extrahiert, das geöffnete Vektorplasmid mit EtOH ausgefällt und der mit 70%igem EtOH gewaschene Niederschlag in 30 µl $H_2O$ gelöst. Gleichzeitig werden 0,4 $A_{260}$-Einheiten des Oligonukleotides L11 zur Anlagerung an 4 µg mit PNK kinasiert und nach Inaktivierung der PNK mit der gleichen molaren Menge des Linkers L12 durch die Annealing-Reaktion zum ds-Linkerpaar komplementiert. Zu diesem werden 12 µl des SfuI-gespaltenen Vektorplasmides hinzugefügt, auf 100 µl Ligationspuffer eingestellt und mit 5 Einheiten T4-DNA-Ligase 4 Stunden bei 16°C inkubiert. Nach EtOH-Präzipitation und -Waschung wird das in 80 µl M-Puffer (Boehringer) gelöste Plasmid mit 20 Einheiten SphI (Boehringer) bei 37°C in der Polyklonierungssequenz (PKS) des pUC19-Replikons gespalten. Das L11/L12-modifizierte und SphI-Enden tragende Plasmidfragment wird durch Elektrophorese in einem 1-%igen Agarosegel mit anschließender Fragmentisolierung gereinigt.

An etwa 600 ng des gereinigten Fragmentes wird nach der vorangehend beschriebenen Verfahrensweise das zweite Linkerpaar, nämlich L13/L14*, in 70 µl Reaktionsvolumen mittels 4 Einheiten T4-DNA-Ligase über Nacht angelagert, wobei in einem vorangehenden Schritt etwa 0,05 $A_{260}$-Einheiten L14 in 15 µl Kinasepuffer vor dem Annealing-Schritt mit der gleichen molaren Menge L13 mittels PNK am 5'-Ende phosphoryliert wurden. Durch 15-minütiges Erhitzen des Ligationsgemisches auf 65°C wird die T4-DNA-Ligase inaktiviert. Zur Kinasierung der nach der zweiseitigen Linkeranlagerung vorliegenden freien 5'-OH-Enden (am L12- und L13-Strangende) wird das temperierte Ligationsgemisch auf 3 mM ATP eingestellt und mit 2 Einheiten PNK 25 Minuten bei 37°C inkubiert. Das nunmehr 5'-L12*/L13*-flankierte Plasmidfragment wird durch AGE wie beschrieben von dem Linkerüberschuß abgetrennt und isoliert.

Etwa 30 ng der so gewonnenen Vektor-DNA werden im abschließenden gentechnischen Konstruktionsschritt über die L12- und L13- komplementären Hexanukleotid-Enden mit 1 Einheit T4-DNA-Ligase über Nacht *in vitro* zirkularisiert. Der Ligationsansatz wird in kompetente Zellen von *E. coli* TG1 transformiert und 1/3 des Ligtionsansatzes auf Amp-NA-Platten ausgestrichen. Von 12 Klonen werden Plasmid-Minipräps hergestellt und diese auf Anwesenheit des singulären NaeI-Restriktasespaltortes (Boehringer) getestet. Einer der NaeI-positiven Klone wird nach DNA-Sequenzanalyse als Donor für das Basisplasmid mit dem erwarteten Linker-Insert ausgewählt.

Dieses Plasmid erhält die Bezeichnung pUC19sakA1 (erstes Basisplasmid) und wird für die folgenden Konstruktionsschritte in präparativem Maßstab bereitgestellt.

**Beispiel 6**

**Konstruktion der Zwischenplasmides pUC19sakA2**

Zunächst wird nach dem gleichen Verfahren, welches bei der Herstellung von pUC19sak0 ausführlich beschrieben wurde, ein L14*/L15-flankiertes pUC19-Vektorfragment hergestellt, indem das Linkerpaar L14*/L15 an die PstI-Enden (PstI, Boehringer) des linearisierten Vektors pUC19 angelagert und nachfolgend in der PKS mit SalI gespalten wird.

Zur Gewinnung der für den Bereich der Aminosäuren 5 bis 124 der nativen reifen SAK42D kodierenden Gensequenz wird aus dem Plasmid pMET5 eine ausreichende Menge des die gewünschte Gensequenz tragenden, 1,2 kb großen SalI/PstI-Fragmentes isoliert. Dazu werden ca. 50 µg pMET5 in 250 µl H-Puffer erst mit 200 Einheiten SalI verdaut und nach vollständiger Spaltung mit der gleichen Aktivität PstI nachverdaut. Das SalI/PstI-Fragment wird aus einem 1,2%-igen Agarosegel isoliert. Zur Abtrennung des 3'-flankierenden, nicht für SAK42D kodierenden DNA-Bereiches werden etwa 2 µg dieses Fragmentes in 150 µl unter M-Puffer-Bedingungen nach dem in Beispiel 2 beschriebenen Teilverfahren gleichzeitig mit jeweils 15 Einheiten AvaI, AvaII inkubiert und HinfI (alle Enzyme Boehringer) verdaut. Das neben sehr kleinen Spaltfragmenten entstehende 361 bp große SalI/HinfI-*sak42D*-Genfragment wird nach Elektrophorese in einem 1,8%-igen Agarosegel so auf günstige Weise isoliert.

Etwa 50 ng des gewonnenen SalI/HinfI-Fragmentes werden in 30 ng des in dem vorangehenden Abschnitt beschriebenen modifizierten pUC19-Vektors inseriert und in *E. coli* TG1 kloniert. Screening auf Anwesenheit von StyI-Restriktionsspaltorten (StyI, Boehringer) in ausgewählten Klonen und DNA-Sequenzanalyse bestätigen das Vorliegen der erwarteten Insertsequenz in einer repräsentativen Anzahl von Plasmid-Minipräps. Einer der so charakterisierten Klone wird ausgewählt und als Quelle für das pUC19sakA1 benannte Plasmid verwendet.

Nach den gleichen gentechnischen Verfahrensschritten wird an die Enden des EcoRI-geöffneten Plasmides pUC19sakA1 das Linkerpaar L16*/L17 angelagert. Nach Phenolextraktion und EtOH-Fällung werden etwa 3 µg des linkersubstituierten Plasmidfragmentes in 80 µl B-Puffer mit 15 Einheiten HindIII verdaut und das Spaltgemisch in einem 1,6%-igen AG elektrophoretisch aufgetrennt. Aus dem Gel wird sowohl das L16/L17-HindIII-flankierte, im nachfolgenden Teilschritt als Vektorfragment fungierende DNA-Fragment als auch das *sak*-Gen-tragende 414 bp große Bruchstück isoliert.

Etwa 250 ng des letztgenannten Subfragmentes werden in 50 µl NE2-Puffer mit 10 Einheiten MnlI (NEB) nachverdaut, so daß aus dem Reaktionsgemisch das 290 bp große MnlI/HindIII-Fragment nach Gelelektrophorese aus einem 1,8%-igen AG isoliert werden kann. Etwa 25 ng des so gewonnenen, für den Bereich ab Aminosäure 46 von SAK42D kodierenden DNA-Fragmentes werden abschließend in 30 ng des oben isolierten Vektorfragmentes gemäß Standardprozeduren in *E. coli* TG1 kloniert.

Eine repräsentative Anzahl von Plasmid-Minipräps werden durch Doppelverdauuung sowohl mit SalI und HindIII als auch mit SacII (Boehringer) und HindIII unter jeweils angepaßten Bedingungen auf das Vorhandensein der gesuchten DNA-Abschnitte getestet. Nach DNA-Sequenzanalyse wird einer der positiven Klone zur Isolierung des nunmehr als pUC19sakA2 bezeichneten zweiten Basisplasmides ausgewählt.

**Beispiel 7**

**Herstellung der SAKM26X kodierenden Donorplasmide pUC19sakM26X**

Mit Hilfe der automatisierten Oligonukleotidsynthese werden durch simultanen Einbau von 3 bzw. 4 verschiedenen Nukleotidbausteinen in die beiden ersten Positionen des Kodons 26 der reifen SAK42D die Adaptergemische AG1, AG2, AG3 und AG4 bereitgestellt, die jeweils eine Mischung von 12 Sequenzvarianten darstellen. Gemäß vorangehend beschriebener Verfahrensweise wird in einem ersten Ansatz das Adaptergemisch AG2 sowie in einem anderen Ansatz das Adaptergemisch AG4 kinasiert. Die phosphorylierten Linkergemische werden durch Annealing mit den jeweils komplementären Adaptergemischen AG1 bzw. AG3 zu doppelsträngigen DNA-Fragmenten vereinigt. Nach der Ankopplung der so erhaltenen Adapterpaargemische an die SalI-Enden des in separaten Reaktionsschritten mit diesem Enzym geöffneten zweiten Basisplasmides pUC19sakA2 mit nachfolgender HindIII-Verdauung werden die jeweiligen Reaktionsgemische erhalten, aus welchen durch gelelektrophoretische Auftrennung in einem Fall das AG1/AG2- und im anderen Fall das AG3/AG4-determinierte Fragmentgemisch isoliert wird. Diese Gemische werden jeweils durch ein HindIII- und ein stumpfes (blunt), damit NaeI-kompatibles Ende begrenzt.

Jeweils 150 ng jedes Fragmentgemisches werden mit 100 ng NaeI/HindIII-linearisiertem erstem Basisplamid pUC19sakA1 rekombiniert und in *E. coli* TG1 kloniert. Eine Vielzahl von Klonen beider Serien werden durch Restriktasedoppelspaltungen der Plasmid-Minipräps mit EcoRI und HindIII auf das Vorliegen von 436 bp großen DNA-Inserten vorselektiert. In einem umfangreichen Programm der DNA-Sequenzanalyse unter Verwendung des Primers S26 werden schließlich aus der AG1/AG2-Serie die Plasmide gefunden, die an Position 26 des reifen *sakM26X*-Genes folgende Kodone tragen:

ATA(Ile), AGA(Arg), GTA(Val), ACA(Thr), AAA(Lys) und CTA(Leu)

Ausgehend von der AG3/AG4-Serie wurden in gleicher Weise Plasmide selektiert, die an Position 26 des *sakM26X*-Genes folgende Kodone aufweisen:

TGC(Cys), GCC(Ala), CAC(His), GGC(Gly) und TCC(Ser).

**Beispiel 8**

**Herstellung der Expressionsplasmide der pMEx6sakM26X-Familie**

Etwa 20 µg des Vektors pMEX6 werden mit 100 Einheiten EcoRI bis zur vollständigen Linearisierung gespalten und mit 100 Einheiten PstI unter gleichen Bedingungen nachverdaut. Der so gespaltene Vektor wird durch Elektrophorese in einem 1%-igen AG von dem PKS-Fragment abgetrennt. Aus den 11 Donorplasmiden, die die unterschiedlichen *sak*-Gen-Varianten kodieren, werden jeweils die 433 bp großen ECORI/pstI-Fragmente, welche am 5'-Ende zusätzlich die Translationssignalsequenzen (RBS und ATG-Startkodon) tragen, wie beschrieben gelelektrophoretisch aus einem 1,6%-igen AG isoliert.

Etwa 50 ng jedes dieser expressionsangepaßten *sak*-Genfragmente werden in jeweils separaten Ansätzen mit ca. 20 ng des EcoRI/PstI-linearisierten pMEX6-Vektors ligiert und in *E. coli* TG1 kloniert. Aus je 10 ml Kulturlösung der das jeweilige Expressionsplasmid enthaltenden *E. coli* TG1-Stämme werden nach der Minipräp-Methode die entsprechenden Plasmide isoliert und durch Restriktionsanalyse mit EcoRI und PstI sowie durch Sequenzanalyse beider Stränge charakterisiert. Die jeweils die erwartete *sak*-Gensequenz tragenden Plasmide werden dem Plasmidtyp pMEX6sakM26X zugeordnet.

**Beispiel 9**

**Qualitativer Nachweis der Überexpression von SAK42D und der erfindungsgmemäßen SAK-Polypeptide**

Zum analytischen Nachweis der induzierbaren Überexpression dererfindungsgemäßen SAK-Polypeptide in den entsprechenden, mit den Plasmiden pMEX602sak, pMEX6ΔN10sak, pMEX6ΔN14sak und pMEX6sakM26X transformierten *E. coli* TG1-Stämmen werden jeweils 5 ml eines 2-fach konzentrierten Trypton-Hefextrakt-Mediums, welches zusätzlich mit 50 µg/ml Ampicillin ausgestattet ist (2xTY-Amp-Medium), mit 100 µl einer üN-Kultur beimpft, die aus einer Einzelkolonie der jeweiligen rekombinanten *E. coli* TG1-Produzentenstämme bereitet worden ist. Es wird 3 Stunden bei 37°C unter Schütteln kultiviert und danach die Expression durch Zugabe einer sterilen IPTG-Lösung (40 mg/ml Isopropanol) bis zu einer Endkonzentration von 0,2 mM induziert. Die Fermentation wird 4 Stunden fortgeführt und die Zellen von 1 ml Kulturmedium dann durch Zentrifugation gesammelt. Das Zellpellet wird in 400 µl 40 mM Phosphatpuffer (pH 7,0) suspendiert, mit 100 µl 5-fach konzentrierter Zellaufschlußlösung (20%-ige SDS-Lösung/-Mercaptoäthanol/Glycerol/0,02%-ige Bromphenolblau-Lösung; 6:1:1:0,1; V/V ) versetzt und während 5 Minuten im siedenden Wasserbad aufgeschlossen. Aliquote (2 bis 6 µl) der so hergestellten Lysate werden nach Standardprotokoll in einem 1 mm dicken 15%-igen SDS-Polyacrylamid-Gel nach Coomassie-Brilliant-Blue-G250-Färbung in Hinsicht auf das Auftreten einer Bande, die überexprimiertes Protein enthält, analysiert (Figur 13a). Alle analysierten Rohlysate zeigen die Überexpression des jeweiligen SAK-Polypeptides in Gestalt einer dominierenden Proteinbande.

**Beispiel 10**

**Fermentation der *E. coli* TG1-Produzentenstämme und Reinigung der erfindungsgemäßen SAK-Polypeptide**

Alle Stämme werden grundsätzlich gemäß nachfolgender Beschreibung fermentiert und aufgearbeitet.

Die Fermentation der Stämme erfolgt bei 37°C in mit 200 ml Medium gefüllten 500ml-Rundkolben unter intensivem Schütteln ohne zusätzliche Belüftung. Dazu werden 5 ml 2xTY-Amp-Medium mit einer Einzelkolonie des entsprechenden Stammes beimpft und ca. 16 Stunden bei 37°C unter Schütteln vorkultiviert. Jeweils 2 ml dieser Vorkultur werden zum Beimpfen von 200 ml des gleichen Mediums verwendet. Diese Kulturen werden bei 37°C geschüttelt, bis eine optische Dichte von 0,4 bis 0,9 $A_{600}$-Einheiten erreicht ist. Nach Erreichen dieser Zelldichte wird die Expression der auf den Expressionsplasmiden kodierten Staphylokinase-Gene durch Zugabe von 400 µl einer sterilen wäßrigen IPTG-Lösung (40mg/ml) induziert. Im Anschluß daran werden die Kulturen weitere 2 bis 6 Stunden bei 37°C geschüttelt und dann durch Zentrifugation bei 4°C und 4000 rpm geerntet. Die Zellniederschläge werden in 1/20 bis 1/40 Volumenäquivalenten (bezüglich des Kulturvolumens) Aufschlußpuffer (40 mM Phosphatpuffer (pH 6,5), 30 mM NaCl, 10 mM EDTA, 10 mM EGTA und 10 mM β-Mercaptoethanol) suspendiert und mit Phenylmethylsulfonylfluorid bis zu einer Endkonzentration von 1 mM versetzt. Die Suspensionen werden auf 0°C abgekühlt und bei dieser Temperatur 15 Minuten mit Ultraschall (Leistung 100 bis 120 Watt) aufgeschlossen.

Die Aufschlüsse werden bis zur Aufarbeitung bei -20°C gelagert. Am Beginn der Aufreinigung werden jeweils maximal 30 ml Aufschlußsuspension im Wasserbad (30°C) aufgetaut und danach 60 Minuten bei 4°C und 25000 rpm zentrifugiert. Die Überstände werden mit $H_2O$ auf das 4-fache Volumen verdünnt und anschließend auf eine mit 10 mM Phosphatpuffer (pH 6,5) äquilibrierte Chromatographiesäule gepumpt, die S-Sepharose fast flow (Pharmacia, Freiburg) enthält. Das Gelbett hat einen Durchmesser von 2,6 cm und eine Höhe von 30 cm. Die Flußgeschwindigkeit beim Auftragen sollte 2 ml/min. nicht übersteigen. Anschließend wird mit dem Äquilibrierungspuffer gewaschen (Flußgeschwindigkeit 3 bis 4 ml/min.) bis die Basislinie der UV-Detektion (280 nm) erreicht wird. Das ungebunden die Säule passierende Material enthält nur Spuren der Ziel-Polypeptide und wird verworfen. Die Elution der Säule (Flußrate 3 bis 4 ml/min.) erfolgt mit 10 mM Phosphatpuffer (pH 6,5), der 250 mM NaCl enthält. Die Elutionsprofile zeigen mehrere Peaks, von denen einer das entsprechende SAK-Polypeptid in angereicherter Form enthält (Nachweis in SDS-PAGE). Die entsprechenden Peak-Fraktionen werden vereinigt und anschließend durch Zugabe von festem NaCl auf eine Endkonzentration von 2,0 M NaCl eingestellt. Auf diese Weise werden die gesammelten Fraktionen direkt für die Chromatographie an Phenyl-Sepharose vorbereitet. Für diesen Schritt wird eine fertiggepackte HiLoad-Phenyl-Sepharose HPXK 26/10-Säule (Pharmacia) verwendet, die mit 10 mM Phosphatpuffer (pH 6,5), der 2,0 M NaCl enthält, äquilibriert worden ist. Nach dem Probenauftrag (Flußrate 5,0 ml/min.) wird mit Äquilibrierungspuffer bis zum Erreichen der Basislinie der UV-Detektion gewaschen (Flußrate 12,6 ml/min.). Danach werden die SAK-Formen mit einem absteigenden NaCl-Gradienten (Flußrate 12,6 ml/min.) eluiert :

100% Puffer A -----> 25% Puffer A

0% Puffer B -----> 75% Puffer B

Puffer A : 10 mM Phosphatpuffer, pH 6,5; 2,0 M NaCl
Puffer B : 10 mM Phosphatpuffer, pH 6,5

Das Gesamtgradientenvolumen beträgt im vorliegenden Maßstab 300 ml. Das Verfahren gestattet die Gewinnung reiner SAK-Polypeptide (Reinheitskriterien SDS-PAGE und isoelektrische Focussierung) in nur zwei Reinigungsschritten. Die Effektivität des Reinigungsverfahrens ist aus Figur 14 ersichtlich, in der die exemplarische Konzentrierung des Ziel-Peptides SAKM26L aus den Zellrohextrakten bis hin zur elektrophoretischen Homogenität anhand eines SDS-Geles dargestellt ist.

**Beispiel 11**

**Bestimmung der spezifischen Aktivität der erfindungsgemäßen SAK-Polypeptide**

Die spezifische Aktivität einer SAK-Form wird definiert als diejenige Menge Plasmin (μmol), die von 1 μmol dieses SAK-Polypeptides aus Plasminogen (eingesetzt im Überschuß) pro Minute freigesetzt wird.

Der Proteingehalt der jeweiligen SAK-Polypeptid-haltigen Stammlösungen wird mittels des Bio-Rad Protein Assays (Bio-Rad, München) unter Verwendung von Rinderserumalbumin als Standardprotein ermittelt.

Die Bestimmung der Plasmin-Freisetzung durch die SAK-Polypeptide erfolgt in zwei Schritten :

- Zunächst werden Verdünnungsreihen (1 ng bis 500 ng) der SAK-Polypeptide in 100 mM Tris (pH 8,0) hergestellt. Danach wird ein Reaktionsgemisch, bestehend aus 30 μl Plasminogenlösung 20 mg Plasminogen (Fluka), gelöst in 1 ml $H_2O$, 150 μl 100 mM Trispuffer (pH 8,0) und 10 μl Aktivatorlösung (Verdünnungsreihen SAK-Polypeptide) angesetzt. Dieses Gemisch wird 10 Minuten bei 25°C inkubiert (Aktivierungsproben).
- Das Plasminsubstrat Chromozym-PL (Boehringer) wird als 2 mM Lösung in $H_2O$ angesetzt. Zu 50 μl dieser Lösung werden 400 μl 100 mM Tris (pH 8,0) und und 50 μl aus dem Aktivierungsansatz gegeben. Dieses Gemisch wird 2 Minuten bei 25°C inkubiert. Danach wird die Reaktion mit 25 μl konzentrierter Essigsäure abgestoppt. Parallel dazu wird eine Plasmin-Eichreihe angesetzt, indem je 50 μl einer Plasmin-Verdünnungsreihe mit 400 μl 100 mM Tris-Puffer (pH 6,5) und 50 μl der Chromozym-PL-Lösung unter den gleichen Bedingungen inkubiert und abgestoppt werden. In Bezugsproben (Nullwertproben) wird die Umsetzung des Plasminsubstrats durch Zugabe von 25 μl konzentrierter Essigsäure in Kombination mit der Aktivierungsprobe unterbunden. Bezugsproben, Eichproben und die eigentlichen Meßproben (je 250 μl) werden in eine Mikrotiterplatte mit 96 Vertiefungen (Flachboden) pipettiert und die Absorption bei 405 nm in einem Multikanalphotometer gemessen.

Aus den Absorptionswerten der Meßproben kann unter Berücksichtigung der Absorptionswerte der Plasmin-Eichreihe und der Bezugsproben die Menge an Plasmin errechnet werden, die durch die in den Ausgangslösungen (Verdünnungsproben) enthaltenen SAK-Polypeptide pro Minute freigesetzt wird.

Unter Berücksichtigung des Proteingehalts sowie der Plasminfreisetzung können nunmehr die spezifischen Aktivitäten der SAK-Polypeptide ermittelt und verglichen werden. Auf diese Weise ergeben sich für die beschriebenen Staphylokinase-Formen die in Tabelle 2 zusammengefaßten spezifischen Aktivitäten.

Tabelle 2

| spezifische Aktivität SAK-Spezies in µmol Plasmin/(µmol SAK x min) $K_D$ in Mol/l | | |
|---|---|---|
| SAK42D | 16,2 | 1,48 x $10^6$ |
| SAKΔN10 | 22,7 | 2,21 x $10^{-6}$ |
| SAKΔ14 | 8,1 | 4,40 x $10^{-6}$ |
| SAKM26L | 36,6 | n.t. |
| SAKM26C | 21,1 | n.t. |

**Beispiel 12**

**Bestimmung der Dissoziationskonstanten von Staphylokinase-Plasminogen-Komplezen mittels analytischer Affinitätschromatographie**

Das Prinzip der Methode besteht darin, daß an einem geeigneten Träger immobilisiertes humanes Plasminogen in eine Chromatographiesäule eingebracht wird und hier mit SAK-Polypeptid in Wechselwirkung tritt, welches in bestimmten Quantitäten über die Säule gepumpt wird. Zwischen dem Elutionsvolumen $V_E$ und der jeweiligen SAK-Konzentration [SAK] in der Probe besteht folgender Zusammenhang:

$$1/(V_E - V_D) = K_D/M_{pg} + [SAK]/M_{pg}.$$

Dabei haben die Variablen folgende Bedeutung:

$V_D$ - Totvolumen der Säule
$V_E$ - Elutionsvolumen der SAK-Polypeptide
$M_{pg}$ - Menge des interagierenden immobilisierten Plasminogens in der Säulenpackung
[SAK] - Konzentration des jeweiligen SAK-Polypeptides in der aufgegebenen Probe
$K_D$ - Dissoziationskonstante des temporären Komplexes aus SAK-Form und Plasminogen.

Die Abhängigkeit des Kehrwertes der Differenz aus Säulentot und Elutionsvolumen der SAK-Formen von der Konzentration der SAK-Polypeptide in der aufgegebenen Probe ist über einen bestimmten Konzentrationsbereich linear. Aus dem Anstieg der Geraden kann $M_{pg}$ bestimmt werden, und durch Extrapolation auf [SAK] = 0 kann so die gesuchte Dissoziationskonstante ermittelt werden.

Experimentell wird das Problem derart gelöst, daß zunächst Plasminogen, das gemäß einer Vorschrift von Deutsch und Mertz (D. G. DEUTSCH und E. T. MERTZ , Science, 170 (1970), 1095-1096) aus Humanplasma isoliert worden ist, unter Benutzung einer Vorschrift der Firma Waters an Protein-Pak™ epoxy-activated (Waters) gekoppelt wird. Der festgestellte Beladungsgrad beträgt 5 mg Protein je mg Säulenmatrix. Mit dem so beladenen Affinitätsgel wird eine Chromatographiesäule HR 5/5 (Pharmacia) gefüllt, die mit einem Kühlmantel versehen und mit einem FPLC-Chromatographie-System (Pharmacia) verbunden wird. Alle beschriebenen Versuche werden bei 16°C mit einer Flußrate von 0,5 ml/min. unter Verwendung von 0,1 M Tris-HCl (pH-Wert 7,3) ausgeführt.

Nach der Konditionierung der Säule werden die SAK-Proben jeweils in 25 µl Volumenzonen auf die Säule aufgebracht, wobei die in diesen Volumina enthaltene Proteinmenge zwischen 0,5 g und 8 µg variiert wird. Zur exakten Ermittlung des jeweiligen Maximums des Elutionsvolumens ($V_E$) wird der im System vorhandene FPLC-Controller benutzt.

Unter Verwendung der geschilderten Methode werden die in Tabelle 2 dargestellten Dissoziationskonstanten für die Wechselwirkung von Plasminogen mit ausgewählten SAK-Polypeptiden erhalten .

**Beispiel 13**

**Herstellung monoklonaler anti-SAK-Antikörper-produzierender Hybridoma-Klone**

Die Immunisierung von weiblichen Balb/c/Han-Mäusen (Alter: 6-8 Wochen) wird mit hochreiner reifer SAK42D, gewonnen nach dem voranstehend angegebenen Verfahren aus dem rekombinanten Produzenten *E. coli* TG1 (pMEX602sak), durchgeführt, wobei der folgende Immunisierungsmodus eingehalten wird :

- Eine erste Immunisierungsdosis von 50 µg reiner SAK42D wird jedem Tier mit komplettem Freund'schen Adjuvans subkutan injiziert.
- Vier Wochen danach wird pro Tier eine zweite Dosis von wiederum 50 µg reiner SAK42D mit inkomplettem Freund'schen Adjuvans injiziert.
- Drei Wochen später wird jedem Tier eine letzte Dosis von erneut 50 µg reiner SAK42D ohne Adjuvans intravenös injiziert.

(Als komplettes bzw. inkomplettes Freund'sches Adjuvans werden die von der Fa. Difco vertriebenen Präparate verwendet.)

3 Tage nach der intravenösen SAK42D-Gabe werden die immunisierten Tiere getötet, und aus den Milzen wird eine hauptsächlich Lymphozyten enthaltende Zellsuspension in an sich bekannter Weise (in HAT-Medium; Ingredienzien werden von der Fa. Serva bezogen) präpariert. Anschließend erfolgt in standardgemäßer Weise der Fusionsschritt mit der Myelomzellinie P3-X-63 Ag8-653. Im Detail werden $10^7$ Myelomzellen mit $5x10^7$ Milzzellen in einem Volumen von 50 ml HAT-Medium koinkubiert.

Zur anschließenden Trennung der entstandenen Zellhybriden von nichtfusionierten Myelomzellen wird die obige Zellsuspension in 200 µl Portionen in die Vertiefungen (1 bis 2 Zellen pro Vertiefung) von Zellkulturplatten (96 Vertiefungen; Fa. Nunc) verteilt. Unter diesen Bedingungen wird 3 Wochen bei 37°C in einem Brutschrank mit Luftbefeuchtung und 5% $CO_2$-Beimischung inkubiert. Die folgende Einzelklon-Kultivierung (Dauer 2 Wochen; übrige Bedingungen wie oben) wird in HT-Medium (Ingredienzien gleichfalls von Serva) vorgenommen. Danach werden sämtliche Einzelklon-Kulturen in RPMI-Standardmedium (Serva), das 15% fötales Kälberserum (Flow) enthält, weiterkultiviert. In diesem Verfahrensstadium erfolgt mit Hilfe eines Festphasen-ELISA die Testung der einzelnen Hybridoma-Zellklone auf eine Produktion von SAK-spezifischen Antikörpern. Verwendet werden in diesem Assay Mikrotestplatten, bei denen an die Oberfläche ihrer Vertiefungen SAK42D (5 µg/Vertiefung; PBS-Puffer) gebunden ist. Die Blockierung der so vorbehandelten Vertiefungen erfolgt mit gelatinehaltigem (0,5% Gelatine) PBS-Puffer der 0,05% Tween-20 (Serva) enthält. Anschließend werden die zu testenden Hybridoma-Kulturüberstände in die Vertiefungen gefüllt. Die Feststellung der Bindung von in den Kulturüberständen enthaltenen anti-SAK-Antikörpern erfolgt mit einem Antikörperkonjugat, das aus mit Peroxidase markierten Ziege-Anti-Mausimmunglobulin-Antikörpern (medac) besteht.

Auf diese Weise werden 11 Hybridoma-Klone identifiziert, die zur Biosynthese von anti-SAK-Antikörpern befähigt sind. Die Zellklone werden in diesem Stadium konserviert und eingefroren.

Die beiden Hybridoma-Linien mit den Arbeitsbezeichnungen alphaSAK IG8/H6 und alphaSAK IIA4/A10 durchlaufen in RPMI-Medium mit 15%-igem FKS-Zusatz (Lieferfirmen s.o.) ein Reklonierungsverfahren (Dauer 14 Tage; 37°C; Brutschrank mit Luftbefeuchtung und 5%-iger $CO_2$-Beimischung). Danach wird von jedem der beiden Klone (durch fortgesetzte Kultivierung in RPMI-Medium) kumulativ eine größere Menge Kulturübertstand gewonnen (ca. 2 1), die jeweils die Gewinnung der anti-SAK-Antikörper IG8/H6 und IIA4/A10 in reiner Form durch Affinitätschromatographie an immobilisierter SAK42D ermöglicht. Dazu wird rekombinante SAK42D an CNBr-Sepharose-4B (Pharmacia) gekoppelt (10mg Protein/ml Sepharose). Die entsprechenden Hybridoma-Kulturüberstände werden zusätzlich mit NaC1 (Endkonzentration 0,5 M) und Tween-20 (Endkonzentration 0,05%; Serva) versetzt und anschließend über eine Säule chromatographiert, welche die immobilisierte SAK42D enthält. Die Säulenwaschung wird mit PBS-Puffer, der zusätzlich 0,5 M NaCl enthält, durchgeführt. Die Elution der gebundenen anti-SAK-Antikörper wird mit 0,5 M NaCl; 0,2 M Glycin; pH 2,8 durchgeführt. Die proteinhaltigen Eluatfraktionen werden sofort mit NaOH neutralisiert.

Die gereinigten Antikörper stehen fortan für die Verwendung zu den angegebenen Zwecken (vgl. Beispiele 14 bis 18) bereit.

**Beispiel 14**

**Einsatz des monoklonalen anti-SAK Antikörpers IIA4/A10 für Westernblot-Versuche**

SAK42D-haltige Proteinproben (z.B. Rohlysate) werden in SDS-Polyacrylamid-Gelen elektrophoretisch aufgetrennt und danach auf an sich bekannte Weise auf Nitrozellulose transferiert. Durch eine temporäre Proteinfärbung mittels Ponceau-S (Serva) wird die Lage der Proteinbanden auf der Nitrozellulose-Vorlage sichtbar gemacht, bestimmten Molekulargewichtsmarkern zugeordnet und gegebenenfalls markiert. Danach wird der Nitrozellulose-Blot mit Waschpuffer (0,5 M NaCl,PBS, 0,05% Tween 20; Serva), der 5% Magermilchpulver enthielt, blockiert (ca. 2 Stunden bei Zimmertemperatur).

Im nächsten Schritt erfolgt die Behandlung mit dem Antikörper IIA4/A10, der in Waschpuffer (enthielt 1% Magermilchpulver) 1000-fach verdünnt wird. Anschließend wird der Nitrozellulose-Blot mit einem Ziege-Anti-Mausimmunglobulin-Antikörper-POD-Konjugat (medac) (Verdünnung 1000-fach in Waschpuffer mit 1% Magermilchpulver) behandelt. Die "Entwicklung" des Western-Blots erfolgt mit Diaminobenzidin in Gegenwart von Wasserstoffperoxid. Angefärbt werden nur SAK42D- sowie die Banden anderer SAK-Polypeptide auf der Nitrozellulose-Vorlage (Figur 13b). Das Verfahren kann zur Identifizierung der nativen SAK42D sowie zur Epitop-Charakterisierung unter Einbeziehung von Staphylokinase-Polypeptiden eingesetzt werden.

## Beispiel 15

### ELISA-Testsystem zum quantitativen Nachweis von Staphylokinase

Mikrotitrationsplatten werden zunächst mit einem polyklonalen Schwein-anti-SAK-Antikörper, der affinitätschromatografisch gereinigt wurde, beschichtet (2 µg/Vertiefung).

Besagte Antikörper werden gewonnen, indem zunächst ein Schwein dreimal mit je 500 µg reiner SAK42D immunisiert wird. Das anschließend gewonnene Hyperimmunserum wird an immobilisierter SAK42D (analog dem im Beispiel 13 beschriebenen Verfahren) gereinigt. Die Dauer der Beschichtungsprozedur beträgt 15 Stunden bei 4°C oder 2 Stunden bei 37°C. Anschließend wird wie in Beispiel 13 mit gelatinehaltigem PBS-Puffer behandelt, um die Plastikoberfläche zu blockieren.

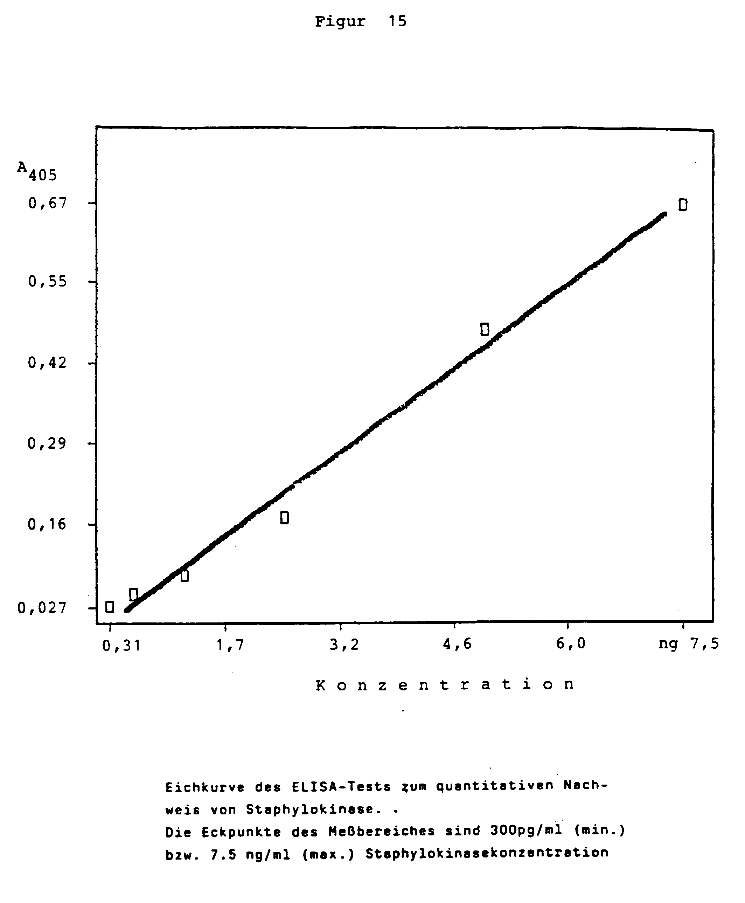

Danach wird eine Standardverdünnungsreihe aus reiner SAK42D in gelatinehaltigem PBS-Puffer hergestellt. Gleichzeitig werden die Arbeitsverdünnungen der zu testenden Proben angesetzt. Standardreihe und Arbeitsverdünnungen werden in die Vertiefungen gefüllt und 60 Minuten bei 37°C inkubiert. Nach einem Waschvorgang wird der Peroxidase-konjugierte Anti-SAK-Antikörper IG8/H6 in 1000-facher Verdünnung (Verdünnungsmedium ist gelatinehaltiger PBS-Puffer) in die Vertiefungen der Testplatte gefüllt. Die quantitative Auswertung des Tests erfolgt kolorimetrisch bei einer Wellenlänge von 492 nm unter Einsatz des Substrats $H_2O_2$ und Orthophenylendiamin. Die dabei gewonnene Eichkurve verläuft über einen Konzentrationsbereich von 300 pg/ml bis 7,5 ng/ml SAK linear (vergleiche Figur 15).

Der Test erlaubt die quantitative SAK-Bestimmung in Bakterienaufschlüssen, in der begleitenden Analytik bei der SAK-Reinigung sowie in humanem Serum.

## Beispiel 16

### Einsatz des monoklonalen Antikörpers IGB/H6 zur Immmpräzipitation von SAK-Polypeptiden

Die Technik der Immunpräzipitation wird zur Identifizierung von *in vivo* und *in vitro* synthetisierter, radioaktiv markierten SAK-Polypeptiden in Zellysaten eingesetzt.

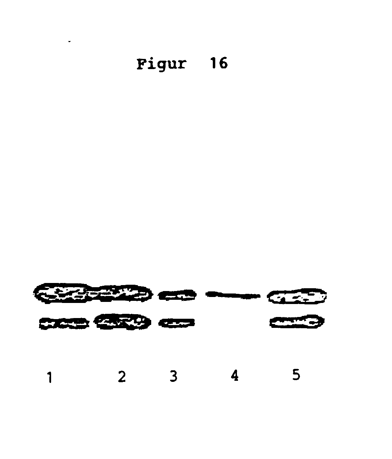

Das zu testende Zellysat wird mit einer SDS-Lösung gemischt und gekocht (Endkonzentration des SDS 1%) und anschließend 10-fach mit STD-Tris-Puffer (0,9% NaCl, 1% Triton X-100, 0,5% Natriumdesoxycholat, 0,1 M Tris-HCl; pH 8,2) verdünnt. Diesem Gemisch wird 1 µl des Antikörpers IG8/H6 (Suspension eines Ammoniumsulfatpräzipitats [50% Sättigung]) zugesetzt und über Nacht bei 4°C inkubiert. Zur Fällung des Antigen-Antikörper-Komplexes werden 10 mg Protein A-SepharoseCL-4B (Pharmacia), vorgequollen in 100 µl STD-Tris-Puffer, zugesetzt und 60 Minuten bei Raumtemperatur geschüttelt. Der Ansatz wird anschließend zentrifugiert, der Überstand verworfen und das Pellet 3 mal mit je 5700 µl STD-Tris-Puffer gewaschen. Nach dem Waschen wird das Pellet in 40 µl SDS-PAGE-Probenpuffer aufgenommen, gekocht und auf ein SDS-Polyakrylamidgel aufgetragen. Die Auswertung erfolgt autoradiografisch. Das Ergebis eines Immunpräzipitationsansatzes ist in Figur 16 dargestellt.

## Beispiel 17

### Nachweis der die plasminogenaktivierende Kapazität von SAK-Polypeptiden hemmenden Aktivität der mAK IG8/H6 und IIA4/A10

Unverdünnte Kulturüberstände der beiden Hybridoma-Klone (je 25 µl) werden mit je 25 µl einer verdünnten SAK42D-Probe (Konzentration: 20 ng Protein / 25 µl) in Phosphatpuffer (10 mM pH 6,5) gemischt (Testansatz). Als Kontrollen dienen Ansätze, denen anstelle von SAK42D der obige Phosphatpuffer bzw. Streptokinase (Kabikinase, Kabivitrum) verdünnt in Phosphatpuffer, beigegeben ist (Kontrollansätze). Diese Ansätze werden 30 Minuten bei Zimmertemperatur inkubiert. Danach werden je 10 µl aus den Test- und Kontrollansätzen entnommen und in einen weiteren Versuchsansatz eingebracht, der die Messung der durch ein erfindungsgemäßes SAK-Polypeptid, vorzugsweise durch SAK42D, induzierten Plasmin-Freisetzung gestattet (Durchführung des Tests in einer Mikrotestplatte mit 96 Vertiefungen). Dazu werden die o.g. 10 µl-Aliquote mit 30 µl einer Plasminogenlösung (20 mg/ml Plasminogen [Behring], gelöst in $H_2O$) und 150 µl Trispuffer (100 mM Tris, pH 8,0) versetzt und 10 Minuten bei 25°C inkubiert.

Danach werden je 50 µl entnommen und einem weiteren Ansatz, bestehend aus 400 µl Tris-Puffer (100 mM Tris, pH 8,0) sowie aus 50 µl Plasminsubstrat CHROMOZYM-PL (Boehringer; 2mM Lösung in $H_2O$), zugesetzt und 2 Minuten bei 25°C inkubiert. Die Reaktion wird mit je 25 µl konzentrierter Essigsäure gestoppt. Die bei einer Wellenlänge von 405 nm gemessene Absorption steht in direktem Verhältnis zur ursprünglich durch SAK42D induzierten Plasminfreisetzung. Eine die Wirkung von SAK-Polypeptiden hemmende Aktivität der Antikörper äußert sich in verminderten Absorptionswerten und damit in einer verminderten Plasminfreisetzung.

Im Falle der monokonalen Antikörper IG8/H6 und IIA4/A10 ist dieser Effekt deutlich nachweisbar: In der angegebenen Versuchsanordnung hemmen die Antikörper IG8/H6 und IIA4/A10 die Plasminfreisetzung um das 18,8-fache bzw. um das 17,5-fache.

**Beispiel 18**

**Einsatz des mAK IG8/H6 zur immunaffinitätschro-matografischen Reinigung von Staphylokinase**

Das Verfahren besteht aus zwei Teilschritten. Im ersten Schritt (1) wird der mAK IG8/H6 an einem chromatografiefähigen Träger immobilisiert. Der zweite Schritt (2) umfaßt die unmittelbare chromatografische Gewinnung von nativer SAK42D bzw. von SAK-Polypeptiden z.B. aus Bakterienrohextrakten.

(1) Der affinitätschromatografisch gereinigte mAK IG8/H6 (Gesamtmenge 20 mg) wird zunächst gegen Kopplungspuffer (0,5 M NaCl, 0,1 M $NaHCO_3$; pH 8,3) dialysiert. Die Kopplung erfolgt an CNBr-Sepharose CL-4B (Pharmacia). Eine entsprechende Menge dieser Sepharose (0,7 g) wird in 1 mM HC1 vorgequollen . Die Kopplung des mAK selbst erfolgt in Kopplungspuffer (2 Stunden bei Zimmertemperatur unter gelegentlichem vorsichtigem Rühren und anschließend über Nacht). Danach wird der Überstand abgenommen und die Sepharose mehrfach mit Kopplungspuffer gewaschen.
Anschließend wird die mit mAK IG8/H6 beladene Sepharose mit 1 M Ethanolamin (pH 8,0) behandelt (2 Stunden bei Zimmertemperatur). An diesen Schritt schließen sich Waschungen mit 0,1 M Boratpuffer, der 0,5 M NaCl (pH 8,0) enthält, und 0,1 M Acetatpuffer, der 0,5 M NaCl enthält (pH 4,0), an. Die Waschungen werden je 5 mal im Wechsel Borat-Puffer/Acetat-Puffer durchgeführt. Zum Schluß wird mit 0,2 M Glycin/HCl-Puffer (pH 2,8), der 0,5 M NaCl enthält, gewaschen und die Sepharose anschließend in PBS-Puffer überführt.

(2) Die mit mAK IG8/H6 beladene Sepharose wird in eine Chromatographiesäule (Durchmesser 1 cm) überführt und mit PBS-Puffer, der 0,05 % Tween-20 und zusätzlich 0,5 M NaCl enthielt, gewaschen (mindestens 20 Säulenvolumen) . Die in 4-fach konzentriertem PBS-Puffer unter Zusatz von Protease-Hemmsubstanzen hergestellten, SAK-enthaltenden Bakterienaufschlüsse werden durch Verdünnen mit destilliertem Wasser und unter Zusatz von NaCl und Tween-20 in eine Pufferkomposition gebracht, die dem Säulenwaschpuffer entspricht . Danach erfolgt die Beladung der Affinitätssäule mit diesem Material. Anschließend wird mit PBS-Puffer, der 0,5 M NaCl (aber kein Tween-20) enthält, gewaschen (mindestens 20 Säulenvolumina) bis kein Protein mehr von der Säule eluiert wird (UV-Detektor; Wellenlänge 280 nm). Die Elution der Säule erfolgt mit 0,2 M Glycin/HCl-Puffer (pH 2,8), der 0,5 M NaCl enthält. Die proteinhaltigen Eluatfraktionen werden ebenso wie die Säulenmatrix umgehend neutralisiert.

Im Säuleneluat liegen SAK42D bzw. die SAK-Polypeptide in elektrophoretisch reiner Form vor.

**Tabelle 1**

| Bezeichnung der Linker | Sequenz der Linker vom 5'-Ende zum 3'-Ende |
|---|---|
| L1 | A G C T T G A A T T C A G G A G G C C T C A T A T G T C A A G T T C A T |
| L2 | T C G A A T G A A C T T G A C A T A T G A G G C C T C C T G A A T T C A |
| L3 | A T T C A A C T T A A T T A C A A A G G T T G T T A T A G A A A A G A A A T A A C T G C A |
| L4 | G T T A T T T C T T T T C T A T A A C A A C C T T T G T A A T T A A G T T G |
| L5 | C C T C A T A T G A A A G G C G A T G A C G |
| L6 | C G T C A T C G C C T T T C A T A T G A G G |
| L7 | C C T C A T A T G G C G A G T T A T T T T G A A C C A C A G G |
| L8 | C C T G T G G T T C A A A A T A A C T C G C C A T A T G A G G |
| L9 | A A T T C A G G A G G C C T C A T A T G T C A A G T T C A T T C G A A G |
| L10 | G A T C C T T C G A A T G A A C T T G A C A T A T G A G G C C T C C T G |
| L11 | C G A C A A A G G A A A A T T T A A A A A A G G C G A T |
| L12 | C G C G T C A T C G C C T T T T T T A T A T T T T C C T T T G T |
| L13 | G A C G C G A G T T A T T T T G A A C C A A C A G G C C G G C A T G |
| L14 | C C G G C C T G T T G G T T C A A A A T A A C T |
| L15 | A T T C A A C T T A A T T A C C A A G G T T G T T A T A G A A A A G A A A T A A C T G C A |
| L16 | G T T A T T T C T T T T C T A T A A C A A C C T T G G T A A T T A A G T T G |

**Tabelle 1 (Fortsetzung)**

| | |
|---|---|
| L17 | A A T T C G T C G A C G G T A A A A G A A A T G A G C |
| | T C T T G T C C C C G C G G T A T G T |
| L18 | C A T A C C G C G G G A C A A G A G C T C A T T T C |
| | T T T T A C C G T C G A C G |
| AG1 | C G T A T T T G C G A G T A A A T G T G A C T G G A G |
| | A A |
| | G C |
| | T |
| AG2 | T C G A C T C C A G T C A C A T T T A C T C G C A A A |
| | A C |
| | G T |
| | T |
| | T A C G |
| AG3 | C G T A T T T G C C C G T A A A T G T G A C T G G A G |
| | G A |
| | T G |
| | T |
| AG4 | T C G A C T C C A G T C A C A T T T A C G C C C A A A |
| | A A |
| | G G |
| | T |
| | T A C G |
| SG 26 | T G T A G T C C C A G G T T T A A T A G G |

**Patentansprüche**

1. Für Staphylokinase-Polypeptide mit Plasminogen-Aktivator-Wirkung kodierende DNA-Sequenzen, **dadurch gekennzeichnet,** daß die Sequenzen frei sind von Regionen, die für Signalpeptide kodieren.

2. DNA-Sequenzen nach Anspruch 1, **dadurch gekennzeichnet**, daß sie die Nukleotidfolge gemäß Figur 1 (SEQ ID Nr. 1) aufweisen, sowie allelische Varianten und/oder Fragmente derselben, soweit diese für Staphylokinase-Polypeptide mit Plasminogen-Aktivator-Wirkung kodieren.

**3.** DNA-Sequenzen nach den Ansprüchen 1 oder 2, **dadurch gekennzeichnet**, daß sie die Nukleotidfolge der natürlichen allelischen Varianten gemäß den Figuren 2 (SEQ ID Nr. 2) oder 3 (SEQ ID Nr. 3) aufweisen.

**4.** DNA-Sequenz nach den Ansprüchen 1 oder 2, **dadurch gekennzeichnet**, daß sie die Nukleotidfolge des Fragments gemäß Figur 4 (SEQ ID Nr. 4) aufweist.

**5.** DNA-Sequenzen nach den Ansprüchen 1 oder 2, **dadurch gekennzeichnet**, daß sie die Nukleotidfolge der artifiziellen allelischen Varianten gemäß den Figuren 6 (SEQ ID Nr. 6) oder 7 (SEQ ID Nr. 7) aufweisen.

**6.** Expressionsplasmide, **dadurch gekennzeichnet**, daß sie eine der DNA-Sequenzen nach den Ansprüchen 1 bis 5 operativ verknüpft mit Expressionskontroll-Sequenzen enthalten, wobei die Expressionsplasmide frei sind von Sequenzen, die für Staphylokinase-Signalpeptide kodieren.

**7.** Expressionsplasmide nach Anspruch 6, **dadurch gekennzeichnet,** daß sie eine DNA-Sequenz nach den Ansprüchen 1 bis 5 enthalten, an ihrem 5'-Ende operativ verknüpft mit der Translations-Initiations-Region gemäß Figur 8 (SEQ ID Nr. 8).

**8.** Wirtszellen, **dadurch gekennzeichnet,** daß sie Expressionsplasmide nach den Ansprüchen 6 oder 7 tragen und zur Expression von Staphylokinase-Polypeptiden mit Plasminogen-Aktivator-Wirkung befähigt sind.

**9.** Wirtszellen nach Anspruch 8, **dadurch gekennzeichnet**, daß sie prokaryotische Zellen sind.

**10.** Wirtszellen nach Anspruch 9, **dadurch gekennzeichnet**, daß sie der Spezies *E. coli* angehören.

**11.** Wirtszellen nach Anspruch 9, **dadurch gekennzeichnet,** daß sie der Gattung *Bacillus spec.* angehören.

**12.** Verfahren zur rekombinanten Herstellung von Staphylokinase-Polypeptiden mit Plasminogen-Aktivator-Wirkung als Ziel-Polypeptid, **dadurch gekennzeichnet**, daß man Wirtszellen nach den Ansprüchen 8 bis 11 in einem geeigneten Medium kultiviert und die Ziel-Polypeptide aus den Wirtszellen isoliert.

**13.** Verfahren nach Anspruch 12, **dadurch gekennzeichnet,** daß die Wirtszellen der Spezies *E. coli* angehören.

**14.** Verfahren nach den Ansprüchen 12 oder 13, **dadurch gekennzeichnet**, daß man zur Isolierung des Ziel-Polypeptids

a) die Wirtszellen aufschließt und die löslichen Anteile des Aufschlusses von den unlöslichen Anteilen trennt,

b) die löslichen Anteile direkt auf einen Ionenaustauscher leitet,

c) das Ziel-Polypeptid selektiv mit einer wäßrigen NaCl-Lösung von dem Ionenaustauscher eluiert,

d) das Eluat nach Anreichern mit NaCl direkt einer hydrophoben Interaktionschromatographie zuleitet und

e) das Ziel-Polypeptid selektiv mit einem absteigenden NaCl-Gradienten von dem hydrophoben Interaktions-Chromatographie-Medium eluiert und anschließend aus dem Eluat gewinnt.

**15.** Verfahren nach Anspruch 14, **dadurch gekennzeichnet**, daß die NaCl-Konzentration in den Stufen

c) von 0 bis 500 mM,
d) von 2,0 bis 30 M und
e) von 20 bis 30 M als Anfangskonzentration und von 15 bis 100 mM als Endkonzentration betragen.

**16.** Verfahren nach den Ansprüchen 12 bis 15, **dadurch gekennzeichnet**, daß das isolierte Staphylobimase-Polypeptid nach folgend zu einer pharmazeutischen Zusammensetzung formuliert wird.

17. Verfahren nach Anspruch 16, **dadurch gekennzeichnet,** daß die Formulierung durch Kombination mit pharmazeutisch verträglichen Verdünnungsmitteln, Adjuvantien oder Trägerstoffen erfolgt.

## Claims

1. DNA sequences coding for staphylokinase polypeptides with plasminogen activator effect, **characterized in that** the sequences are free from regions which code for signal peptides.

2. DNA sequences according to Claim 1, **characterized in that** they display the nucleotide series according to Figure 1 (SEQ ID No. 1) plus allelic variants and/or fragments of same, if these code for staphylokinase polypeptides with plasminogen activator effect.

3. DNA sequences according to Claims 1 or 2, **characterized in that** they display the nucleotide series of the natural allelic variants according to Figures 2 (SEQ ID No. 2) or 3 (SEQ ID No. 3).

4. DNA sequence according to Claims 1 or 2, **characterized in that** it displays the nucleotide series of the fragment according to Figure 4 (SEQ ID No. 4).

5. DNA sequences according to Claims 1 or 2, **characterized in that** they display the nucleotide series of the artificial allelic variants according to Figures 6 (SEQ ID No. 6) or 7 (SEQ ID No. 7).

6. Expression plasmids, **characterized in that** they contain one of the DNA sequences according to Claims 1 to 5, operatively linked with expression control sequences, the expression plasmids being free from sequences which code for staphylokinase signal peptides.

7. Expression plasmids according to Claim 6, **characterized in that** they contain a DNA sequence according to Claims 1 to 5, operatively linked at their 5'-end with the translation/initiation region according to Figure 8 (SEQ ID No. 8).

8. Host cells, **characterized in that** they carry expression plasmids according to Claims 6 or 7 and are capable of the expression of staphylokinase polypeptides with plasminogen activator effect.

9. Host cells according to Claim 8, c**haracterized in that** they are procaryotic cells.

10. Host cells according to Claim 9, **characterized in that** they belong to the species *E. coli.*

11. Host cells according to Claim 9, **characterized in that** they belong to the genus *Bacillus spec.*

12. Process for the recombinant production of staphylokinase polypeptides with plasminogen activator effect as target polypeptide, **characterized in that** host cells according to Claims 8 to 11 are cultivated in a suitable medium and the target polypeptides are isolated from the host cells.

13. Process according to Claim 12, **characterized in that** the host cells belong to the species *E. coli.*

14. Process according to Claims 12 or 13, **characterized in that**, for the isolation of the target polypeptide

    a) the host cells are decomposed and the soluble portions of the decomposition are separated from the insoluble portions,

    b) the soluble portions are passed directly onto an ion exchanger,

    c) the target polypeptide is selectively eluted from the ion exchanger with an aqueous NaCl solution,

    d) the eluate is conducted directly to a hydrophobic interaction chromatography after enrichment with NaCl and

    e) the target polypeptide is selectively eluted with a falling NaCl gradient from the hydrophobic interaction chromatography medium and then obtained from the eluate.

**15.** Process according to Claim 14, **characterized in that** the NaCl concentrations in the stages are

c) from 0 to 500 mM,
d) from 2.0 to 30 M and
e) from 20 to 30 M as initial concentration and from 15 to 100 mM as final concentration.

**16.** Process according to claims 12 to 15, characterized in that the isolated staphylokinase polypeptide is subsequently formulated as a pharmaceutical preparation.

**17.** Process according to claim 16, characterized in that the formulation is effected by combination with pharmaceutically acceptable diluents, adjuvants or carrier agents.

**Revendications**

**1.** Séquences d'ADN codant pour un polypeptide de staphylokinase ayant une activité d'activateur de plasminogène, caractérisées en ce que les séquences sont exemptes de régions qui codent pour un peptide signal.

**2.** Séquences d'ADN selon la revendication 1, caractérisées en ce qu'elles présentent la séquence nucléotidique selon la figure 1 (SEQ ID N° 1), ainsi que les variants et/ou fragments alléliques de cette séquence, dans la mesure où ils codent pour un polypeptide de staphylokinase ayant une activité d'activateur de plasminogène.

**3.** Séquences d'ADN selon les revendications 1 ou 2, caractérisées en ce qu'elles présentent la séquence nucléotidique des variants d'allèles naturels selon la Figure 2 (SEQ ID N° 2) ou 3 (SEQ ID N° 3).

**4.** Séquences d'ADN selon les revendications 1 ou 2, caractérisées en ce qu'elles présentent la séquence nucléotidique du fragment selon la Figure 4 (SEQ ID N° 4).

**5.** Séquences d'ADN selon les revendications 1 ou 2, caractérisées en ce qu'elles présentent la séquence nucléotidique des variants d'allèles synthétiques selon la Figure 6 (SEQ ID N° 6) ou la Figure 7 (SEQ ID N° 7).

**6.** Plasmides d'expression, caractérisés en ce qu'ils contiennent une des séquences d'ADN selon les revendications 1 à 5, reliée de façon opérante avec des séquences de contrôle d'expression, les plasmides d'expression étant exempts de séquences codant pour un peptide signal de staphylokinase.

**7.** Plasmides d'expression selon la revendication 6, caractérisés en ce qu'ils contiennent une séquence d'ADN selon les revendications 1 à 5 liée de façon opérante à son extrémité 5' à la région d'initiation de translation selon la Figure 8 (SEQ ID N° 8).

**8.** Cellules hôtes, caractérisées en ce qu'elles portent des plasmides d'expression selon les revendications 6 ou 7 et sont capables d'exprimer des polypeptides de staphylokinase ayant une activité d'activateur de plasminogène.

**9.** Cellules hôtes selon la revendication 8, caractérisées en ce qu'elles sont des cellules prokaryotes.

**10.** Cellules hôtes selon la revendication 9, caractérisées en ce qu'elles appartiennent à l'espèce *E. coli.*

**11.** Cellules hôtes selon la revendication 9, caractérisées en ce qu'elles appartiennent au genre *Bacillus spec.*

**12.** Procédé de préparation par voie recombinante d'un polypeptide cible qui est un polypeptide de staphylokinase ayant une activité d'activateur de plasminogène, caractérisé en ce que l'on cultive des cellules hôtes selon les revendications 8 à 11 dans un milieu approprié et l'on isole le polypeptide cible à partir des cellules hôtes.

**13.** Procédé selon la revendication 12, caractérisé en ce que les cellules hôtes appartiennent à l'espèce *E. coli.*

**14.** Procédé selon les revendications 12 ou 13, caractérisé en ce que pour l'isolement des polypeptides cibles

a) l'on décompose les cellules hôtes et l'on sépare la fraction soluble de la fraction insoluble du produit de décomposition,

b) l'on fait passer la fraction soluble directement sur un échangeur d'ions,
c) le polypeptide cible est élué sélectivement de l'échangeur d'ions avec une solution aqueuse de NaCl,
d) l'éluat est soumis directement après l'enrichissement avec NaCl à une chromatographie d'affinité hydrophobe et
e) le polypeptide cible est élué sélectivement du milieu de chromatographie d'affinité hydrophobe avec un gradient décroissant de NaCl et ensuite récupéré à partir de l'éluat.

**15.** Procédé selon la revendication 14, caractérisé en ce que la concentration en NaCl dans les étapes suivantes est

c) de 0 à 500 mM,
d) de 2,0 à 30 M et
e) de 20 à 30 M comme concentration de départ et de 15 à 100 mM comme concentration finale.

**16.** Procédé selon les revendications 12 à 15, caractérisé en ce que le polypeptide de staphylokinase isolé est ensuite mis sous forme de compostion pharmaceutique.

**17.** Procédé selon la revendication 16, caractérisé en ce que la formulation est réalisée par combinaison avec des diluants, adjuvants ou véhicules pharmaceutiquement acceptables.

**Figur 1**

SEQ ID NO:1
ART DER SEQUENZ:Nucleotid mit entsprechendem Protein
SEQUENZLÄNGE:414 Basenpaare
STRANGFORM:Einzelstrang
TOPOLOGIE:linear
ART DES MOLEKÜLES:rekombinante DNA
URSPRÜNGLICHE HERKUNFT ORGANISMUS:Staphylococcus aureus Phage 42D
UNMITTELBARE EXPERIMENTELLE HERKUNFT:Plasmid pMET5
MERKMALE:
von 1 bis 3 bp ATG-Startcodon
von 4 bis 411 bp reifes Protein
von 412 bis 414 bp TAA-Stopcodon

```
                 10                  20                  30                  40
  1  ATG TCA AGT TCA TTC GAC AAA GGA AAA TAT AAA AAA GGC GAT GAC
     MET Ser Ser Ser Phe Asp Lys Gly Lys Tyr Lys Lys Gly Asp Asp

         50                60                  70                80                  90
 46  GCG AGT TAT TTT GAA CCA ACA GGC CCG TAT TTG ATG GTA AAT GTG
     Ala Ser Tyr Phe Glu Pro Thr Gly Pro Tyr Leu MET Val Asn Val

                 100                 110                 120                 130
 91  ACT GGA GTT GAT GGT AAA AGA AAT GAA TTG CTA TCC CCT CGT TAT
     Thr Gly Val Asp Gly Lys Arg Asn Glu Leu Leu Ser Pro Arg Tyr

         140                 150                 160                 170                 180
136  GTC GAG TTT CCT ATT AAA CCT GGG ACT ACA CTT ACA AAA GAA AAA
     Val Glu Phe Pro Ile Lys Pro Gly Thr Thr Leu Thr Lys Glu Lys

                 190                 200                 210                 220
181  ATT GAA TAC TAT GTC GAA TGG GCA TTA GAT GCG ACA GCA TAT AAA
     Ile Glu Tyr Tyr Val Glu Trp Ala Leu Asp Ala Thr Ala Tyr Lys

         230                 240                 250                 260                 270
226  GAG TTT AGA GTA GTT GAA TTA GAT CCA AGC GCA AAG ATC GAA GTC
     Glu Phe Arg Val Val Glu Leu Asp Pro Ser Ala Lys Ile Glu Val

                 280                 290                 300                 310
271  ACT TAT TAT GAT AAG AAT AAG AAA AAA GAA GAA ACG AAG TCT TTC
     Thr Tyr Tyr Asp Lys Asn Lys Lys Lys Glu Glu Thr Lys Ser Phe

         320                 330                 340                 350                 360
316  CCT ATA ACA GAA AAA GGT TTT GTT GTC CCA GAT TTA TCA GAG CAT
     Pro Ile Thr Glu Lys Gly Phe Val Val Pro Asp Leu Ser Glu His
```

**Figur 1 (Fortsetzung)**

```
                   370                 380                 390                   400
361  ATT AAA AAC CCT GGA TTC AAC TTA ATT ACM AAG GTT GTT ATA GAA
     Ile Lys Asn Pro Gly Phe Asn Leu Ile Thr Lys Val Val Ile Glu


         410
406  AAG AAA TAA
     Lys Lys ---
```

**Figur 2**

SEQ ID NO:2
ART DER SEQUENZ:Nucleotid mit entsprechendem Protein
SEQUENZLÄNGE:414 Basenpaare
STRANGFORM:Einzelstrang
TOPOLOGIE:linear
ART DES MOLEKÜLES:rekombinante DNA
URSPRÜNGLICHE HERKUNFT ORGANISMUS:Staphylococcus aureus Phage øC
UNMITTELBARE EXPERIMENTELLE HERKUNFT:
MERKMALE:
von 1 bis 3 bp ATG-Startcodon
von 4 bis 411 bp reifes Protein
von 412 bis 414 bp TAA-Stopcodon

```
                   10                  20                  30                  40
  1   ATG TCA AGT TCA TTC GAC AAA GGA AAA TAT AAA AAG GGC GAT GAC
      MET Ser Ser Ser Phe Asp Lys Gly Lys Tyr Lys Lys Gly Asp Asp

          50                  60                  70                  80                  90
 46   GCG AGT TAT TTT GAA CCA ACA GGC CCG TAT TTG ATG GTA AAT GTG
      Ala Ser Tyr Phe Glu Pro Thr Gly Pro Tyr Leu MET Val Asn Val

                  100                 110                 120                 130
 91   ACT GGA GTT GAT GGT AAA GGA AAT GAA TTG CTA TCC CCT CAT TAT
      Thr Gly Val Asp Gly Lys Gly Asn Glu Leu Leu Ser Pro His Tyr

         140                 150                 160                 170                 180
136   GTC GAG TTT CCT ATT AAA CCT GGG ACT ACA CTT ACA AAA GAA AAA
      Val Glu Phe Pro Ile Lys Pro Gly Thr Thr Leu Thr Lys Glu Lys

                  190                 200                 210                 220
181   ATT GAA TAC TAT GTC GAA TGG GCA TTA GAT GCG ACA GCA TAT AAA
      Ile Glu Tyr Tyr Val Glu Trp Ala Leu Asp Ala Thr Ala Tyr Lys

         230                 240                 250                 260                 270
226   GAG TTT AGA GTA GTT GAA TTA GAT CCA AGC GCA AAG ATC GAA GTC
      Glu Phe Arg Val Val Glu Leu Asp Pro Ser Ala Lys Ile Glu Val

                  280                 290                 300                 310
271   ACT TAT TAT GAT AAG AAT AAG AAA AAA GAA GAA ACG AAG TCT TTC
      Thr Tyr Tyr Asp Lys Asn Lys Lys Lys Glu Glu Thr Lys Ser Phe

         320                 330                 340                 350                 360
316   CCT ATA ACA GAA AAA GGT TTT GTT GTC CCA GAT TTA TCA GAG CAT
      Pro Ile Thr Glu Lys Gly Phe Val Val Pro Asp Leu Ser Glu His
```

**Figur 2 (Fortsetzung)**

```
                370             380             390             400
361  ATT AAA AAC CCT GGA TTC AAC TTA ATT ACA AAG GTT GTT ATA GAA
     Ile Lys Asn Pro Gly Phe Asn Leu Ile Thr Lys Val Val Ile Glu

        410
406  AAG AAA TAA
     Lys Lys ---
```

# Figur 3

SEQ ID NO:3
ART DER SEQUENZ:Nucleotid mit entsprechendem Protein
SEQUENZLÄNGE:414 Basenpaare
STRANGFORM:Einzelstrang
TOPOLOGIE:linear
ART DES MOLEKÜLES:rekombinante DNA
URSPRÜNGLICHE HERKUNFT ORGANISMUS: Staphylococcus aureus Stamm 23, genomische DNA

UNMITTELBARE EXPERIMENTELLE HERKUNFT:
MERKMALE:
von 1 bis 3 bp ATG-Startcodon
von 4 bis 411 bp reifes Protein
von 412 bis 414 bp TAA-Stopcodon

```
                 10          20          30          40
  1  ATG TCA AGT TCA TTC GAC AAA GGA AAA TAT AAA AAA GGC GAT GAC
     MET Ser Ser Ser Phe Asp Lys Gly Lys Tyr Lys Lys Gly Asp Asp

         50          60          70          80          90
 46  GCG AGT TAT TTT GAA CCA ACA GGC CCG TAT TTG ATG GTA AAT GTG
     Ala Ser Tyr Phe Glu Pro Thr Gly Pro Tyr Leu MET Val Asn Val

                100         110         120         130
 91  ACT GGA GTT GAT AGT AAA GGA AAT GAA TTG CTA TCC CCT CAT TAT
     Thr Gly Val Asp Ser Lys Gly Asn Glu Leu Leu Ser Pro His Tyr

        140         150         160         170         180
136  GTC GAG TTT CCT ATT AAA CCT GGG ACT ACA CTT ACA AAA GAA AAA
     Val Glu Phe Pro Ile Lys Pro Gly Thr Thr Leu Thr Lys Glu Lys

                190         200         210         220
181  ATT GAA TAC TAT GTC GAA TGG GCA TTA GAT GCG ACA GCA TAT AAA
     Ile Glu Tyr Tyr Val Glu Trp Ala Leu Asp Ala Thr Ala Tyr Lys

        230         240         250         260         270
226  GAG TTT AGA GTA GTT GAA TTA GAT CCA AGC GCA AAG ATC GAA GTC
     Glu Phe Arg Val Val Glu Leu Asp Pro Ser Ala Lys Ile Glu Val
```

**Figur 3 (Fortsetzung)**

```
                 280                 290                 300                 310
271  ACT TAT TAT GAT AAG AAT AAG AAA AAA GAA GAA ACG AAG TCT TTC
     Thr Tyr Tyr Asp Lys Asn Lys Lys Lys Glu Glu Thr Lys Ser Phe

         320                 330                 340                 350                 360
316  CCT ATA ACA GAA AAA GGT TTT GTT GTC CCA GAT TTA TCA GAG CAT
     Pro Ile Thr Glu Lys Gly Phe Val Val Pro Asp Leu Ser Glu His

                 370                 380                 390                 400
361  ATT AAA AAC CCT GGA TTC AAC TTA ATT ACA AAG GTT GTT ATA GAA
     Ile Lys Asn Pro Gly Phe Asn Leu Ile Thr Lys Val Val Ile Glu

         410
406  AAG AAA TAA
     Lys Lys ---
```

**Figur 4**

SEQ ID NO:4
ART DER SEQUENZ:Nucleotid mit entsprechendem Protein
SEQUENZLÄNGE:384 Basenpaare
STRANGFORM:Einzelstrang
TOPOLOGIE:linear
ART DES MOLEKÜLES:rekombinante DNA
URSPRÜNGLICHE HERKUNFT ORGANISMUS:Staphylococcus aureus Phage 42D
UNMITTELBARE EXPERIMENTELLE HERKUNFT:Plasmid pMET5
MERKMALE:
von 1 bis 3 bp ATG-Startcodon
von 4 bis 381 bp verkürztes reifes Protein
von 382 bis 384 bp TAA-Stopcodon

```
                 10                  20                  30                  40
  1  ATG AAA GGC GAT GAC GCG AGT TAT TTT GAA CCA ACA GGC CCG TAT
     MET Lys Gly Asp Asp Ala Ser Tyr Phe Glu Pro Thr Gly Pro Tyr

         50                  60                  70                  80                  90
 46  TTG ATG GTA AAT GTG ACT GGA GTT GAT GGT AAA AGA AAT GAA TTG
     Leu MET Val Asn Val Thr Gly Val Asp Gly Lys Arg Asn Glu Leu

                100                 110                 120                 130
 91  CTA TCC CCT CGT TAT GTC GAG TTT CCT ATT AAA CCT GGG ACT ACA
     Leu Ser Pro Arg Tyr Val Glu Phe Pro Ile Lys Pro Gly Thr Thr

        140                 150                 160                 170                 180
136  CTT ACA AAA GAA AAA ATT GAA TAC TAT GTC GAA TGG GCA TTA GAT
     Leu Thr Lys Glu Lys Ile Glu Tyr Tyr Val Glu Trp Ala Leu Asp

                190                 200                 210                 220
181  GCG ACA GCA TAT AAA GAG TTT AGA GTA GTT GAA TTA GAT CCA AGC
     Ala Thr Ala Tyr Lys Glu Phe Arg Val Val Glu Leu Asp Pro Ser

        230                 240                 250                 260                 270
226  GCA AAG ATC GAA GTC ACT TAT TAT GAT AAG AAT AAG AAA AAA GAA
     Ala Lys Ile Glu Val Thr Tyr Tyr Asp Lys Asn Lys Lys Lys Glu

                280                 290                 300                 310
271  GAA ACG AAG TCT TTC CCT ATA ACA GAA AAA GGT TTT GTT GTC CCA
     Glu Thr Lys Ser Phe Pro Ile Thr Glu Lys Gly Phe Val Val Pro

        320                 330                 340                 350                 360
316  GAT TTA TCA GAG CAT ATT AAA AAC CCT GGA TTC AAC TTA ATT ACM
     Asp Leu Ser Glu His Ile Lys Asn Pro Gly Phe Asn Leu Ile Thr
```

**Figur 4 (Fortsetzung)**

```
                 370             380
361  AAG GTT GTT ATA GAA AAG AAA TAA
     Lys Val Val Ile Glu Lys Lys ---
```

**Figur 5**

SEQ ID NO:5
ART DER SEQUENZ:Nucleotid mit entsprechendem Protein
SEQUENZLÄNGE:372 Basenpaare
STRANGFORM:Einzelstrang
TOPOLOGIE:linear
ART DES MOLEKÜLES:rekombinante DNA
URSPRÜNGLICHE HERKUNFT ORGANISMUS:Staphylococcus aureus Phage 42D
UNMITTELBARE EXPERIMENTELLE HERKUNFT:Plasmid pMET5
MERKMALE:
von 1 bis 3 bp ATG-Startcodon
von 4 bis 369 bp verkürztes reifes Protein
von 370 bis 372 bp TAA-Stopcodon

```
            10          20          30          40
  1 ATG GCG AGT TAT TTT GAA CCA ACA GGC CCG TAT TTG ATG GTA AAT
    MET Ala Ser Tyr Phe Glu Pro Thr Gly Pro Tyr Leu MET Val Asn

        50          60          70          80          90
 46 GTG ACT GGA GTT GAT GGT AAA AGA AAT GAA TTG CTA TCC CCT CGT
    Val Thr Gly Val Asp Gly Lys Arg Asn Glu Leu Leu Ser Pro Arg

            100         110         120         130
 91 TAT GTC GAG TTT CCT ATT AAA CCT GGG ACT ACA CTT ACA AAA GAA
    Tyr Val Glu Phe Pro Ile Lys Pro Gly Thr Thr Leu Thr Lys Glu

        140         150         160         170         180
136 AAA ATT GAA TAC TAT GTC GAA TGG GCA TTA GAT GCG ACA GCA TAT
    Lys Ile Glu Tyr Tyr Val Glu Trp Ala Leu Asp Ala Thr Ala Tyr

            190         200         210         220
181 AAA GAG TTT AGA GTA GTT GAA TTA GAT CCA AGC GCA AAG ATC GAA
    Lys Glu Phe Arg Val Val Glu Leu Asp Pro Ser Ala Lys Ile Glu

        230         240         250         260         270
226 GTC ACT TAT TAT GAT AAG AAT AAG AAA AAA GAA GAA ACG AAG TCT
    Val Thr Tyr Tyr Asp Lys Asn Lys Lys Lys Glu Glu Thr Lys Ser

            280         290         300         310
271 TTC CCT ATA ACA GAA AAA GGT TTT GTT GTC CCA GAT TTA TCA GAG
    Phe Pro Ile Thr Glu Lys Gly Phe Val Val Pro Asp Leu Ser Glu

        320         330         340         350         360
316 CAT ATT AAA AAC CCT GGA TTC AAC TTA ATT ACM AAG GTT GTT ATA
    His Ile Lys Asn Pro Gly Phe Asn Leu Ile Thr Lys Val Val Ile
```

Figur 5 (Fortsetzung)

```
               370
361  GAA AAG AAA TAA
     Glu Lys Lys ---
```

**Figur 6**

SEQ ID NO:6
ART DER SEQUENZ:Nucleotid mit entsprechendem Protein
SEQUENZLÄNGE:414 Basenpaare
STRANGFORM:Einzelstrang
TOPOLOGIE:linear
ART DES MOLEKÜLES:rekombinante DNA
URSPRÜNGLICHE HERKUNFT ORGANISMUS:Staphylococcus aureus Phage 42D
UNMITTELBARE EXPERIMENTELLE HERKUNFT:Plasmid pMET5
MERKMALE:
von 1 bis 3 bp ATG-Startcodon
von 4 bis 411 bp reifes Protein
von 412 bis 414 bp TAA-Stopcodon

```
                        10          20          30          40
  1  ATG TCA AGT TCA TTC GAC AAA GGA AAA TAT AAA AAA GGC GAT GAC
     MET Ser Ser Ser Phe Asp Lys Gly Lys Tyr Lys Lys Gly Asp Asp

             50          60          70          80          90
 46  GCG AGT TAT TTT GAA CCA ACA GGC CCG TAT TTG TGY GTA AAT GTG
     Ala Ser Tyr Phe Glu Pro Thr Gly Pro Tyr Leu Cys Val Asn Val

                       100         110         120         130
 91  ACT GGA GTY GAY GGT AAA AGA AAT GAR CTS YTR TCC CCK CGK TAT
     Thr Gly Val Asp Gly Lys Arg Asn Glu Leu Leu Ser Pro Arg Tyr

           140         150         160         170         180
136  GTC GAG TTT CCT ATT AAA CCT GGG ACT ACA CTT ACA AAA GAA AAA
     Val Glu Phe Pro Ile Lys Pro Gly Thr Thr Leu Thr Lys Glu Lys

                       190         200         210         220
181  ATT GAA TAC TAT GTC GAA TGG GCA TTA GAT GCG ACA GCA TAT AAA
     Ile Glu Tyr Tyr Val Glu Trp Ala Leu Asp Ala Thr Ala Tyr Lys

           230         240         250         260         270
226  GAG TTT AGA GTA GTT GAA TTA GAT CCA AGC GCA AAG ATC GAA GTC
     Glu Phe Arg Val Val Glu Leu Asp Pro Ser Ala Lys Ile Glu Val

                       280         290         300         310
271  ACT TAT TAT GAT AAG AAT AAG AAA AAA GAA GAA ACG AAG TCT TTC
     Thr Tyr Tyr Asp Lys Asn Lys Lys Lys Glu Glu Thr Lys Ser Phe

           320         330         340         350         360
316  CCT ATA ACA GAA AAA GGT TTT GTT GTC CCA GAT TTA TCA GAG CAT
     Pro Ile Thr Glu Lys Gly Phe Val Val Pro Asp Leu Ser Glu His
```

**Figur 6 (Fortsetzung)**

```
                370          380          390          400
361  ATT AAA AAC CCT GGA TTC AAC TTA ATT ACC AAG GTT GTT ATA GAA
     Ile Lys Asn Pro Gly Phe Asn Leu Ile Thr Lys Val Val Ile Glu

         410
406  AAG AAA TAA
     Lys Lys ---
```

Figur 7

SEQ ID NO:7
ART DER SEQUENZ: Nucleotid mit entsprechendem Protein
SEQUENZLÄNGE: 414 Basenpaare
STRANGFORM: Einzelstrang
TOPOLOGIE: linear
ART DES MOLEKÜLES: rekombinante DNA
URSPRÜNGLICHE HERKUNFT ORGANISMUS: Staphylococcus aureus Phage 42D
UNMITTELBARE EXPERIMENTELLE HERKUNFT: Plasmid pMET5
MERKMALE:
von 1 bis 3 bp ATG-Startcodon
von 4 bis 411 bp reifes Protein
von 412 bis 414 bp TAA-Stopcodon

```
                10          20          30          40
  1  ATG TCA AGT TCA TTC GAC AAA GGA AAA TAT AAA AAA GGC GAT GAC
     MET Ser Ser Ser Phe Asp Lys Gly Lys Tyr Lys Lys Gly Asp Asp

         50          60          70          80          90
 46  GCG AGT TAT TTT GAA CCA ACA GGC CCG TAT TTG CTA GTA AAT GTG
     Ala Ser Tyr Phe Glu Pro Thr Gly Pro Tyr Leu Leu Val Asn Val

                100         110         120         130
 91  ACT GGA GYT GAY GGT AAA AGA AAT GAR CTS YTR TCC CCK CGK TAT
     Thr Gly Val Asp Gly Lys Arg Asn Glu Leu Leu Ser Pro Arg Tyr

         140         150         160         170         180
136  GTC GAG TTT CCT ATT AAA CCT GGG ACT ACA CTT ACA AAA GAA AAA
     Val Glu Phe Pro Ile Lys Pro Gly Thr Thr Leu Thr Lys Glu Lys

                190         200         210         220
181  ATT GAA TAC TAT GTC GAA TGG GCA TTA GAT GCG ACA GCA TAT AAA
     Ile Glu Tyr Tyr Val Glu Trp Ala Leu Asp Ala Thr Ala Tyr Lys

         230         240         250         260         270
226  GAG TTT AGA GTA GTT GAA TTA GAT CCA AGC GCA AAG ATC GAA GTC
     Glu Phe Arg Val Val Glu Leu Asp Pro Ser Ala Lys Ile Glu Val

                280         290         300         310
271  ACT TAT TAT GAT AAG AAT AAG AAA AAA GAA GAA ACG AAG TCT TTC
     Thr Tyr Tyr Asp Lys Asn Lys Lys Lys Glu Glu Thr Lys Ser Phe

         320         330         340         350         360
316  CCT ATA ACA GAA AAA GGT TTT GTT GTC CCA GAT TTA TCA GAG CAT
     Pro Ile Thr Glu Lys Gly Phe Val Val Pro Asp Leu Ser Glu His
```

Figur 7 (Fortsetzung)

```
                 370            380            390              400
361  ATT AAA AAC CCT GGA TTC AAC TTA ATT ACM AAG GTT GTT ATA GAA
     Ile Lys Asn Pro Gly Phe Asn Leu Ile Thr Lys Val Val Ile Glu

        410
406  AAG AAA TAA
     Lys Lys ---
```

**Figur 8**

SEQ ID NO:8
ART DER SEQUENZ:Nucleotid
SEQUENZLÄNGE:29 Basenpaare
STRANGFORM:Einzelstrang
TOPOLOGIE:linear
ART DES MOLEKÜLES:synthetische DNA
URSPRÜNGLICHE HERKUNFT ORGANISMUS:
UNMITTELBARE EXPERIMENTELLE HERKUNFT:Oligonukleotidsynthese
MERKMALE:
von 1 bis 4 bp Ribosomenbindungsort 1
von 15 bis 20 bp Ribosomenbindungsort 2
von 27 bis 29 bp Translationsstartcodon

1 AGGAAACAGA ATTCAGGAGG CCTCAT|ATG

**Figur 9**

SEQ ID NO:9
ART DER SEQUENZ:Nucleotid
SEQUENZLANGE:1023 Basenpaare
STRANGFORM:Einzelstrang
TOPOLOGIE:linear
ART DES MOLEKÜLES:genomische DNA
URSPRÜNGLICHE HERKUNFT ORGANISMUS:Staphylococcus aureus Phage 42D
UNMITTELBARE EXPERIMENTELLE HERKUNFT:Plasmid pDB17
MERKMALE:
von 224 bis 229 bp -35 Region des Promoters
von 248 bis 253 bp -10 Region des Promoters
von 332 bis 336 bp Shine-dalgarno-Sequenz
von 344 bis 415 bp Signalpeptid
von 416 bis 832 bp reifes Protein SAK42D

```
  1                                              ACT  AACTATAGAT

 14 TCGATGTTGG  ACAGGCTGTA  TACGCGCCTG  GAACATTAAT  ATATGTGTTT

 64 GAAATTATAG  ATGGTTGTTG  TCGCATTTAT  TGGAACAATC  ATAATGAGTG

114 GATATGGCAT  GAGAGATTGA  TTGTGAAAGA  AGTGTTTTAA  TTCTAAGGTT

164 AAAATGTTAA  ATATTTGTTA  ATTATTTTTT  AATGTAAGTT  TAGTTTCTTT

214 TAATATTTTA  TTGATTTTTA  ATATTTTCTC  AATATAAAAT  GAAGTTGTTG

264 ATATTTATCA  TCTTAAATAA  GGGTGTTAGC  TATAAAAAGA  GATAAATAAA

314 AACAAATATA  TTATATTTGG  AGGAAGCGCC  ATG CTC AAA AGA AGT TTA
                                        MET Leu Lys Ser Ser Leu

362 TTA TTT TTA ACT GTT TTA TTG TTA TTA TTC TCA TTT TCT TCA ATT
    Leu Phe Leu Thr Val Leu Leu Leu Leu Phe Ser Phe Ser Ser Ile
```

**Figur 9 (Fortsetzung)**

```
407 ACT AAT GAG GTA AGT GCA TCA AGT TCA TTC GAC AAA GGA AAA TAT
    Thr Asn Glu Val Ser Ala Ser Ser Ser Phe Asp Lys Gly Lys Tyr

452 AAA AAA GGC GAT GAC GCG AGT TAT TTT GAA CCA ACA GGC CCG TAT
    Lys Lys Gly Asp Asp Ala Ser Tyr Phe Glu Pro Thr Gly Pro Tyr

497 TTG ATG GTA AAT GTG ACT GGA GTT GAT GGT AAA AGA AAT GAA TTG
    Leu MET Val Asn Val Thr Gly Val Asp Gly Lys Arg Asn Glu Leu

542 CTA TCC CCT CGT TAT GTC GAG TTT CCT ATT AAA CCT GGG ACT ACA
    Leu Ser Pro Arg Tyr Val Glu Phe Pro Ile Lys Pro Gly Thr Thr

587 CTT ACA AAA GAA AAA ATT GAA TAC TAT GTC GAA TGG GCA TTA GAT
    Leu Thr Lys Glu Lys Ile Glu Tyr Tyr Val Glu Trp Ala Leu Asp

632 GCG ACA GCA TAT AAA GAG TTT AGA GTA GTT GAA TTA GAT CCA AGC
    Ala Thr Ala Tyr Lys Glu Phe Arg Val Val Glu Leu Asp Pro Ser

677 GCA AAG ATC GAA GTC ACT TAT TAT GAT AAG AAT AAG AAA AAA GAA
    Ala Lys Ile Glu Val Thr Tyr Tyr Asp Lys Asn Lys Lys Lys Glu

722 GAA ACG AAG TCT TTC CCT ATA ACA GAA AAA GGT TTT GTT GTC CCA
    Glu Thr Lys Ser Phe Pro Ile Thr Glu Lys Gly Phe Val Val Pro

767 GAT TTA TCA GAG CAT ATT AAA AAC CCT GGA TTC AAC TTA ATT ACA
    Asp Leu Ser Glu His Ile Lys Asn Pro Gly Phe Asn Leu Ile Thr

812 AAG GTT GTT ATA GAA AAG AAA TAA     AACAAAATAG  TTGTTTATTA
    Lys Val Val Ile Glu Lys Lys ---

856 TAGAAAGCAA  TGTCTTGCTT  GAATATGTGT  AGTGAAAATT  ATCTTTCATC

906 AAATTCTCAT  TCATGCACGA  ATGGCTCTTC  CCCACCTAAT  CAGATATTAG

956 GTGACTTATG  GGGAGAAATC  AGTTAGGATA  AAAAGTGGAT  AATCCTTTTT

1006 TTAGGCAGGT  TCCAGGCA
```

**Figur 10**

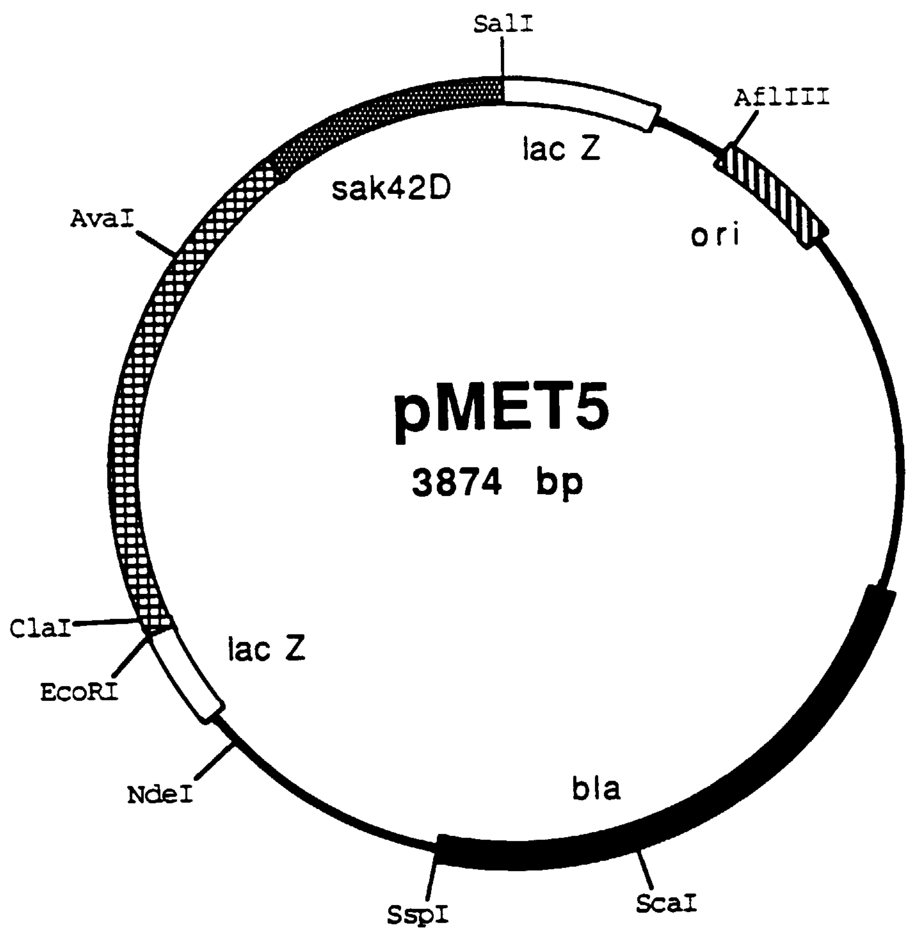
SalI
AflIII
lac Z
sak42D
AvaI
ori
pMET5
3874 bp
ClaI
EcoRI
lac Z
NdeI
bla
SspI
ScaI

**Figur 11**

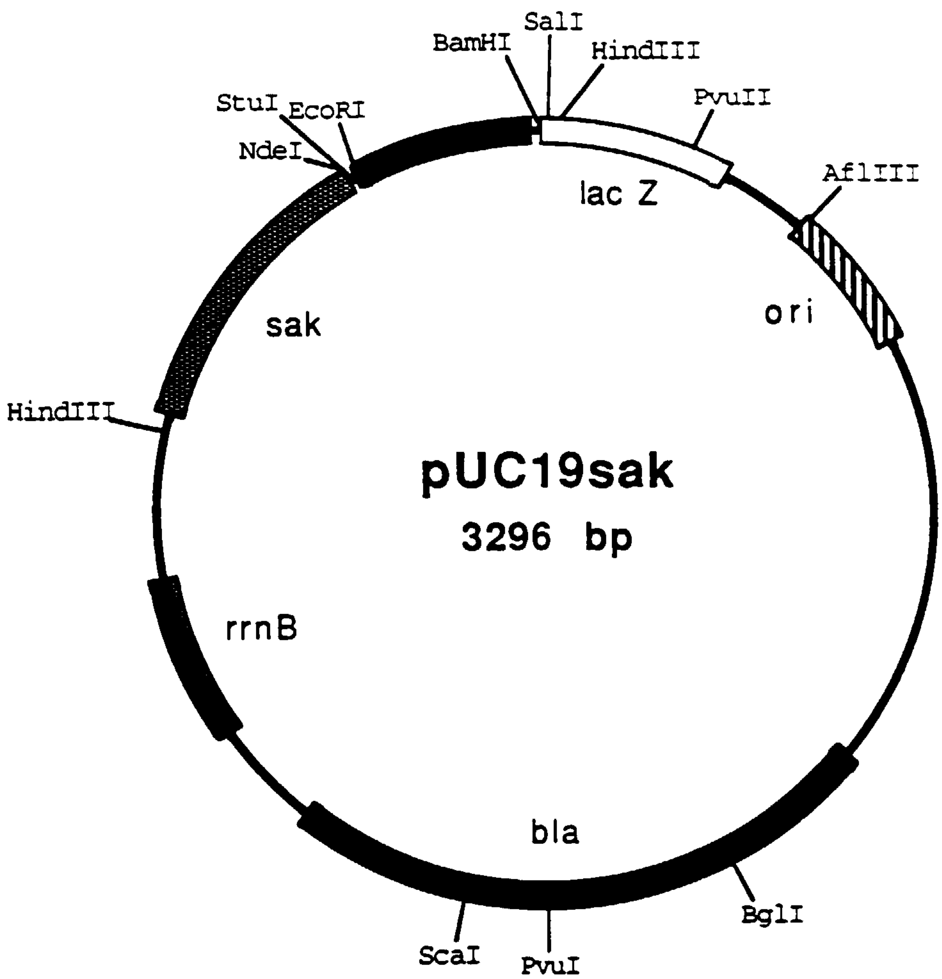
SalI
BamHI
HindIII
PvuII
StuI
EcoRI
NdeI
AflIII
lac Z
ori
sak
HindIII
pUC19sak
3296 bp
rrnB
bla
BglI
ScaI
PvuI

Figur 12

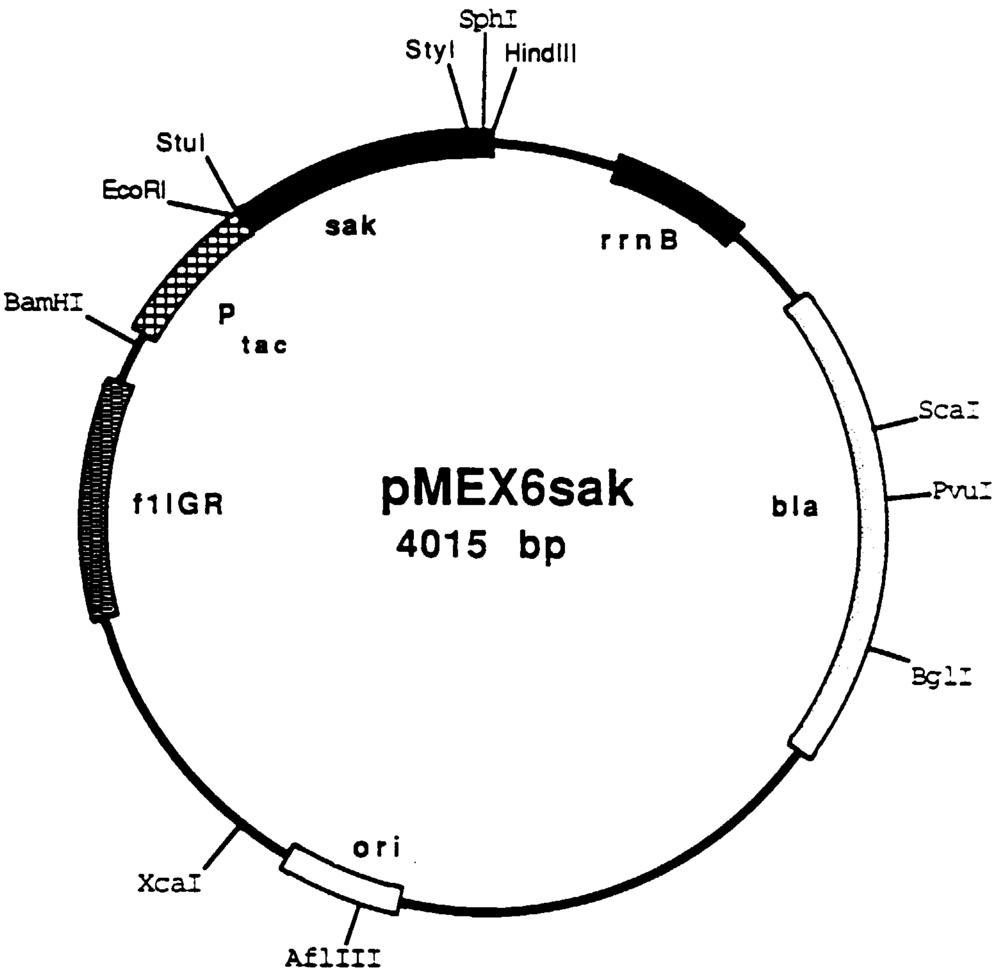
SphI
StyI
HindIII
StuI
EcoRI
sak
rrnB
BamHI
P
tac
ScaI
PvuI
f1IGR
pMEX6sak
4015 bp
bla
BglI
ori
XcaI
AflIII

Figuren 13a und 13b

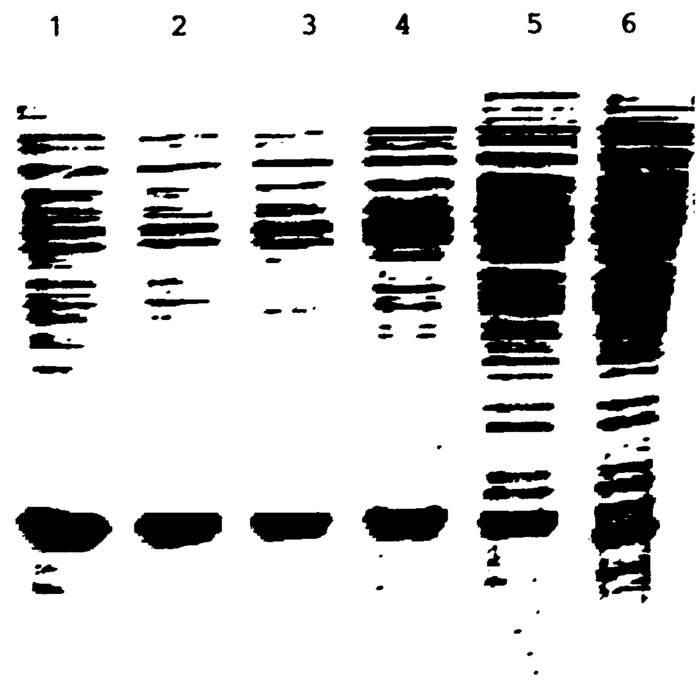


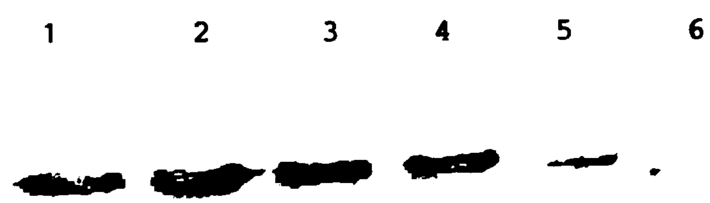

Figur 14

1 2 3 4 5 6

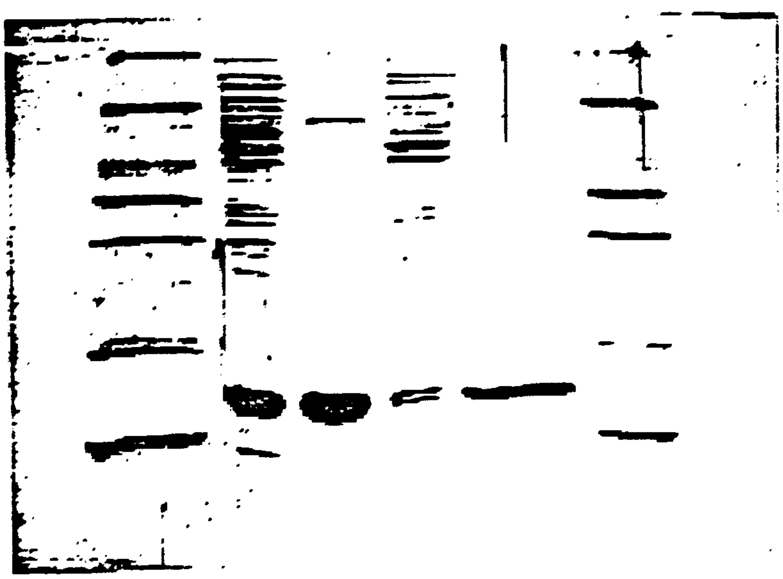

Figur 15

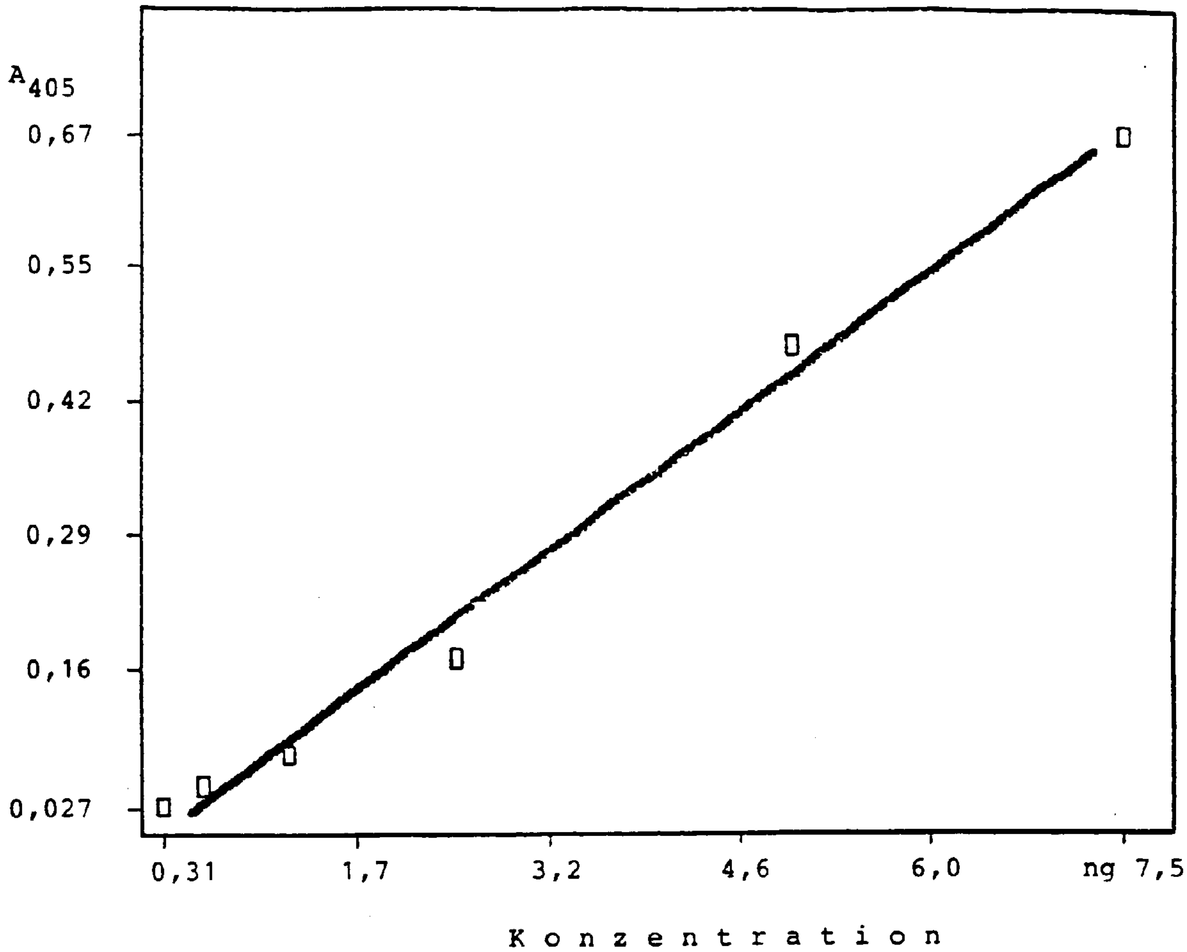


Eichkurve des ELISA-Tests zum quantitativen Nachweis von Staphylokinase.
Die Eckpunkte des Meßbereiches sind 300pg/ml (min.) bzw. 7.5 ng/ml (max.) Staphylokinasekonzentration

Figur 16